# EUROPEAN PATENT SPECIFICATION

(11) **EP 3 362 138 B1**
(45) Date of publication and mention of the grant of the patent: **13.04.2022**
(21) Application number: 16790679.1
(22) Date of filing: 14.10.2016
(51) Int. Cl.: A61N 1/32, A61N 1/36

(54) **KIT FOR PROMOTING NERVE REGENERATION USING ELECTRICAL STIMULATION**
KIT ZUR FÖRDERUNG DER NERVENREGENERATION MIT ELEKTRISCHER STIMULATION
KIT POUR FAVORISER LA RÉGÉNÉRATION NERVEUSE À L'AIDE D'UNE STIMULATION ÉLECTRIQUE

(30) Priority: 15.10.2015 GB 201518263
(43) Date of publication of application: 22.08.2018
(73) Proprietor: Oxford Bioelectronics Ltd, Newcastle upon Tyne NE4 6DB (GB)
(72) Inventor: BAYAT, Ardeshir, Manchester Lancashire M20 2PS (GB); SEBASTIAN, Anil, Manchester Lancashire M18 7NA (GB)
(74) Representative: Creation IP Ltd
(86) International application number: PCT/GB2016/053185
(87) International publication number: WO 2017/064500

(56) References cited:
- WO-A1-2005/014105
- WO-A2-2005/105981
- US-A1- 2002 120 309
- US-A1- 2004 215 290
- US-A1- 2007 038 311
- US-A1- 2007 129 759
- US-A1- 2013 178 785
- US-A1- 2013 304 174
- US-B2- 6 941 173

## Description

### Field of the Invention

The invention relates to promoting nerve reinnervation using electrical stimulation.

### Background of the Invention

Cutaneous wound re-innervation involves sequentially regulated events including morphological and biochemical changes leading to neurogenesis, synaptogenesis, and further organisation of the nervous tissue^{1,2}. Thus, re-innervation of afferent sensory nerve fibres³, characterised by neural differentiation and synthesis of functional neuropeptides⁴ takes place throughout cutaneous repair. These neuropeptides exhibit paracrine signalling⁵, which augments the healing process in part by regulating cell proliferation, growth factor and cytokine production, and neovascularization^{6,7}. Re-innervation is primarily responsible for the maintenance of an intact epidermis⁸. Certain nerve fibres end freely in the dermis⁹ while in many others, although, the central trunk terminates in the dermis, subsequent branching extends throughout the epidermis¹⁰. However, re-innervation to the epidermal component remains poorly characterised. Several hypothesis involving cytokine/neurotrophin interactions have been proposed for enhancing re-innervation, indeed, most of them have considered only the inflammation aspect of the multi-staged healing process⁸. Additionally, there is lack of evidence on spatial and temporal expression of neural/neural-related differentiation proteins during repair.

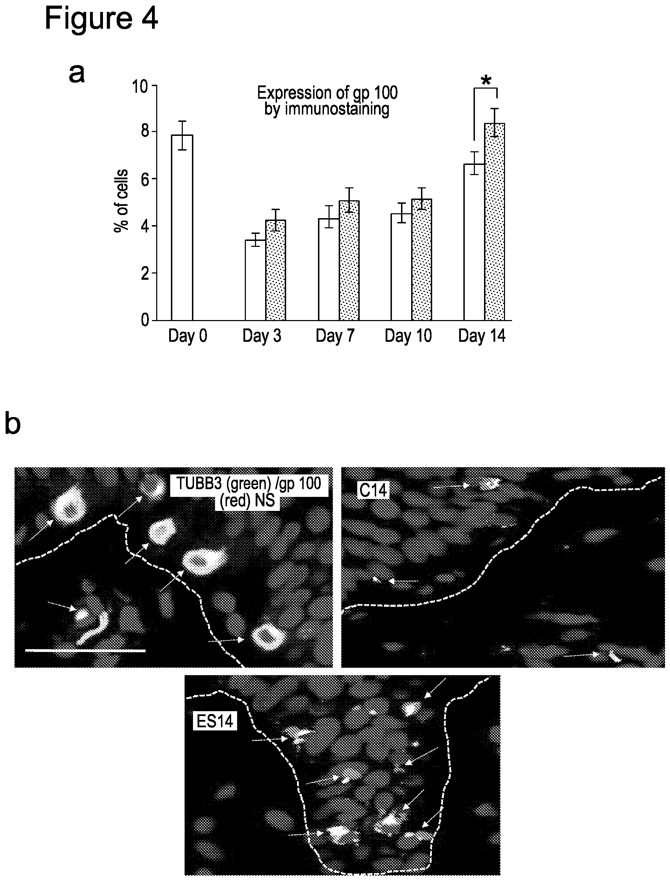

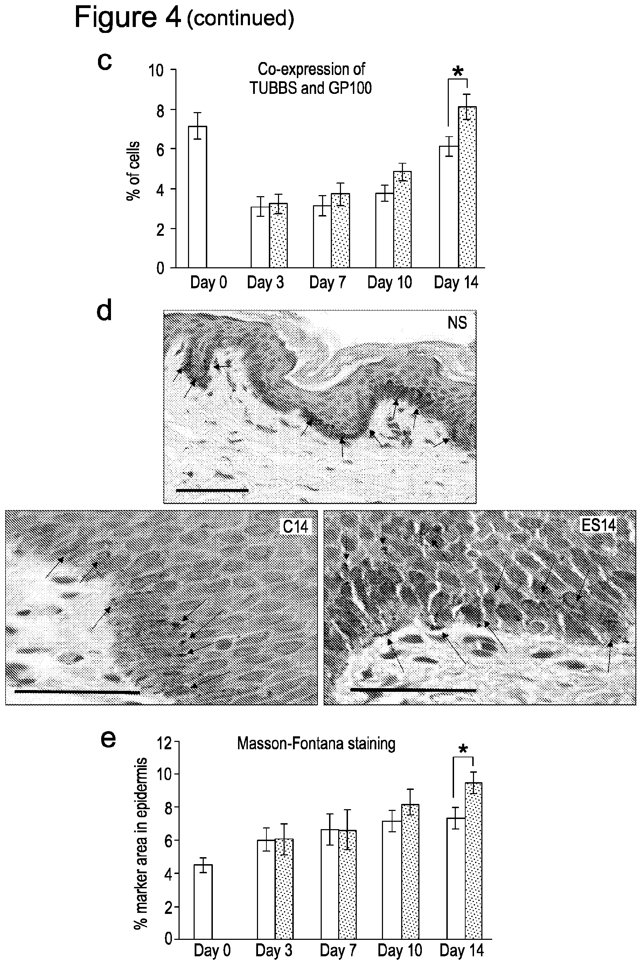

Neural differentiation is characterised by early neurite outgrowth¹¹ which augments reinnervation. Biomarkers for neural differentiation extends from mitotic spindle components¹² to lipid membrane domains¹³. Even though a microtubule protein- Class III β-tubulin (TUBB3) has been classified as neural-specific differentiation marker¹⁴ and has been used for indicating reinnervation in tissue regeneration¹⁵, recently it has been reported to be associated to a minor extent with multiple cell types¹⁶. Factor Induced Gene 4 (FIG4), which is a key component in synthesising phosphatidylinositol (3,4,5)-triphosphate ((PI(3,4,5)P₃ or PIP₃) in the plasma membrane, is another mediator during neural differentiation¹⁷ Interestingly, neural differentiation promoters such as nerve growth factor (NGF) has also shown to modulate gene expression in epidermal melanocytes¹⁸ and Merkel cells¹⁹.

Electrical stimulation (ES) has been shown an *in vitro* model to enhance neural differentiation events, facilitated by either cell-based^{20,21} or substrate-based modifications^{22,23}. In this context, previous reports have shown ES and neural differentiation as a result of neurite outgrowth, neuronal tubulin induction, neurofilament 200 and *c-fos* gene expression in chick embryos²⁴ and rats²⁵, however, little information is available during repair process in humans. Importantly, ES has been shown to promote cutaneous wound repair^{26,27}. Recently, we demonstrated acceleration in human skin healing by ES with enhanced angiogenesis²⁸ and increased rate of re-epithelialisation²⁹. The rationale for exogenous application of ES is to mimic the *in vivo* bioelectric system to augment the endogenous wound current that promotes repair. However, the mechanisms through which ES impacts on healing process and the key target cell populations remain unknown¹¹.

US2002/0120309 discloses a method of stimulating recovery of peripheral nerve damage by the application of electrical signals along a damaged nerve to evoke an action potential in one or more motor axons. US2013/0178785 discloses a system for healing wound/damage of warm-blood organism using therapeutic agents, the system having a suction mechanism placed in fluid communication with compartment, and power source placed in electrical communication with electrode.

### Summary of the Invention

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

The inventors have surprisingly shown that it is possible to promote the reinnervation of a damaged nerve in a patient by electrically stimulating the skin of the patient at different location from the site of damage to the nerve. In particular, the inventors have surprisingly shown that it is possible to promote the reinnervation of a damaged nerve in a patient even if the electrically stimulation is applied far enough away from the site of damage such that the electrical signals or implulses are not conducted by the skin to the site of damage.

### Brief Description of the Figures

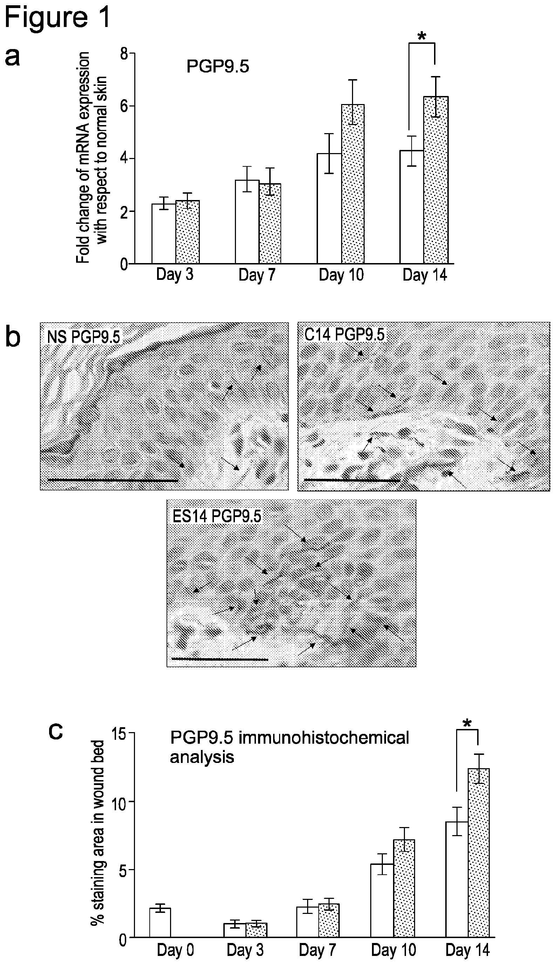

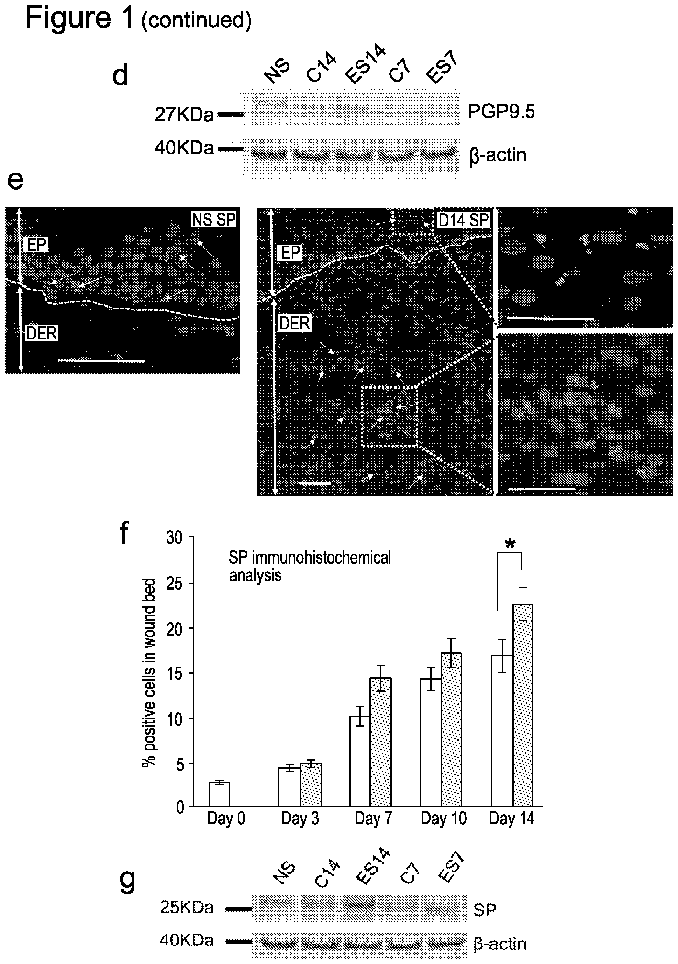

Figures 1 to 13 illustrate clinical results illustrating nerve reinnervation in wounds following electrical stimulation of the skin of a patient.

### Description of the Sequence Listing

SEQ ID NO: 1 shows the mRNA sequence of human reticulon-4 isoform A. http://www.ncbi.nlm.nih.gov/nuccore/47519458
SEQ ID NO: 2 shows the amino acid sequence of human reticulon-4 isoform A. The sequence of Nogo-66 is underlined, http://www.ncbi.nlm.nih.gov/nuccore/47519458
SEQ ID NO: 3 shows the mRNA sequence of the human Nogo-66 receptor-1 (NGRH1). http://www.ncbi.nlm.nih.gov/nuccore/AF532858.1
SEQ ID NO: 4 shows the amino acid sequence of sequence of the human Nogo-66 receptor-1 (NGRH1). http://www.ncbi.nlm.nih.gov/nuccore/AF532858.1
SEQ ID NO: 5 shows the mRNA sequence of the human Nogo-66 receptor-2 (NGRH2). http://www.ncbi.nlm.nih.gov/nuccore/AF532859.1
SEQ ID NO: 6 shows the amino acid sequence of the human Nogo-66 receptor-2 (NGRH2). http://www.ncbi.nlm.nih.gov/nuccore/AF532859.1
SEQ ID NOs: 7 to 18 are the forward and reverse primers used in the Examples and listed in Table 4.

### Detailed Description of Embodiments

### Reinnervation

The method concerns promoting reinnervation of a damaged nerve. The terms nerve and neuron are interchangeable here and so the method may concern promoting reinnervation of a damaged neuron. The nerve may be damaged in any way, such as by an injury, such as a wound, or by a disease or disorder, such as a skin disese or skin disorder. The nerve may be damaged to any extent. For instance, the nerve may be severed.

The nerve may be myelinated. The nerve may be unmyelinated.

The damged nerve may be anywhere in the patient. The nerve may be present in the central nervous system (CNS), *i.e.* is centrally located or is a central nerve. The nerve may be a centrally located cranial or spinal. CNS nerves are typically unmyelinated. If the damaged nerve is in the CNS, then electrically stimulating the skin inherently involves electrical stimulation at a different location from the site of damage to the nerve.

The nerve is in one embodiment present in the peripheral nervous system (PNS), *i.e.* is a peripheral nerve. PNS nerves may be myelinated or unmyelinated. The damaged nerve is in one embodiment in the skin of the patient. The site of damage to the nerve is in one embodiment in the skin of the patient. The damaged nerve in one embodiment innervated the skin of the patient before it was damaged. In such instances, the method involves electrically stimulating the skin of the patient at a different location from the site of damage to the nerve in the skin. Suitable distances between the location of damage and location of stimulation are discussed in more detail below.

Skin typically comprises (a) an epidermis, (b) a dermis and (c) a hypodermis. The damaged nerve or the site of damge may be present in any of these or any combination thereof, such as (a); (b); (c); (a) and (b); (a) and (c); (b) and (c); or (a), (b) and (c). The epidermis is typically electrically stimulated in accordance with the one example.

Reinnervation is the restoration of the nerve supply to a part of the body from which it has been lost. The treatment typically restores the supply of the damaged nerve to the site of damage. The treatment may help to heal an injury, such as a wound, or a site damaged by a disease or disorder. The treatment may restore the damaged nerve's innveration of the skin of the patient.

The treatment may help to partially restore the supply of the damaged nerve to the site of damage. The treatment may partially restore the damaged nerve's innveration of the skin of the patient. For instance, the supply of the damaged nerve to the site of damage or to the skin of the patient my be restored by at least about 10%, at least about 20%, at least about 30%, at least about 40%, at least about 50%, at least about 60%, at least about 70%, at least about 80% or at least about 90%. The treatment may completely restore the damaged nerve's innveration of the skin of the patient. The treatment may completely restore the damaged nerve's innveration of the skin of the patient. Nerve innervation may be measured using routine techniques, such as those described in the Examples. Reinnervation may be measured using increased expression of Class III β-tubulin (TUBB3 and Factor Indiced Gene 4 (FIG4) at the site of the damaged nerve. This is discussed in more detail below.

### Patient

Any patient may be treated. The patient is typically human. However, patient may be another mammalian animal, such as a commercially farmed animal, such as a horse, a cow, a sheep, a fish, a chicken or a pig, a laboratory animal, such as a mouse or a rat, or a pet, such as a guinea pig, a hamster, a rabbit, a cat or a dog.

The human patient may be any age.

The patient may be diagnosed with with a damaged nerve, i.e. known to have a damaged nerve. The patient typically demonstrates the symptoms of a damaged nerve, in one embodiment in the skin. The patient may be suspected of having a damaged nerve. The patient may have an injury, such as a wound, or a disease or disorder, in one embodiment a skin disease or disorder.

### Electrical stimulation

The skin of the patient may be electrically stimulated in any way. AC signals can be used, or the signals may be pulsed. The AC signals may be modulated by a pulsed waveform.

The skin is typically electrically stimulated with a series of signals or impulses. The skin is typically electrically stimulated with a series of alternating current (AC) signals or impulses. The signals or impulses are typically provided in a particular waveform. Any waveform may be used. The skin is in one embodiment electrically stimulated with an analogue nerve impulse waveform, an assymetric waveform or a biphasic waveform. The skin may be electrically stimulated with a regenerate waveform. The skin is in one embodiment electrically stimulated with a degenerate waveform. This is discussed in more detail below.

The skin of the patient is in one embodiment electrically stimulated with a current of from about 0.001 milliamps (mA) to 0.006 mA, such as from about 0.002 mA to about 0.005 mA or from about 0.003 mA to about 0.004 mA. The skin is in one embodiment electrically stimulated with a current of about 0.004 mA.

The skin of the patient is in one embodiment electrically stimulated with an amplitude of from about 10 volts (V) to about 100 V, such as from about 15V to about 90 V or from about 20 V to about 80V.

The skin of the patient is in one embodiment electrically stimulated with a frequency of from about 15 hertz (Hz) to about 351 Hz, such as from 50 Hz to about 300 Hz, from about 80 Hz to about 250 Hz or from about 100 Hz to about 200 Hz. The skin of the patient is in one embodiment electrically stimulated with a frequency of from about 120 Hz to about 180 Hz. The skin of the patient is in one embodiment electrically stimulated with a frequency centered at about 150 Hz.

The skin of the patient is in one embodiment electrically stimulated with a fluctuating frequency cycle of from about 15 Hz to about 351 Hz (or any of the ranges disclosed above) wherein the frequency changes within a cycle of from about 1 second to about 20 seconds, such from about 3 second to about 15 seconds or from about 5 second to about 10 seconds. The skin of the patient is more in one embodiment electrically stimulated with a fluctuating frequency cycle of from about 15 to about 351 Hz (or any of the ranges disclosed above) wherein the frequency changes within a cycle of about 8 seconds. The fluctuating frequency cycle is in one embodiment used at the end of a particular treatment round for the reasons discussed below. The method in one embodiment comprises carrying out one or more, such as 2, 3, 4, 5, 6, 7, 8, 9 or 10, fluctuating frequeny cycles at the end of a round of treatment.

The skin of the patient may be electrically stimulated with interrupted cycles. In one embodiment interrupted cycles include where the amplitude drops to its minimum for a certain amount of time, such as about 0.5 seconds or 1 second, and the amplitude is at its appropriate amount (such as those discussed above) for a different, typically longer and in one embodiment double, amount of time, such as about 1 second or about 2 seconds.

The skin of the patient is in one embodiment electrically stimulated with a series of signals or impulses which last from about 1 microsecond (µs) to about 800 µs, such as 2 µs to about 700 µs, from about 5 µs to about 600 µs, from about 10 µs to about 500 µs, from about 20 µs to about 300 µs, from about 30 µs to about 200 µs, or from about 50 µs to about 100 µs. The skin of the patient is in one embodiment electrically stimulated with a series of signals or impulses which last from about 1 microsecond (µs) to about 100 µs. The skin of the patient is in one embodiment electrically stimulated with a series of signals or impulses which last about 60 µs.

### Location of electrical stimulation

Any part of the skin (or location on the skin) may be electrically stimulated as long as it is at a different location from the site of damage to the nerve. The different location is in one embodiment far enough away from the site of damage such that the electrical signals or impulses are not conducted by the skin to the site of damage. The different location is in one embodiment at least about 1.0 mm from the site of damage, such as at least about 1.5 mm, at least about 2.0 mm, at least about 2.5 mm, at least about 3.0 mm, at least about 3.5 mm, at least about 4.0 mm, at least about 4.5 mm, at least about 5.0 mm, at least about 10 mm, at least about 20 mm, at least about 30 mm, at least about 40 mm, at least about 50 mm or at least about 100 mm from the site of damage. The different location may be further from the site of damage, such as at least about 15 centimetres (cm), at least about 20 cm, at least about 30 cm or at least about 50 cm from the site of damage. In one embodiment the electrical stimulation is applied on the same limb or body part as the site of nerve damage so as to apply the electrical stimulation with a close region but far enough away from the site of damage such that the electrical signals or impulses are not conducted by the skin to the site of damage

Specific sites within the different location of skin can be electrically stimulated as discussed in more detail below.

### Duration

The skin of the patient may be electrically stimulated for any length of time. In one embodiment lengths of time range from about 5 seconds to about an hour, from about 10 seconds to about 50 minutes, from about 1 minute to about 40 minutes or from about 15 minutes to about 30 minutes. As discussed in more detail below, in one embodiment the electrical stimulation of the skin may involve measuring the rate of change of the skin's impedance. In one embodiment the treatment may be carried out until the rate of change of the impedance of the patient's skin is zero.

### Number of treatments

The patient may receive any number of different rounds of treatment *(i.e.* different rounds of electrical stimulation), such as about 5, about 10, about 12, about 15 or about 20 treatments. The patient may receive from about 1 to about 10 rounds of treatment in the first two weeks.

The different rounds of treatments may be the same in terms of the (a) electrical stimulation used, (b) sites and/or location of treatment and (c) duration or of treatment or may be different on the basis of one or more of (a) to (c), such as (a); (b); (c); (a) and (b); (a) and (c); (b) and (c); or (a), (b) and (c).

The different rounds of treatments may be spread over any length of time, such as about a week, about a month or about a year. The treatment of the patient may be continued until there is any amount of restoration of the nerve supply to the damaged site. The treatment of the patient may be continued until the damaged nerve is successfully reinnervated.

### Degenerate waveforms

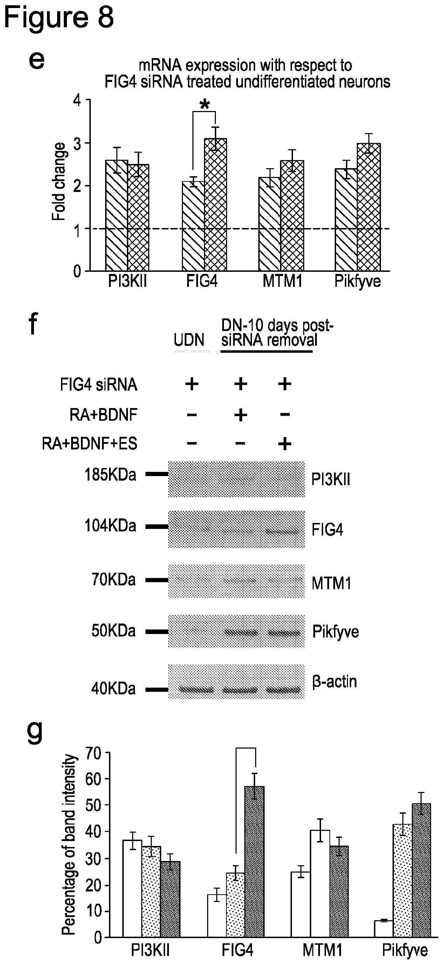

The skin of the patient is in one embodiment electrically stimulated with a degenerate waveform. Such a waveform can be biphasic and asymmetric. In one embodiment, a suitable waveform can be represented by an AC sine waveform modulated by a sawtooth waveform to provide pulses of decaying AC signals. Such a waveform is illustrated in figures 8 to 13 of WO2015/118061.

The skin may be electrically stimulated by detecting the skin's electrical impedance and automatically adjusting the electrical stimulation accordingly. As discussed in more detail below, the electrical stimulation in one embodiment involves measuring the skin's impedance and/or the rate of change of the skin's impedance. The dint of the skin may form a bridge between the electrodes of the device used for electrical stimulation and thereby form the final part of an 'open' circuit such that changes in the skin electrical behaviour alter the flow in the output circuit.

Before treatment, the method in one example comprises electrically stimulating several sites at a particular location on the patient's skin and measuring the impedance of the skin and/or the rate of change of the impedance of the skin at each site. The site of the skin with the highest (or maximum) impedance and/or having the highest rate of change of impedance may then be further electrically stimulated, for instance using one or more fluctuating frequency cycles as described above. The electrical stimulation used to monitor changes in skin impedance may be any of those discussed above.

Before treatment, the method of one example comprises electrically stimulating several sites at a particular location on the patient's skin and monitoring for any changes in (i) friction of the skin, (ii) sensation of the patient, (iii) colour of the skin or (iv) any combination thereof, such as (i); (ii); (iii); (i) and (ii); (i) and (iii); (ii) and (iii); or (i), (ii) and (iii). The site of the skin exhibiting any changes in (i) to (iv) or the most significant changes in (i) to (iv) may then be further electrically stimulated, for instance using one or more fluctuating frequency cycles as described above. The electrical stimulation used to monitor changes in (i) to (iv) may be any of those discussed above.

Each site may be any shape, such as a circle, square or rectangle. Other shapes are also possible. Each site may be any size, such as from about 1 cm² to about 20 cm², from about 2 cm² to about 15 cm^{2 or} from about 5 cm² to about 10 cm².

### Electrobiofeedback (EBF) or neurobiofeedback (NBF)

The electrical stimulation of the skin typically results in a stimulatory adjustment of the body's own self-repair-system in the patient. The stimulatory adjustment of the body's own self-repair-system is used to reinnervate the damaged nerve. In other words, the electrical stimulation of the skin may stimulate the patient's own body to reinnervate the damaged nerve. This may be known as electrobiofeedback (EBF) or neurobiofeedback (NBF).

Without wishing to be bound by any theory, EBF/NBF assumes a "dialogue" of sorts between skin cells and the central nervous system via nerve fibres, in particular unmyelinated c-fibres, most likely due to the common ancestry of skin cells and nerve cells, both being embryologically derived from the neuroectoderm. It can be assumed that the skin can be considered as a "peripheral brain" receptor and transmitter through which a dialogue with the central nervous system can be initiated and manifest. Changes within the body, such as pathology, may result in an altered skin impedance which may occur, for example over the site of the diseased organ, or it may even manifest itself in an apparently unrelated area of the skin. Hence, specific centres in the central nervous system, for example the spine may be addressed through electrical stimulation of the skin (for instance using a plurality of electrons as the "pharmacological substance" as discussed in more detail below). The body is essentially "initiated" to undergo a process of self-repair and healing and the electrical stimulation when applied to the correct site initiates the body's self-repair system. In this context, it should be noted that inflammation (along with pain) is one of the body's "attention seekers" for the body's self- repair-systems (the body's "works departments", so to speak). Initiating a dialogue between skin cells and the CNS may lead to an alert of the CNS, which, in turn, causes an allocation of resources of the body's self-repair system. The electrostimulation may ensure that this allocation of resources is done in the right, i.e. appropriate way, thus leading to a reduction of inflammation, concomitantly with such repair being initiated and successfully completed.

Again, without wishing to be bound by any theory, the physiological processes underlying the pharmacological effect provided by the electrical stimulation when applied to the skin are believed to be as follows. As described above, the electrical stimulation may act via a biofeedback system based on reaction to skin impedance. The signals or impulses are typically of short duration (typically from about 1 µs to about 100 µs, in one embodiment about 60 µs) and of relatively high amplitude (from about 10 V to about 100 V, in one embodiment about 80V). The influence is critically controlled by careful observation using specific measured parameters of the series or impulses depicted on the device screen. Due to the short duration of impulse the energy of the signal is extremely small and harmful effects highly unlikely.

Devices suitable for the method are discussed in more detail below. The device is typically able to detect the areas of lowest skin impedance in an "area of possibility" (between two electrodes, e.g., concentric rectangular electrodes) which it denotes by numerical readout. Dialogue is typically initiated through points of the skin having low impedance , where the biofeedback is relatively more active (and so more efficacious), especially when guided by observation of this dialogue by a trained practitioner. The term "biofeedback", as used herein, is generally meant to refer to the responsivity of the skin towards the application of electrical stimulation, as measured for example, by the rate of change of skin impedance. "Biofeedback" is "active" in those sites where the rate of change of skin impedance is higher in comparison to adjacent sites. Other indicators of "biofeedback" include redness of the skin, friction of the skin, skin sensation of the patient and other detectable parameters as discussed above.

Via nerve endings the afferent impulses from the electrical stimulation enter the central nervous system (CNS) at the anterior horns of the spinal cord. Both myelinated and unmyelinated nerves are stimulated by the impulses. By numerical supremacy the majority of the dialogue takes place via the c-fibres. Impulses are typically conducted up the dorsal and ventral spinothalamic tracts, the dorsal and ventral spinocerebellar tracts and the spinotectal tracts. There may also be a contribution via the reticulo- cerebral fibres and the pontine tegmentum. Some of the facilitatory effects of the electro stimulatory system are believed to be mediated by this part of the reticular formation. Anohin demonstrated continuation of the reticular formation communications beyond brain stem to the cortex with associated influence on cortical responses. Efferent signals typically descend via the corticospinal tracts. Frequently, more than one segmental level is influenced simultaneously. This may result in 'unexpected' recovery from old pathology whilst treating an apparently unconnected presenting complaint.

Via diverse neurotransmitters, influences alter the signals on foci of dominance relating to both reflex arcs and autonomic regulation with attendant distal influence and alteration of efferent impulses on both the autonomic and peripheral systems. The degree to which these responses are affected is in turn adjusted by the higher cortical and pontine impact on the lability of the lower spinal system. Foci in the spinal cord, possibly present for many years as a result of unresolved or incompletely resolved pathology may be re-stimulated by the electrostimulation so that 'normal' pathways can resume the 'stalled' resolution process. A 'hierarchy of dominance' of these foci prevails in the spinal cord. This hierarchy may relate either to the relative survival advantage of a particular complaint or the historic acquisition of the pathology. Fushpan and Potter (in the 1950s) demonstrated the variability of synaptic response between synapses predominating in chemical stimulation and those predominating in electrical stimulation. Many disorders relate to disturbed chemistry of synaptic transmission. Some of these effects become longstanding 'locked' processes (e.g., Parkinsons). Electrostimulatory activity can help to break chronic cycles to allow previously 'healthy' patterns of synaptic activity to become reestablished.

Electrostimulatory influences may have small local effects *(i.e.* effects in the damaged nerve) in the form of polarisation of molecules and local vasomotor effects; with some possible influence on the graded potentials locally. Mediation of local influences is via neuropeptide release.

The majority of the beneficial influence of electrical stimulation is via efferent nerves from the CNS. At a segmental level, there is also sometimes influence on pain pathways via the saturation of transmitter at the site of entry into the lateral spinothalamic tract, particularly if there is marked A fibre involvement.

Electrostimulation signals act on both local reflex arcs (also influencing the sympathetic chain) with their concomitant effects on internal organ, vessels and muscles; as well as entering the CNS via the ascending tracts for higher connections which will lead to general neuropeptide release (with resultant effect on general homoeostasis), endocrine release, parasympathetic influence and efferent signals down the corticospinal tracts to the relevant levels. Processes of disease control and pain with this form of electrostimulation are mainly mediated via the descending impulses in the CNS to an appropriate level for subsequent peripheral 'local' neuropeptide release. Further mediation is influenced through the autonomic nervous system both via local effects and general physiology.

At the end of a treatment round, a protocol of 'frequency modulation' can be selected. This is discussed above in terms of a fluctuating frequency cycle. In this process, the device output signal can be asked to cycle through a range of frequencies as described above, such as from about 15 to about 351 Hz in about an 8 second cycle. The primary value of this process is believed to be based on the fact that the 'end' of a biofeedback treatment at a specific point on the skin leads to a prolongation of the wave pulse as part of a specific communication loop via either a reflex arc or a spino-cerebral path. This 'dominant' communication is the more significant part of the healing stimulus. But there are other associated communication paths, which are accessory to the focal process, or are linked to previous pathology, which are affected by the dominant process. These accessory pathways may function at different frequencies and cannot simultaneously be addressed by the major waveform during the biofeedback process. Again, without wishing to be bound by any theory, it is believed that by cycling through the frequency range these other accessory networks are reached.

A method of promoting reinnervation of a damaged nerve in a patient through the patient's skin, comprises the steps of (a) electrically stimulating the skin of the patient at different location from the site of damage to the nerve; (b) detecting changes in the skin impedance and generating output signals representing the skin impedance; (c) monitoring the responsivity of the skin; and (d) indicating firstly when a predetermined level of responsivity is reached and secondly when a predetermined treatment has been administered.

A method of promoting reinnervation of a damaged nerve in a patient through the patient's skin, comprises the steps of: (a) placing a pair of electrodes in contact with the skin at different location from the site of damage to the nerve; (b) electrically stimulating the skin of the patient through the electrodes; (c) detecting changes in the skin impedance and generating output signals representing the skin impedance; (d) monitoring the responsivity of the skin; and (e) indicating firstly when a predetermined level of responsivity is reached and secondly when a predetermined treatment has been administered.

In an alternative method as discussed above, the skin is electrically stimulated at indicated positions *(i.e.* sites) sequentially. The skin biofeedback is measured and recorded, for example by measuring skin impedance and its rate of change. This is performed for a number of adjacent sites, and the site having the strongest biofeedback, such as highest rate of change of skin impedance or the site having a local greatest difference of skin impedance or the site having a skin impedance rate of change higher than adjacent sites, is treated further by electrical stimulation with the same initial duration and same initial amplitude, for a defined period of from about 30 second to about 5 minutes and, optionally, until the skin impedance does not change anymore. This is also sometimes referred to as the "numerical technique", without such term necessarily implying a strictly quantitative approach being taken. For example, the user interface of the device used does not have to be numerical in a literal sense; it suffices if such user interface allows a qualitative comparison of skin impedances or of skin impedance rates of change at different sites.

### Device

The skin is typically electrically stimulated using a device having electrodes. Any device known in the art may be used. The device is in one embodiment a low intensity device. The device in one embodiment produces a series of alternating current (AC) signals or impulses. The signals or impulses are typically produced in a particular waveform. Any waveform may be used in embodiments of the invention. The device in one embodiment produces an analogue nerve impulse waveform, an asymmetric waveform or a biphasic waveform. The device in one embodiment produces a degenerate waveform. The device in one embodiment detects the skin's electrical impedance and automatically adjusts its output accordingly.

The skin impedance alterations, which occur as a result of both the local and general state, may, for example, be depicted numerically on the device and influence the next outgoing signal from the device. Alternatively, the skin impedance alterations may also be represented by the device through audible or otherwise detectable signals, or both audible and visual signals. Moreover, several other aspects of the signal exchange between the skin and the device may be depicted numerically on the screen of the device (amplitude, rate, gradient, speed and so on) or may be represented by audible or other detectable signals. Some of these numbers use mathematical algorithms to be able to generate the best possible use of the electrostimulatory dialogue. The numerical or other representations are used by the practitioner to guide the treatment processes, via a number of protocols. The intention is to guide the locked or disturbed CNS foci into a restorative state; thereby initiating or re-stimulating normal repair processes; both centrally and locally. Due to the strong CNS (*versus* local) component of the process of exchange, 'old' foci from previous pathological states can be influenced simultaneously, leading to unexpected final resolution of past disease states.

A device suitable to be used in embodiments of the invention has, for example, been described in WO 2005/118061. Such a device comprises, for example, a pair of electrodes for contact with the skin; a waveform generator for repeatedly generating an AC waveform for applying electrical impulses through the electrodes to the skin; a detector for detecting changes in the skin impedance and for generating output signals representing a skin impedance; means responsive to the output signals from the detector for monitoring the responsivity of the skin; and indicator means for generating a first indication when a predetermined level of responsivity is reached and a second indication when a predetermined treatment has been administered. In this device, the skin impedance alterations which occur as a result of changes both in the local and the general state are depicted numerically on a screen of the device and influence the next outgoing signal from the device. Moreover, several other aspects of the signal exchange between the skin and the treatment device may be depicted numerically on the screen, such as the amplitude, the rate, the gradient, the speed etc. Some of these numbers use mathematical algorithms to be able to generate the best possible use of the electro stimulatory dialog. The numerical representations may then be used by the practitioner to guide the treatment processes via a number of protocols. The intention is to guide the locked or disturbed centres within the central nervous system, which are herein also sometimes referred to as CNS foci into a restorative state, thereby initiating or re-stimulating normal repair processes, both centrally and locally. Due to the strong CNS (versus local) component of the process of dialog exchange, "old" foci from previous pathological states can be influenced simultaneously, leading also to unexpected resolutions of past disease states. The device may be embodied in the form of a handheld battery-powered device.

In in one embodiment, the detection-means generates output signals in the form of pulses whose duration represents the skin impedance; the monitoring means measures the duration t of each pulse; and the indicating means is arranged to generate each indication when t satisfy a predetermined function oft. The indicating means is in one embodiment arranged to generate the first indication when t₂ = 4.087 t₁^{0.7131} and to generate the second indication when dZ/bT = 0, wherein Z = skin impedance. The electrical impulses generated by the handheld device are typically of high initial amplitude and brief duration as described above. The resulting treatment is non-invasive and does not generate harmful side-effects.

The device may be embodied in the form of a handheld battery-powered device.

The skin may be electrically stimulated using any device where the output signal depends on skin electrical properties completing the circuit whether the site for application is found manually by a practitioner or is 'found' automatically by means of an array of electrodes where electronic programming is used to assess dialogue between pairs of electrodes such that the focus for treatment can be targeted around those showing best response. The device may even be a wearable device.

The device may be used in any of the ways discussed above.

### Combination therapy

The electrical stimulation of the skin may be administered in combination with one or more other therapies intended to reinnervate the damaged nerve. A combination means that the therapies may be administered simultaneously to the patient. The therapies may be administered separately or sequentially, in either order, to a patient as part of the same therapeutic regimen. For example, electrical stimulation may be used in combination with another therapy intended to reinnervate the damaged nerve. The other therapy may be a general therapy aimed at treating or improving the condition of the patient. For example, treatment with methotrexate, glucocorticoids, salicylates, nonsteroidal anti-inflammatory drugs (NSAIDs), analgesics, other DMARDs, aminosalicylates, corticosteroids, and/or immunomodulatory agents (e.g., 6-mercaptopurine and azathioprine) may be combined with the electrical stimulation. The other therapy may be a specific treatment directed at the damage suffered by the patient, or directed at a particular symptom of the damaged. In one embodiment substances and compositions for use in combination with the electrical stimulation comprise a scaffold which promotes reinnervation, a graft which promotes reinnervation, a population of cells which promotes reinnervation, or a pharmaceutical which promotes reinnervation. These are discussed in more detail below. The pharmaceutical is in one embodiment an inhibitor of Nogo-66 signalling.

### Plurality of electrons

An embodiment of the invention also provides a plurality of electrons for use in a method of promoting reinnervation of a damaged nerve in a patient, wherein the plurality of electrons are administered to the skin of the patient at a different location from the site of damage to the nerve. The plurality of electrons may be administered in any of the forms discussed above, such as a series of signals or impulses or a waveform as discussed above. The plurality of electrons may be administered at any of the sites or locations discussed above. The plurality of electrons may be administered in any of the manners discussed above.

### Clinical results

The following clinical results were obtained on wounds in the arm of patients with the device as described in WO2005/118061 used to apply the electrical stimulation to the arm of the patents. A degenerative waveform was used for the electrical stimualtion.

**Figure 1****:** Higher re-innervation and neuropeptide synthesis in ES-treated compared to normal healing wounds, (a) qRT-PCR of PGP9.5 showed up-regulation in ES-treated wounds on day 14. (b) IHC analysis of PGP9.5 expression in normal skin (NS; day 0), C14 (spontaneous healing on day 14) and ES14 (electrically stimulated healing on day 14) wounds, (c) IHC showed ~ 44% increase in PGP9.5⁺ cells in ES14 compared to C14 wounds, (d) Western blotting of PGP9.5 showed up-regulation in ES14 wounds, (e) Substance P (SP) expression in normal skin, and a representative image of SP expression on day 14. Dotted lines separate epidermis from dermis. (f) IHC showed ~ 34% increase in SP⁺ cells in ES14 compared to C14 wounds. This also supports enhancement of cutaneous re-innervation by ES. (g) Western blotting also showed up-regulation of SP post-ES.

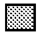 normal healing (control) wounds, 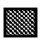 ES-treated wounds. Scale bar is 100 µm. 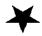 Indicates statistical significance.

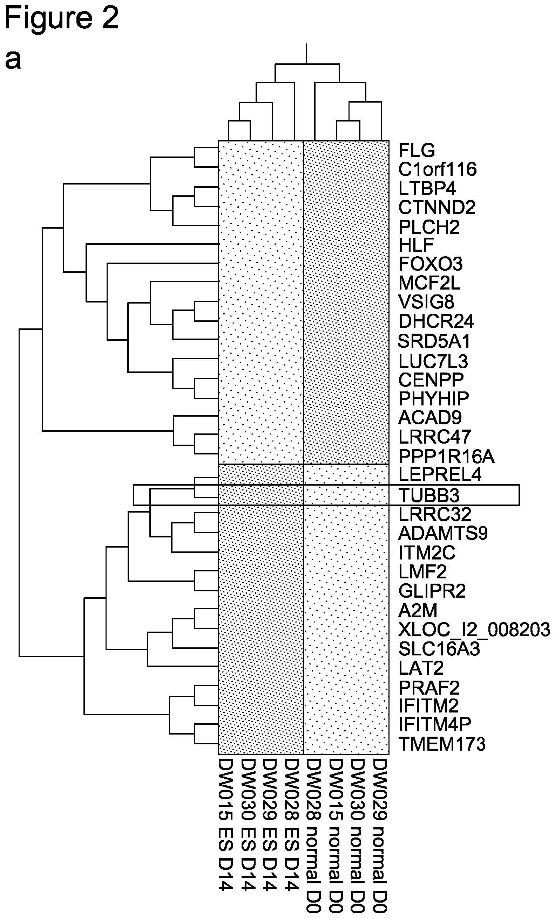

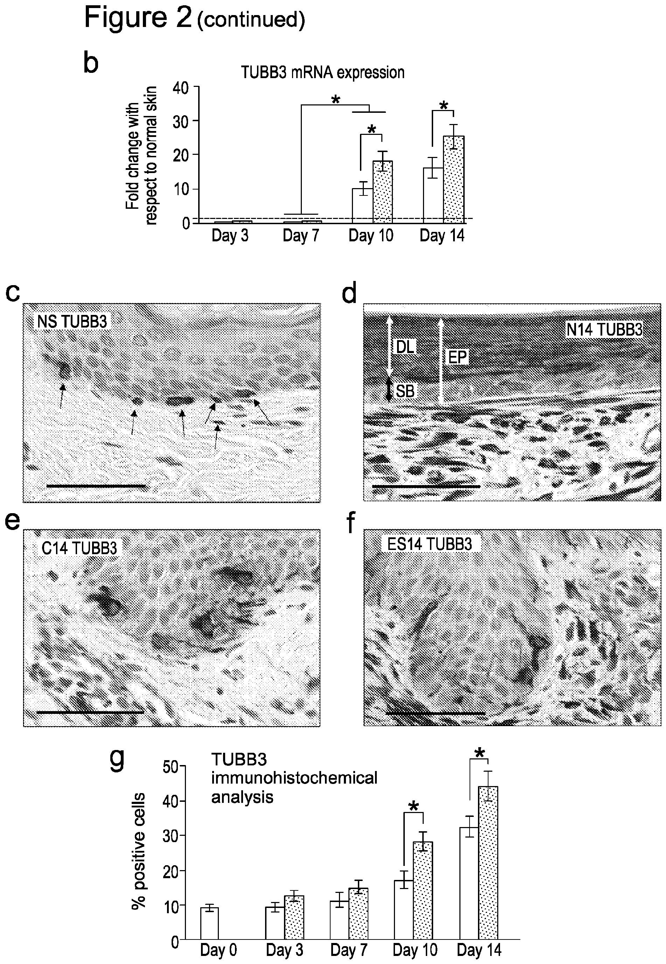

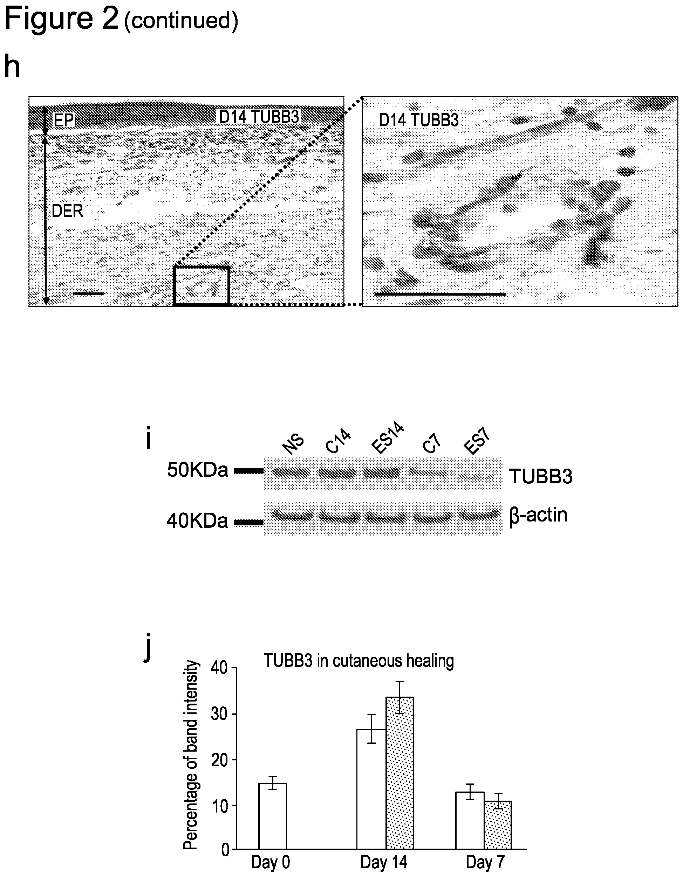

**Figure 2****:** Increased TUBB3 expression post-ES in healing human skin *in vivo.* (a) Microarray analysis of ES14 and normal healthy skin shows up-regulation of TUBB3. ES D14 and Normal D0 are wounds treated with ES till day 14 and normal healthy skin respectively, (b) qRT-PCR of TUBB3 showed ~ 70 and 28% increase in ES-treated wounds compared to normal healing, on days 10 and 14 respectively, (c) TUBB3 protein expression in normal skin. (d) TUBB3 expression in the wound centre (a representative image of healing on day 14). (e) TUBB3 expression in C14 and (f) ES14 wounds, (g) quantitative immunohistochemical analysis showed ~ 67% and 34% more cells were TUBB3⁺in ES10 and ES14 respectively, compared to the corresponding normal healing wounds (C10 and C14). (h) TUBB3 expression in the wound dermal compartment, especially staining neo-capillaries (a representative image of healing on day 14). (i) Western blotting of TUBB3 on days 10 and 14. (j) Analysis of TUBB3 Western blot band intensity. EP is epidermis, DER is dermis, DL is differentiating epidermal layers, SB is stratum basale, NS is normal healthy skin (Day 0), 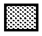 normal healing (control) wounds, 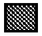 ES-treated wounds. Scale bar is 100 µm.

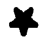 Indicates statistical significance.

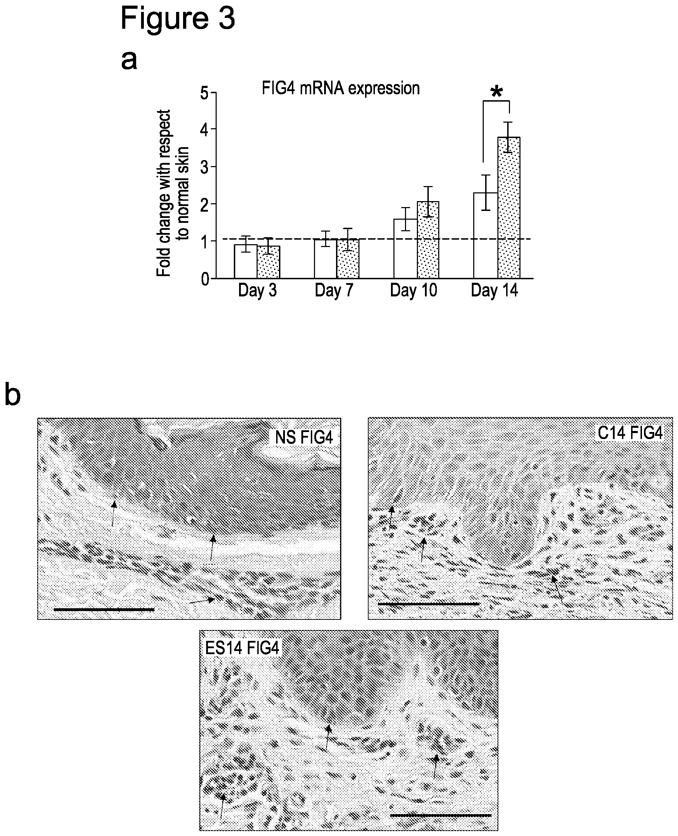

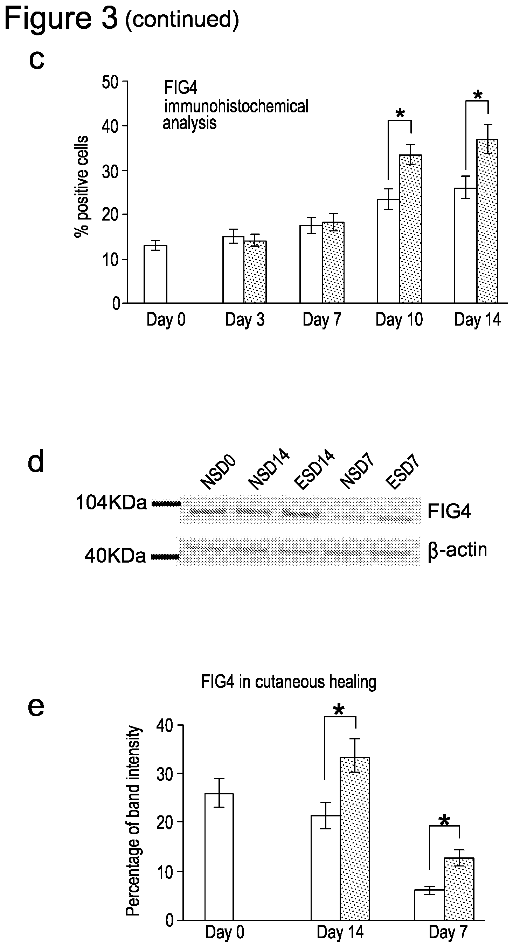

**Figure 3****:** ES enhances FIG4 expression in acute skin wounds, (a) qRT-PCR of FIG4 showed ~ 70% increase in ES-treated wounds compared to normal healing on day 14. (b) FIG4 protein expression in normal skin, C14 wounds, and ES14 wounds, (c) quantitative immunohistochemical analysis showed ~ 40% more cells were FIG4⁺ in ES10 and ES14 respectively, compared to the corresponding normal healing wounds (C10 and C14). (d) Western blotting of FIG4 on days 10 and 14. (e) Analysis of FIG4 Western blot band intensities showed ~ 110% and ~ 50% increase in ES7 and ES14 respectively, compared to the corresponding normal healing wounds (C7 and C14). NS is normal healthy skin (Day 0), 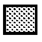 normal healing (control) wounds, 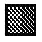 ES-treated wounds. Scale bar is 100 µm.

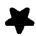 Indicates statistical significance.

**Figure 4****:** ES expands the pool of melanocytes and promotes melanogenesis in healing human skin *in vivo.* (a) Quantitative analysis of gp100⁺ cells in healing human skin. (b) Co-expression of TUBB3/gp100 (merged images) showed up-regulation of TUBB3⁺/gp100⁺ cells in the epidermis of ES14 treated wounds. Dotted lines separate epidermis from dermis. (c) Quantitation of TUBB3⁺/gp100⁺ cells in healing wounds, (**d**) Melanogenesis analysis by Masson-Fontana histochemistry was mainly observed in basal layer cells of human epidermis and was up-regulated in ES-treated wounds, (e) Quantitative analysis of melanogenesis by Masson-Fontana histochemistry. NS is normal healthy skin (Day 0), 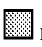 normal healing (control) wounds, 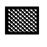 ES-treated wounds. Scale bar is 100 µm.

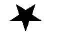 Indicates statistical significance.

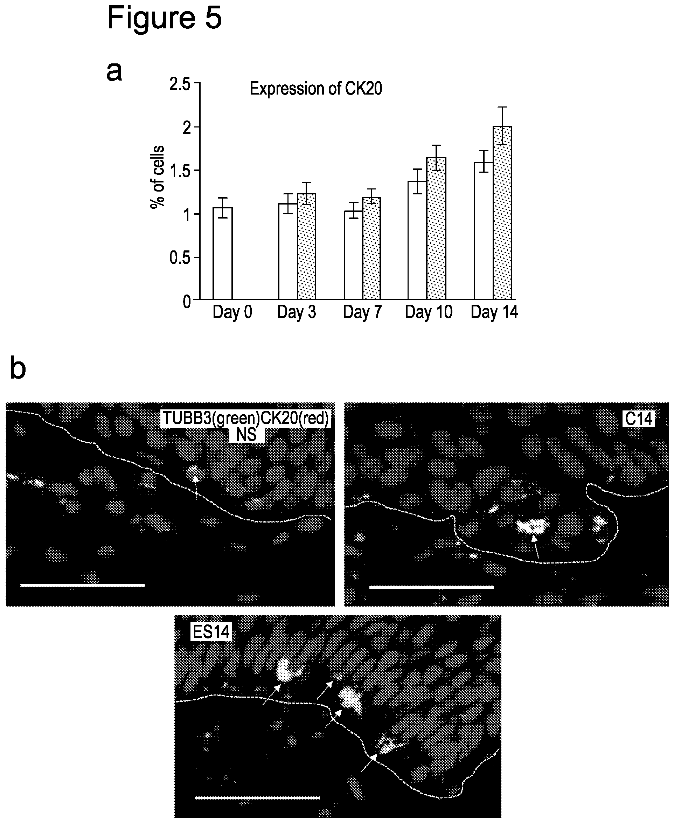

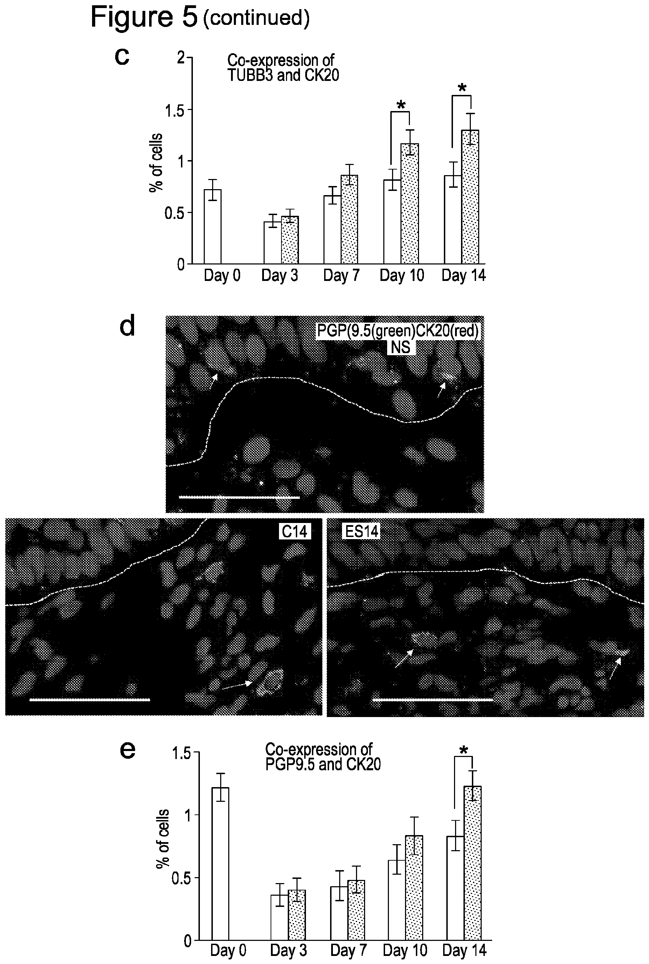

**Figure 5****:** ES up-regulates TUBB3 and PGP9.5 expressions in Merkel cells in healing human skin *in vivo.* (a) Quantitative analysis of CK20⁺ cells in healing human skin. (**b**) Co-expression of TUBB3/CK20 (merged images) showed up-regulation of TUBB3⁺/CK20⁺ cells in the epidermis of ES14 wounds, (c) Quantitation of TUBB3⁺/CK20⁺ cells in healing wounds, (**d**) Re-innervation as shown with co-expression of PGP9.5/CK20 (merged images), (**e**) Quantitation of PGP9.5⁺/CK20⁺ cells. ES14 wounds showed significantly higher number of PGP9.5⁺/CK20⁺ cells than C14 wounds. NS is normal healthy skin (Day 0), 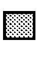 normal healing (control) wounds,

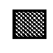 ES-treated wounds. Scale bar is 100 µm. Dotted lines separate epidermis from dermis.

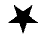 Indicates statistical significance.

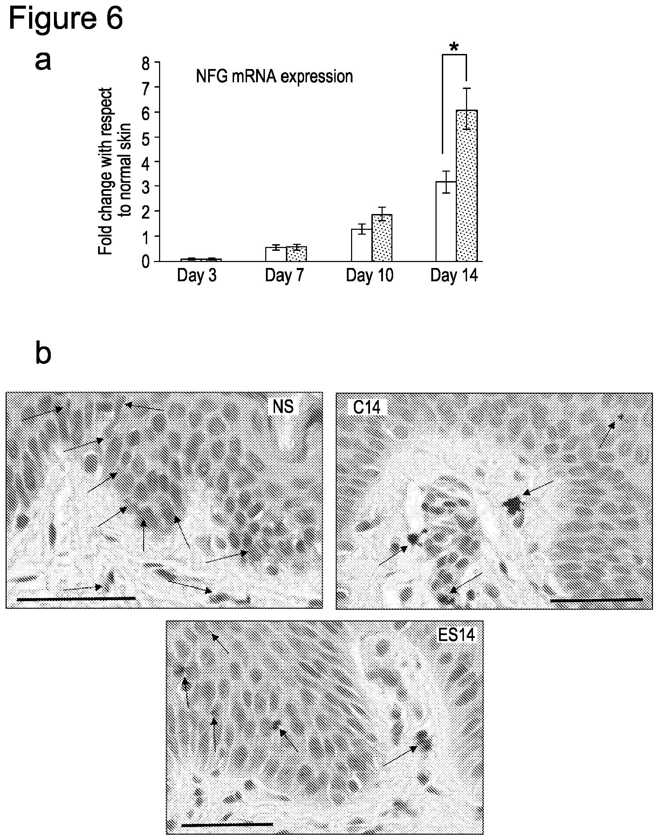

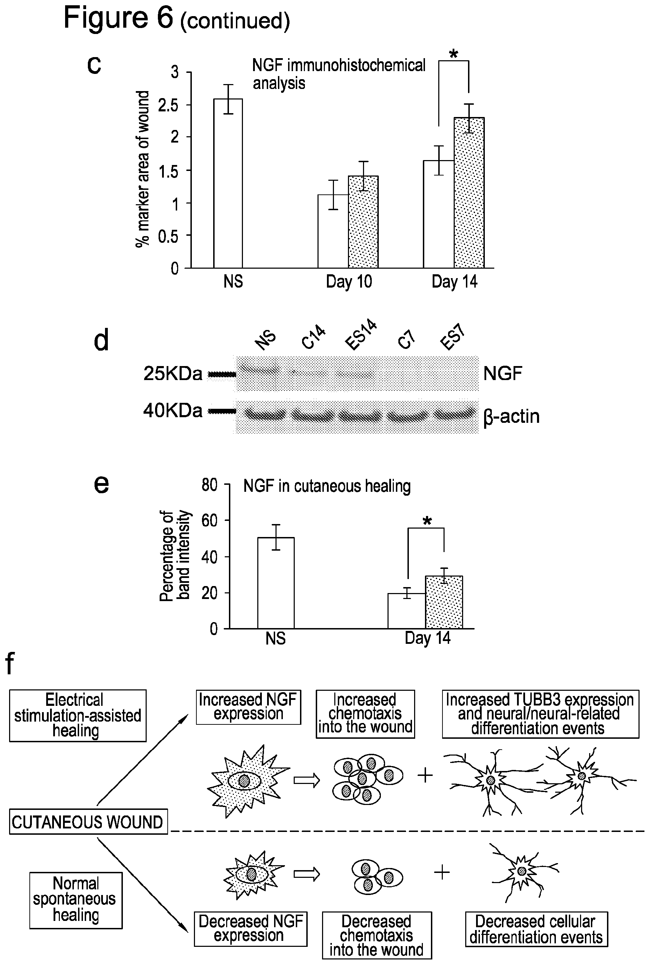

**Figure 6****:** Unaltered proliferation of melanocytes and Merkel cells, and up-regulated NGF expression by ES. (a) qRT-PCR of NGF showed ~ 1.46 and 1.91 fold increase in ES-treated wounds compared to normal healing wounds, on days 10 and 14 respectively, (**b**) NGF protein expression in normal skin, C14 and ES14 wounds, (**c**) quantitative analysis of IHC showed 1.25 and 1.39 fold increase in ES10 and ES14 wounds, compared to C10 and C14 wounds, respectively, (**d**) Western blot analysis of NGF in normal skin and wound healing wounds, (**e**) Quantitative analysis of Western blot showed up-regulation of NGF in ES14 wounds compared to C14 wounds, (**f**) Schematic representation of the up-stream re-innervation events in ES-treated wounds and normal healing wounds. Neurotrophin (NGF) up-regulation in ES-treated wounds leads to higher chemotaxis as well as neural/neural-related cellular differentiation, which results in a higher magnitude of tissue re-innervation. NS is normal healthy skin (Day 0), 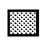 normal healing (control) wounds, 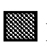 ES-treated wounds. Scale bar is 100 µm.

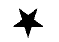 Indicates statistical significance.

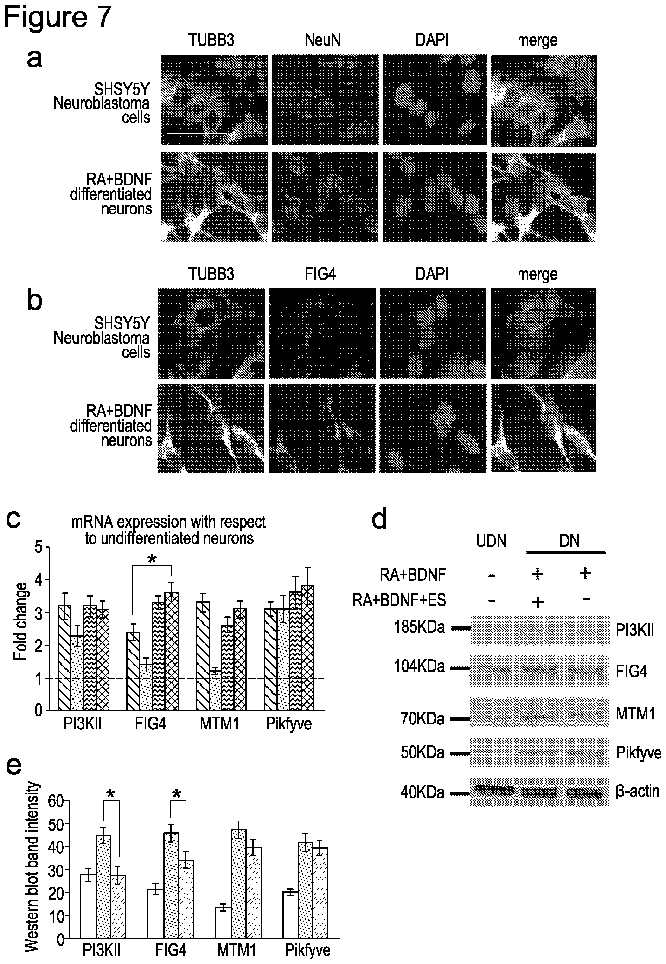

**Figure 7****:** Application of ES enhances human SHSY5Y neuroblastoma cell differentiation, (a) Neuroblastoma cells were differentiated by 'retinoic acid (RA) and brain-derived neurotrophic factor (BDNF)' treatment and the differentiated cells showed up-regulated expression of NeuN. NeuN is a multiply phosphorylated antigen, whose concentration in the nuclei is inversely proportional to chromatin (DNA). (b) FIG4 was also up-regulated post-RA+BDNF treatment, (c) qRT-PCR showed up-regulated expression of FIG4 post-RA+BDNF+ES treatment compared to RA+BDNF treatment. Dotted line represents undifferentiated SHSY5Y neuroblastoma cells, (d) Western blot analysis of PIP3 synthesis pathway, (e) Quantitation of WB bands showed significant increase in PI3KII and FIG4 protein expressions post-ES. UDN is undifferentiated neurons and DN is differentiated neurons. Scale bar is 50 µm.

| | |
|---|---|
| 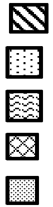 | RA+BDNF treated cells for10 days. |
| | RA+BDNF+ES treated cells for 6 days. |
| | RA+BDNF+ES treated cells for 8 days. |
| | RA+BDNF+ES treated cells for 10 days. |
| | Undifferentiated neurons, 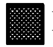 RA+BDNF+ES treated cells, 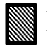 RA+BDNF treated |
| cells | |

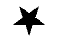 Indicates statistical significance.

**Figure 8****:** ES enhances human SHSY5Y neuroblastoma cell differentiation post-FIG4 siRNA treatment, (a) LAMP2 expression indicating late endosome formation in neuroblastoma cells in the absence and post-FIG4 treatment. LAMP2 is a marker for late endosome stage, further leading to vacuole formation, (b) Cells subjected to RA+BDNF+ES post-FIG4 siRNA treatment had higher NeuN expression compared to control cells which were not exposed to ES. (**c**) FIG4 was also up-regulated post-ES treatment compared to control cells, (**d**) LAMP2 was decreased in cells subjected to ES post-FIG4 siRNA treatment compared to control cells which were not exposed to ES. (**e**) mRNA expression showed higher FIG4 levels in RA+BDNF+ES post-FIG4 siRNA treatment compared to cells not exposed to ES. Dotted line represents undifferentiated SHSY5Y neuroblastoma cells post FIG4 siRNA treatment, (**f**) Western blot analysis of PIP3 synthesis pathway post-FIG4 siRNA treatment, (**g**) Quantitation of WB bands showed significant increase in FIG4 protein expression post-RA+BDNF+ES treatment compared to control cells which were not exposed to ES. Scale bar is 50 µm.

| | |
|---|---|
| 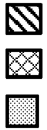 | RA+BDNF treated cells post FIG4 siRNA treatment. |
| | RA+BDNF+ES treated cells post FIG4 siRNA treatment. |
| | FIG4 siRNA treated cells, 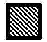 RA+BDNF+ES treated cells post-FIG4 siRNA treatment. |
| 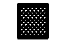 | RA+BDNF treated cells post-FIG4 siRNA treatment. |
| 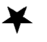 | Indicates statistical significance. |

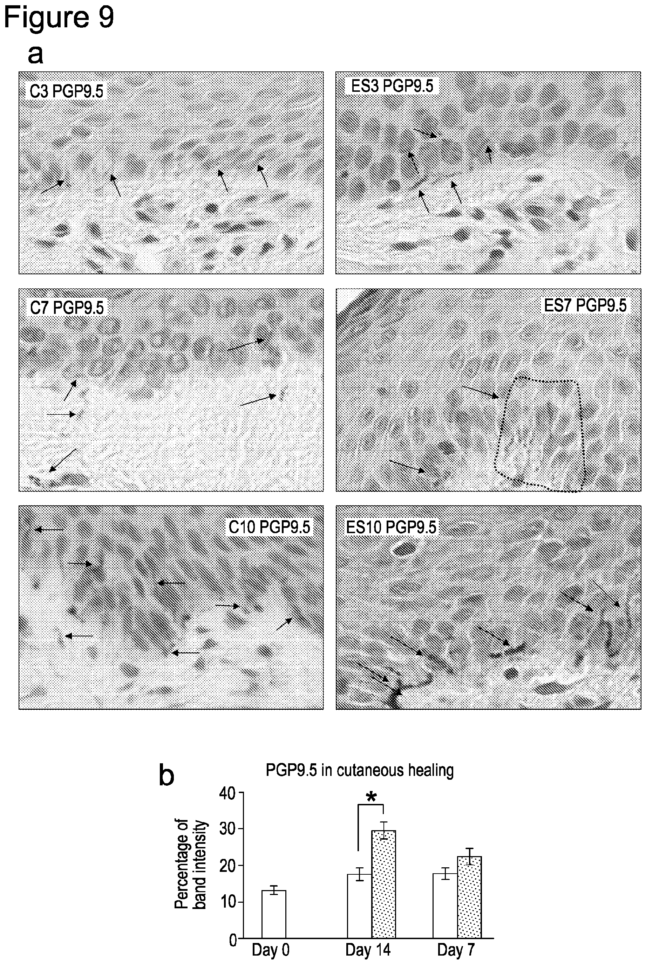

**Figure 9****:** PGP9.5 (re-innervation analysis) in human cutaneous wounds, (**a**) IHC of PGP9.5 expression. In the epidermis, weaker PGP9.5 signals were detected initially on healing day 3, which got stronger by day 14. "C" denotes spontaneously healing wounds and "ES" denotes electrically stimulated healing wounds. (**b**) Quantitation of Western blot band intensities for PGP9.5 showed higher protein expression in ES14 wounds compared to C14 wounds. "ES" denotes electrically stimulated healing wounds. (**b**) Quantitation of Western blot band intensities for PGP9.5 showed higher protein expression in ES14 wounds compared to C14 wounds. Key as for Figure 1.

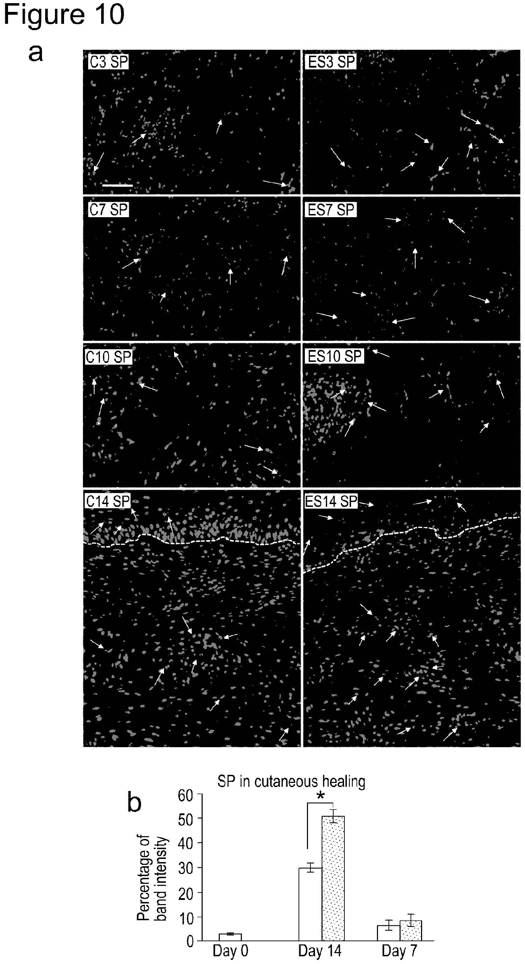

**Figure 10****:** Substance P (neuropeptide expression) in human cutaneous wounds, (a) IHC of SP expression. Similar to PGP9.5 expression, expression of SP was weak till day 10 in the epidermis of healing wounds, (b) Quantitation of Western blot band intensities for SP showed higher protein expression in ES14 wounds compared to C14 wounds. Key as for Figure 1.

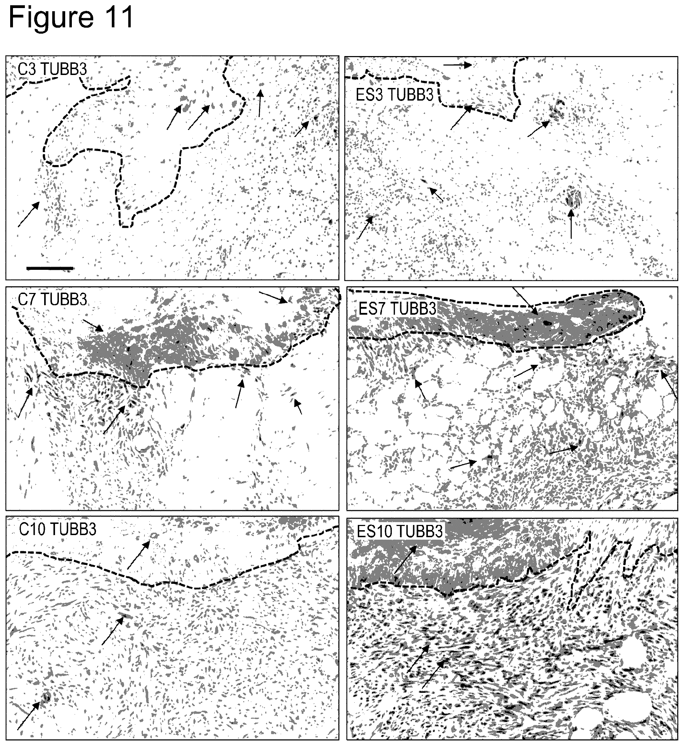

**Figure 11****:** IHC images of TUBB3 expression in healing wounds. Up-regulated TUBB3 expression was observed in ES-treated cutaneous wounds compared to normal spontaneous healing. In all images, dotted lines separate epidermis from dermis.

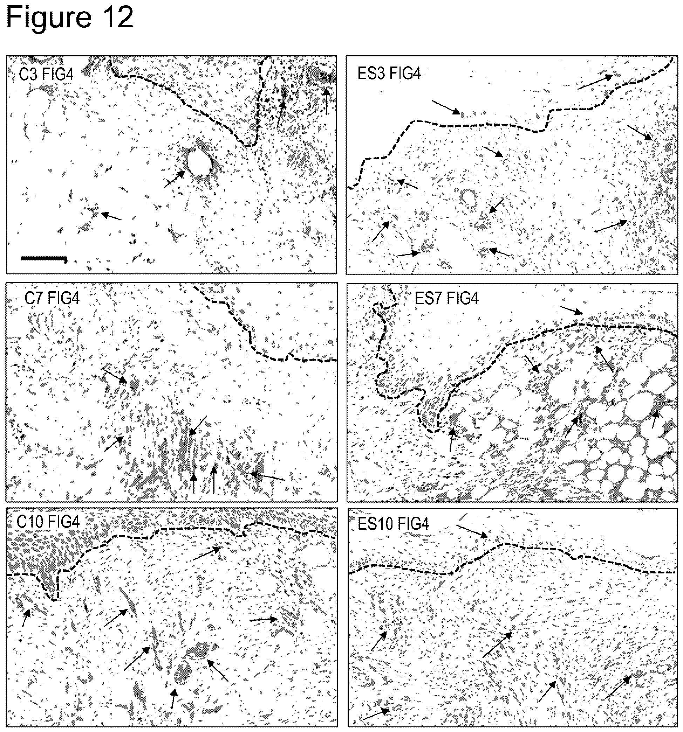

**Figure 12****:** IHC images of FIG4 expression in healing wounds. Up-regulated TUBB3 expression was observed in ES-treated cutaneous wounds compared to normal spontaneous healing. In all images, dotted lines separate epidermis from dermis.

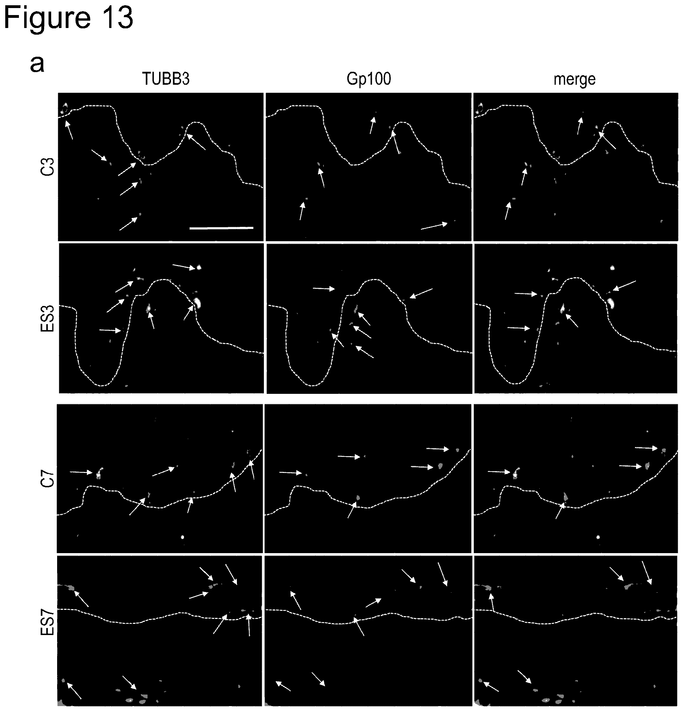

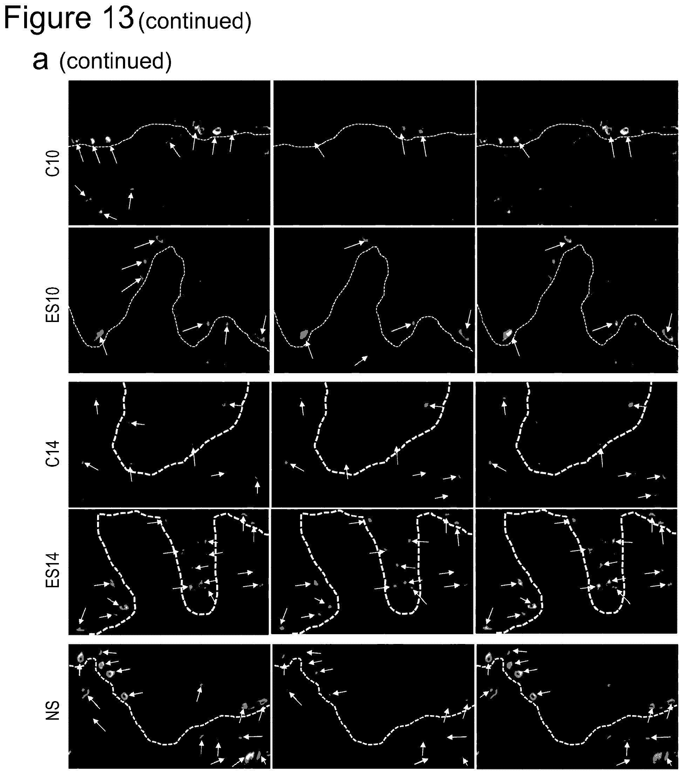

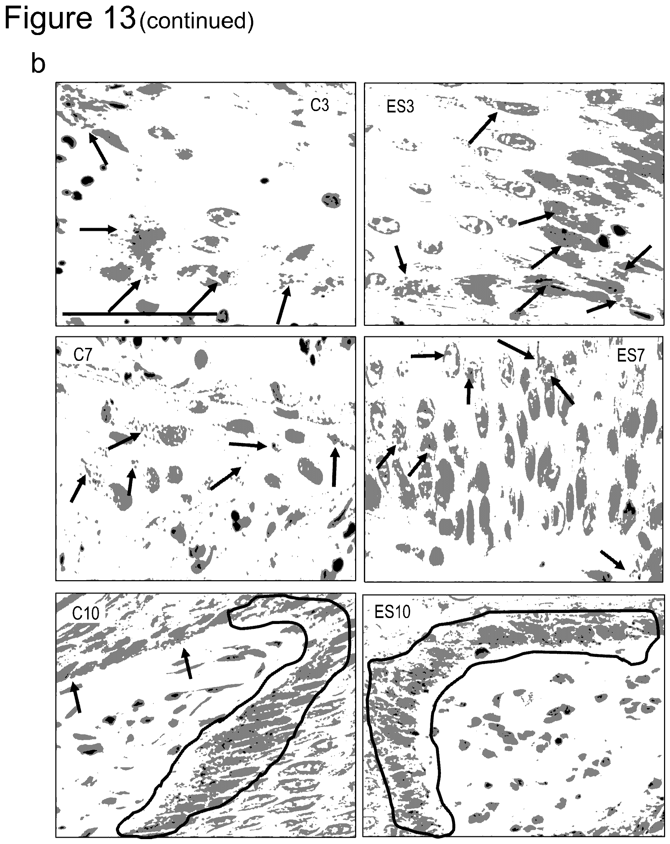

**Figure 13****:** ES up-regulates TUBB3 expression and enhances melanogenesis in healing human skin. (a) Co-expression of TUBB3/gp100 showed up-regulation of TUBB3+/gp100+ cells in the epidermis of ES14 wounds, (b) Melanogenesis analysis by Masson-Fontana histochemistry showed up-regulation in basal layer cells of healing human epidermis post-ES. NS is normal skin (Day 0). Scale bar is 100 µm. Key as for Figure 1.

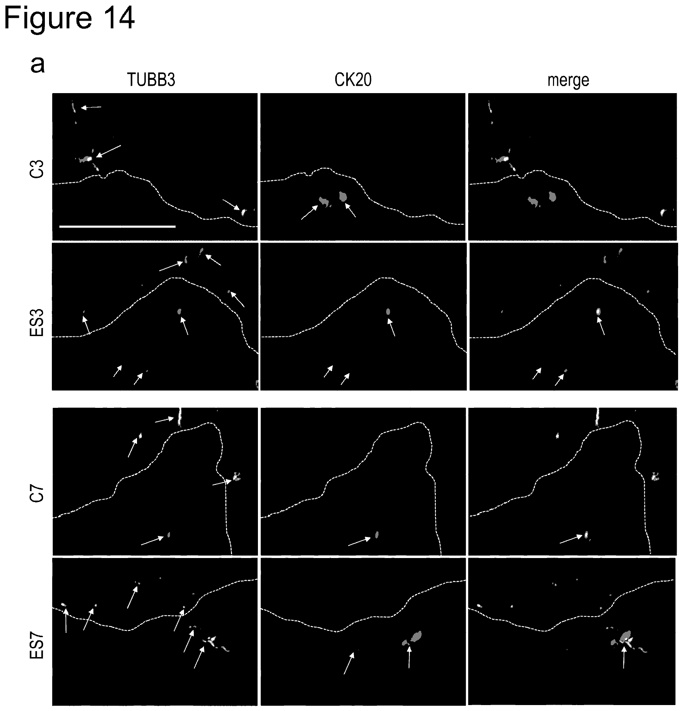

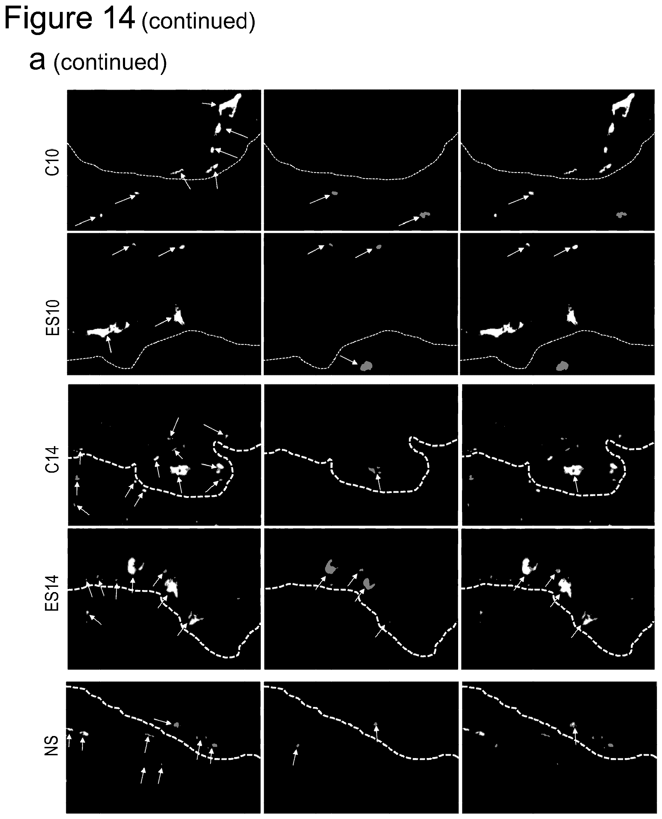

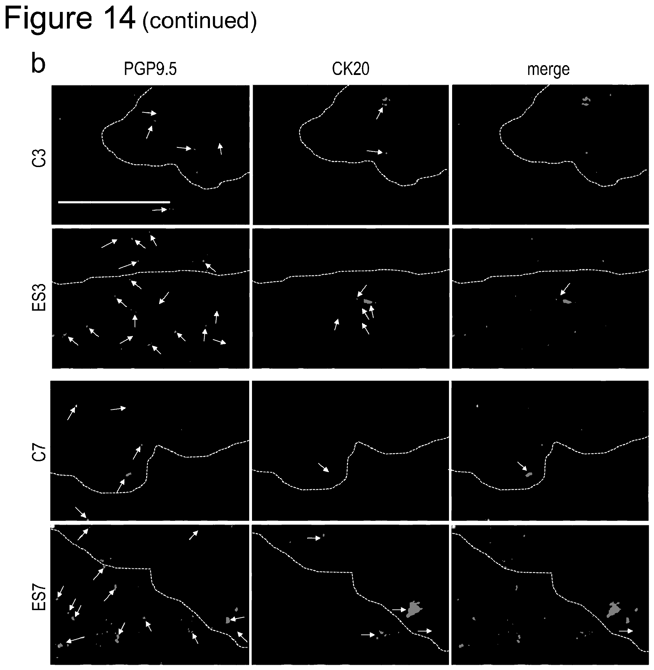

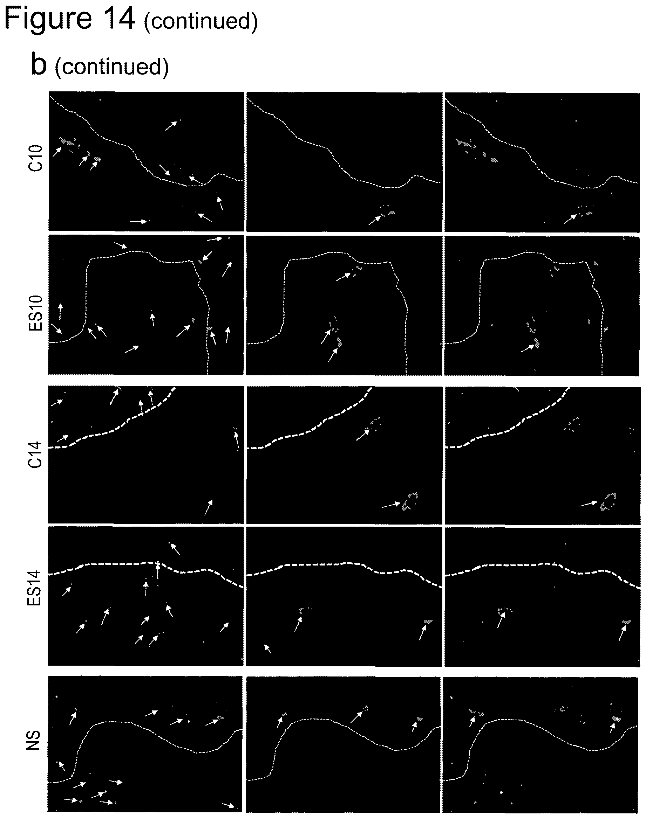

**Figure 14****:** Up-regulated TUBB3 and PGP9.5 expressions in Merkel cells post-ES treatment in human skin wounds, (a) IHC of co-expression of TUBB3/CK20 in human cutaneous healing undergoing ES treatment and normal healing wounds, (b) PGP9.5/CK20 co-expression images of ES treated and normal healing wounds. NS is normal skin (Day 0). Scale bar is 100 µm.

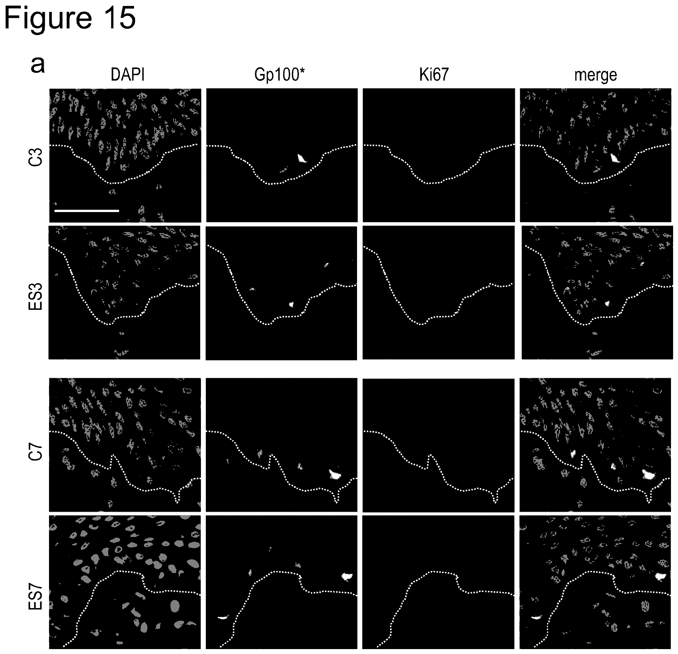

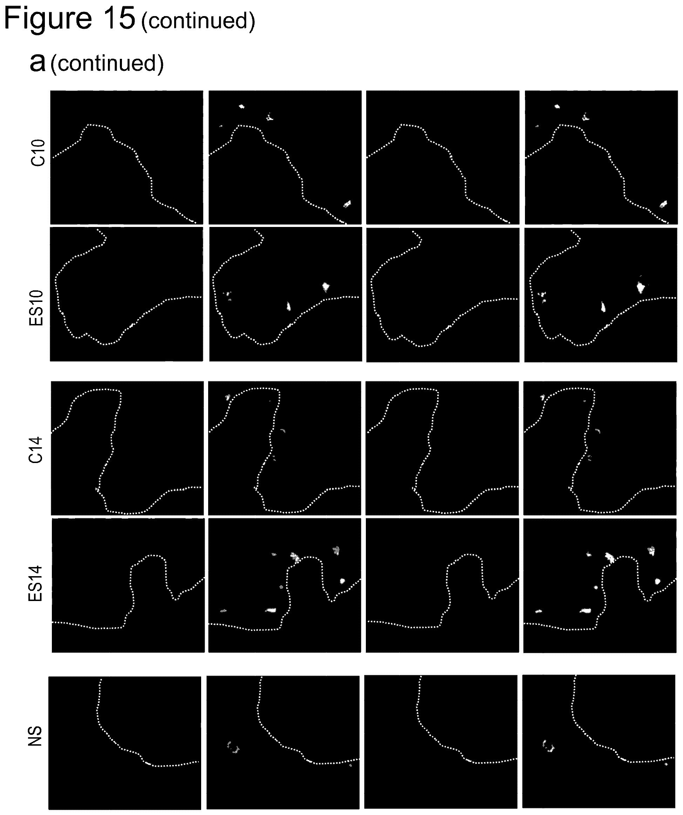

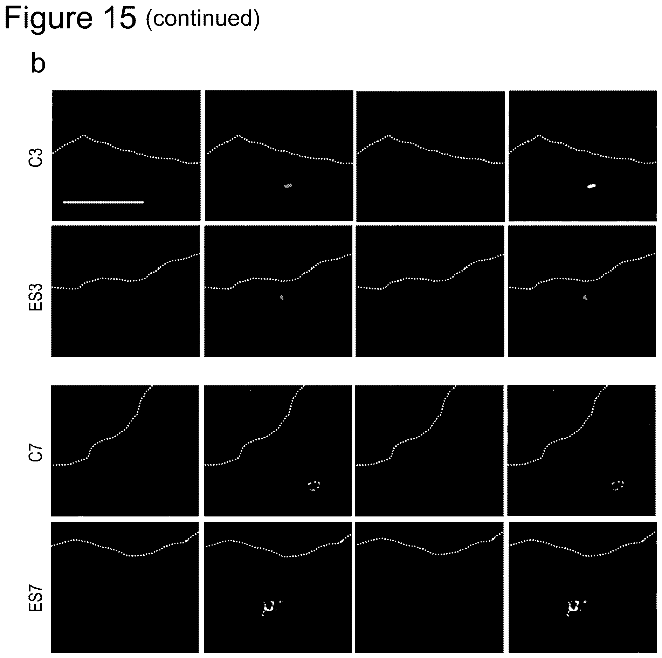

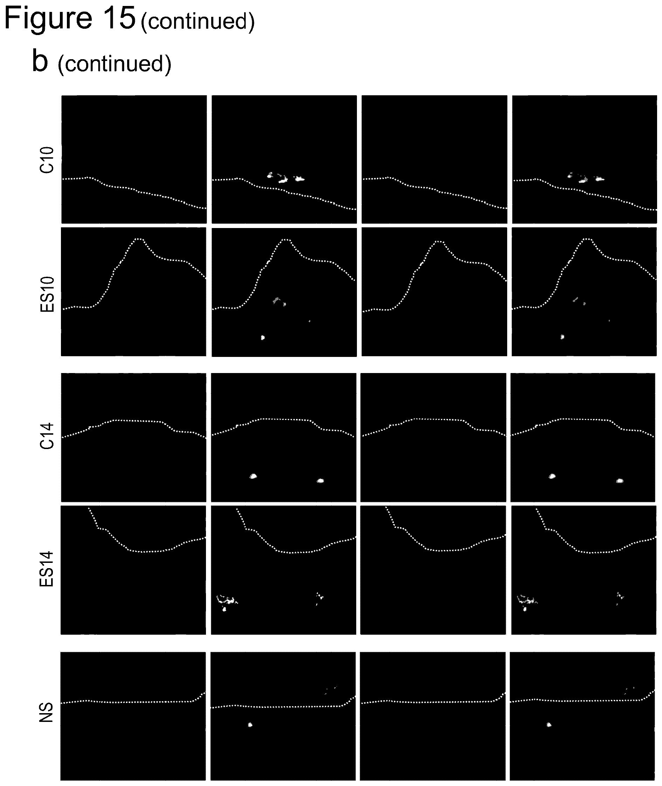

**Figure 15****:** Proliferation analysis of melanocytes and Merkel cells in human cutaneous healing normal healing wounds, (a) Ki67/gp100 and (b) Ki67/CK20 co-expression analyses showed no significant difference in cellular proliferation between ES-treated and normal healing wounds. NS is normal skin. Scale bar is 100 µm.

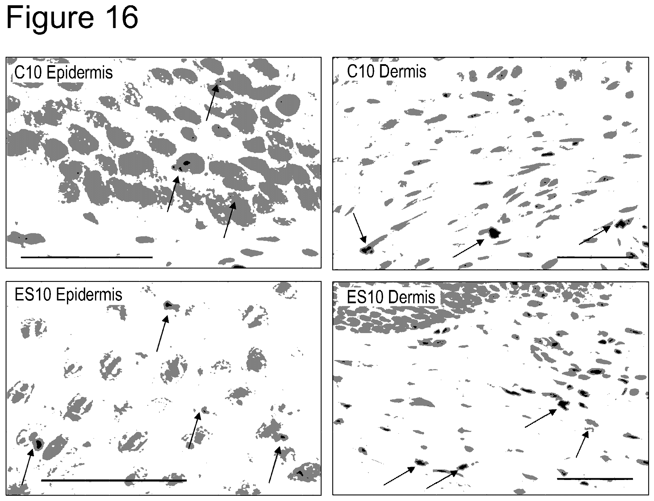

**Figure 16****:** Up-regulated NGF expression in ES-treated wounds compared to normal healing wounds in human skin. IHC of NGF on wounds of healing days 10 and 14. NGF expression level was significantly lower in all other healing days (before 10) for observation and further quantitation. Scale bar is 100 µm.

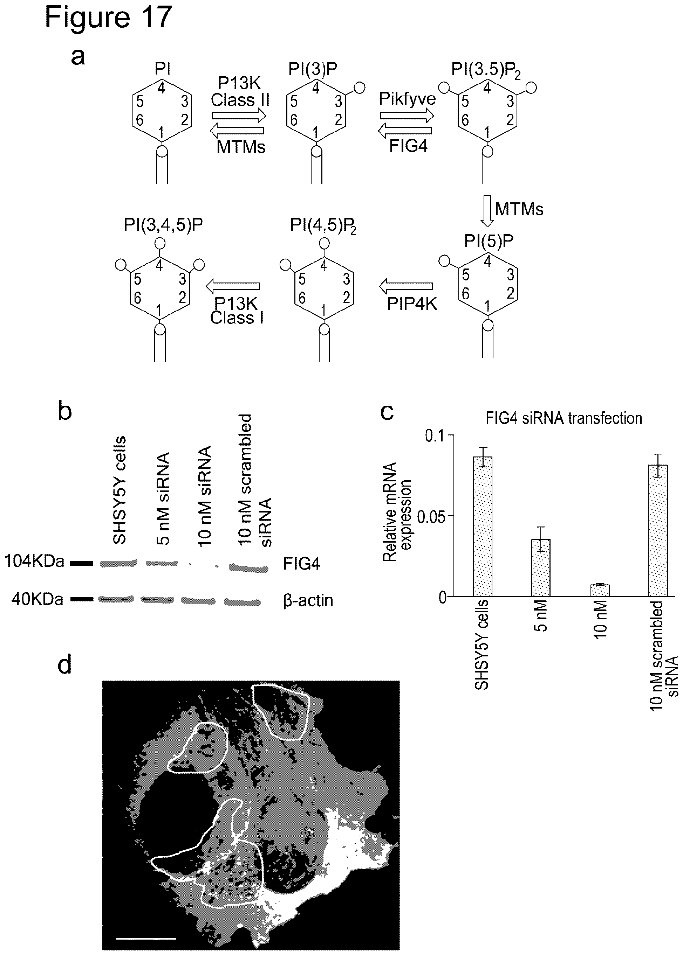

**Figure 17:** (a) PIP3 synthesis pathway, (b) SHSY5Y neuroblastoma cells were transfected with SIVA1 siRNA (5 nM and 10 nM). Ninety-six hours after transfection, proteins were extracted and subjected to Western blot analysis. Successful knock down was achieved at 10 nM concentration of the transfecting siRNA. (**c**) mRNA expression after SIVA1 gene knock down. (**d**) Vacuologenesis post-FIG4 siRNA treatment in undifferentiated SHSY5Y cells. Scale bar is 10 µm.

Although these clinical results show the efficacy of the treatment of nerve damage in wounds, the electrical stimulation method is not limited to reinnervation of damaged nerves in wounds. It is applicable to any form of nerve damage.

### Substances or compositions

A substance or composition can be provided for use in a method promoting reinnervation of a damaged nerve in a patient, wherein the method comprises administering the substance or composition to the patient in combination with electrical stimulation of the skin of the patient at a different location from the site of damage to the nerve.

Electrical stimulation of the skin of the patient is defined above and any of the ways discussed above are applicable. Combination therapy is also defined above.

The substance or composition is in one embodiment a scaffold, which promotes reinnervation, a graft which promotes reinnervation, a population of cells which promotes reinnervation or a pharmaceutical which promotes reinnervation.

Any scaffold, which promotes reinnervation, may be used. The scaffold may comprise one or more biocompatible fibres. A fibre is biocompatible if it does not cause any adverse reactions or side effects when contacted with a damaged tissue. Any number of biocompatible fibres may be present, such as more than one fibre, such at least 2, at least 5, at least 10, at least 20, at least 30, at least 40, at least 50, at least 100, at least 200, at least 500 fibres, at least 1000 fibres or even more fibres.

Suitable biocompatible fibres are known in the art. The one or more biocompatible fibres may be natural or synthetic. Biocompatible fibres can include, but are not limited to, cellulose fibres, collagen fibres, collagen-glycosaminoglycan fibres, gelatin fibres, silk fibroin fibres, one or more fibrin fibres, chitosan fibres, starch fibres, alginate fibres, hyaluronan fibres, poloaxmer fibres or a combination thereof. The glycosaminoglycan is in one embodiment chondroitin. The cellulose is in one embodiment carboxymethylcellulose, hydroxypropylmethylcellulose or methylcellulose. The poloaxmer is in one embodiment pluronic acid, optionally Pluronic F-127. If more than one fibre is present in the composition, the population of fibres may be homogenous. In other words, all of the fibres in the population may be the same type of fibre, e.g. cellulose fibres. Alternatively, the population of fibres may be heterogeneous. In other words, the population of fibres may contain different types of fibre, such cellulose fibres and collagen fibres.

The one or more fibres may be any length. The one or more fibres are in one embodiment approximately the same length as the depth of the damage, which is to be treated using the composition. The length of one or more fibres is in one embodiment designed such that the composition can penetrate a damaged tissue to a prescribed depth. The one or more fibres may be any length. The lower limit of the length of the one or more fibres is typically determined by the diameter of the one or more therapeutic cells. Suitable lengths include, but are not limited to, at least 1µm in length, at least 10µm in length, at least 100µm in length, at least 500µm in length, at least 1mm in length, at least 10mm (1cm) in length, at least 100mm (10cm) in length, at least 500mm (50cm) in length or at least 1000mm (100cm or 1m) in length. The one or more fibres may be even longer. For instance, the one or more fibres may be up to 5m or 10m in length, for instance if being used to repair damage along the human intestinal tract, or even longer if being used in larger animals, such as horses. The length of the one or more fibres is typically determined by their intended use and/or their ability to be manipulated, for instance by a surgeon, by a robot or via some other means, such as magnetically.

The scaffold may comprise a biocompatible adhesive which attaches the one or more therapeutic cells to the one or more fibres. The biocompatible adhesive may attach the one or more therapeutic cells (a) on the surface of the one or more fibres, (b) within the one or more fibres or (c) both on the surface of and within the one or more fibres.
The biocompatible adhesive may be natural or synthetic. Suitable biocompatible adhesives are known in the art. Suitable adhesives include, but are not limited to, fibrin, fibrin gel, integrin, integrin gel, cadherin and cadherin gel.

The scaffold may comprise a biocompatible gel. Suitable biocompatible gels are known in the art. The biocompatible gel may be natural or synthetic. Biocompatible gels can include, but are not limited to, a cellulose gel, a collagen gel, a gelatin gel, a fibrin gel, a chitosan gel, a starch gel, an alginate gel, a hyaluronan gel, an agarose gel, a poloaxmer gel or a combination thereof. The cellulose gel may be formed from any of the celluloses discussed above. The cellulose polymer concentration is in one embodiment from about 1.5% (w/w) to about 4.0% (w/w), such as from about 2.0% (w/w) to about 3.0% (w/w). The cellulose polymer in one embodiment has a molecular weight of from about 450,000 to about 4,000,000, such as from about 500,000 to about 3,500,000, from about 500,000 to about 3,000,000 or from about 750,000 to about 2,500,000 or from about 1000,000 to about 2,000,000.

The poloaxmer gel is in one embodiment a pluronic acid gel, optionally a Pluronic F-127 gel. The adhesive and/or gel in one embodiment has a viscosity in the range of 1000 to 500,000 mPa•s (cps) at room temperature, such as from about 1500 to about 450,000 mPa•s at room temperature, from about 2000 to about 400,000 mPa•s at room temperature, from about 2500 to about 350,000 mPa•s at room temperature, from about 5000 to about 300,000 mPa•s at room temperature, from about 10,000 to about 250,000 mPa•s at room temperature, from about 50,000 to about 200,000 mPa•s at room temperature or from about 50,000 to about 150,000 mPa•s at room temperature.

Viscosity is a measure of the resistance of the adhesive and/or gel to being deformed by either shear stress or tensile stress. Viscosity can be measured using any method known in the art. Suitable methods include, but are not limited to, using a viscometer or a rheometer.

Room temperature is typically from about 18°C to about 25 °C, such as from about 19 °C to about 24°C or from about 20 °C to about 23°C or from about 21 °C to about 22°C. Room temperature is in one embodiment any of 18°C, 19 °C, 20°C, 21 °C, 22°C, 23 °C, 24°C and 25 °C. Viscosity can be measured at 25°C.

In any of the embodiments discussed above, the biocompatible adhesive and/or the biocompatible gel in one embodiment comprises platelet lysate. For instance, the adhesive and/or the gel may be a platelet lysate gel. Platelet lysate refers to the combination of natural growth factors contained in platelets that has been released through lysing those platelets. Lysis can be accomplished through chemical means (i.e. CaCl₂), osmotic means (use of distilled H₂O) or through freezing/thawing procedures. Platelet lysate can be derived from whole blood as described in U.S. Pat. No. 5,198,357. Platelet lysate is in one embodiment prepared as described in PCT/GB12/052911 (published as WO 2013/076507). For instance, it may be prepared by subjecting a population of platelets to at least one freeze-thaw cycle, wherein the freeze portion of each cycle is carried out at a temperature lower than or equal to - 78°C. The platelet lysate is in one embodiment human platelet lysate. Platelet lysate is discussed in more detail below.

The graft is in one embodiment is a nerve graft, vein graft or alloplastic nerve graft. Suitable grafting techniques are known in the art. The graft may be an autograft, an allograft or a xeonugraft. Suitable alloplastic grafts include, but are not limited to, collagen conduits, silicon tubes, a polyglycolic acid tube, such as Neurotube^{®}, filaments and perineurium tubes.

The population of cells are in one embodiment a population of therapeutic cells. Therapeutic cells are cells which are capable of having a therapeutic effect. Therapeutic cells are typically living cells. Therapeutic cells are typically cells which are capable of repairing damaged tissue, such as nerves. The therapeutic cells are in one embodiment autologous. In other words, the cells are in one embodiment derived from the patient into which the cells will be administered. Alternatively, the cells are in one embodiment allogeneic. In other words, the cells are in one embodiment derived from a patient that is immunologically compatible with the patient into which the cells will be administered. The cells may be semi-allogeneic. Semi-allogeneic populations are typically produced from two or more patients that are immunologically compatible with the patient into which the cells will be administered. In other words, the cells are in one embodiment genetically identical with the patient into which they will be administered or sufficiently genetically identical that the cells are immunologically compatible with the patient into which they will be administered.

Any number of cells may be administered. The population typically comprises at least about 2, at least about 5, at least about 10, at least about 20, at least about 30, at least about 40, at least about 50, at least about 100, at least about 200, at least about 500, at least about 1000, at least about 2000, at least about 5000, at least about 10000, at least about 50000, at least about 100000, at least about 5 × 10⁵, at least about 1 × 10⁶, at least about 2 × 10⁶, at least about 5 × 10⁶, at least about 1 × 10⁷, at least about 2 × 10⁷, at least about 5 × 10⁷, at least about 1 × 10⁸ or at least about 2 × 10⁸ cells. In some instances, at least about 1.0 × 10⁷, at least about 1.0 × 10⁸, at least about 1.0 × 10⁹, at least about 1.0 × 10¹⁰, at least about 1.0 × 10¹¹ or at least about 1.0 × 10¹² cells or even more cells may be administered.

The population of cells may be homogenous. In other words, all of the cells in the population may be the same type of cell, e.g. mesenchymal stem cells (MSCs). Alternatively, the population of cells may be heterogeneous. In other words, the population of cells may contain different types of cells, such MSCs and progenitor cells of mesodermal lineage (PMLs).

The cells may be progenitor cells of mesodermal lineage (PMLs) disclosed in PCT/GB2012/051600 (published as WO 2013/005053).

The cells may be are mesenchymal stem cells (MSCs). Suitable MSCs are known in the art. Suitable MSCs are commercially available from Mesoblast^{®} Ltd, Osiris Therapeutics^{®} Inc. or Lonza^{®}. The human MSCs are in one embodiment obtained from Lonza^{®}.

The cells may be mesenchymal precursor cells (MPCs). The cells may be human mesenchymal precursor cells (MPCs).

The cells may be dendritic cells, platelets, fibroblasts or myofibroblasts.

The cells may be embryonic stem (ES) cells, such as human (ES) cells, or induced pluripotent stem cells (iPS cells), such as human iPS cells. Techniques for obtaining such cells are known in the art.

The cells are in one embodiment human.

The cells are typically cultured *in vitro* before being administered. Techniques for culturing cells are well known to a person skilled in the art. The cells may be cultured under standard conditions of 37°C, 5% CO2 in medium without serum.

The cells may be modified *ex vivo* using any known technique. For instance, the population may be transfected or loaded with therapeutic or diagnostics agents.

The pharmaceutical in one embodiment comprises a small molecule, a protein, an antibody, a polynucleotide, an oligonucleotide, an antisense RNA, small interfering RNA (siRNA) or small hairpin RNA (shRNA). The pharmaceutical is in one embodiment part of a pharmaceutical composition comprising a pharmaceutically acceptable carrier or diluent as defined below.

The pharmaceutical is in one embodiment nerve growth factor (NGF), insulin-like growth factor (IGF), platelet-derived growth factor (PDGF), transforming growth factor (TGF) or N-acetylcysteine. Any combination of these may be administered.

### Neurite outgrowth inhibitor-66 (Nogo-66) inhibitors

The pharmaceutical is in one embodiment an inhibitor of Nogo-66 signalling. The substance is in one embodiment an inhibitor of Nogo-66 signalling and the damaged nerve is in the central nervous system (CNS). Nogo-66 is a 66 amino acid domain in Nogo (also known as reticulon-4) which inhibits neurite outgrowth in the central nervous system.

An inhibitor of Nogo-66 signalling is any molecule that decreases or reduces Nogo-66 signalling. The inhibitor may decrease Nogo-66 signalling by any amount. For instance, the signalling may be decreased by at least 10%, at least 30% at least 40%, at least 50%, at least 60%, at least 70%, at least 80%, at least 90% or at least 95%. An inhibitor may abolish Nogo-66 signalling (i.e. the function is decreased by 100%). Nogo-66 signalling may be measured using known techniques. The extent to which an inhibitor affects Nogo-66 may be determined by measuring the signalling in cells, such as oligodendrocytes, in the presence and absence of the inhibitor. The activity of the inhibitor may be measured by determining the effect of the inhibitor on the ability of a ligand of the Nogo-66 receptor to activate any of the Nogo-66 receptor signal transduction pathways using known assays. Inhibitors may also be tested using known assays of angiogenesis.

The inhibitor may affect the Nogo-66 signalling in any manner. For instance, the inhibitor may decrease the amount of the Nogo-66 receptor, for instance by decreasing the expression of or increasing the degradation of the Nogo-66 receptor. The inhibitor may decrease the activity of the Nogo-66 receptor, for instance by binding to the Nogo-66 receptor or the molecule(s) which the Nogo-66 receptor activates. The inhibitor may decrease the amount of and/or the activity of Nogo-66.

The inhibitor may be a competitive inhibitor (which binds the active site of the molecule to which it binds) or an allosteric inhibitor (which does not bind the active site of the molecule to which it binds). The inhibitor may be reversible. The inhibitor may be irreversible.

### Inhibitors of the Nogo-66 receptor

The inhibitor is in one embodiment an inhibitor of the Nogo-66 receptor. The inhibitor may decrease the production of or expression of the Nogo-66 receptor. The inhibitor may decrease the transcription of the Nogo-66 receptor. The inhibitor may disrupt the DNA of the Nogo-66 receptor, for instance by site-specific mutagenesis using methods such as Zinc-finger nucleases. The inhibitor may decrease the mRNA level of the Nogo-66 receptor or interfere with the processing of the Nogo-66 receptor mRNA, for instance by antisense RNA or RNA interference. This is discussed in more detail below.

The inhibitor may increase protein degradation of the Nogo-66 receptor. The inhibitor may increase the level of natural inhibitors of the Nogo-66 receptor. The inhibitor may decrease the function of the Nogo-66 receptor by inhibitory phosphorylation, ubiquitylation, sumoylation or the like.

The inhibitor of the Nogo-66 receptor is in one embodiment a small molecule inhibitor, a protein, an antibody, a polynucleotide, an oligonucleotide, an antisense RNA, small interfering RNA (siRNA) or small hairpin RNA (shRNA).

The inhibitor of the Nogo-66 receptor may be a protein. The inhibitor is in one embodiment a reduced-function form of the Nogo-66 receptor. The function of the reduced-function form may be reduced/decreased by any amount. For instance, the function may be reduced/decreased by at least 10%, at least 30% at least 40%, at least 50%, at least 60%, at least 70%, at least 80%, at least 90% or at least 95% compared with wild-type Nogo-66 receptor. The inhibitor may be a non-functional form of the Nogo-66 receptor. A reduced-function or non-functional form of the Nogo-66 receptor will compete with native (i.e. wild-type) the Nogo-66 receptor and reduce Nogo-66 signalling.

NGRH1 and NGRH 2 mediate cellular responses to Nogo-66. The amino acid sequences of human NGRH 1 and NGRH2 are shown in SEQ ID NOs: 4 and 6. The inhibitor is in one embodiment a reduced-function variant or non-functional variant of SEQ ID NO: 4 or 6. A reduced-function variant is a protein that has an amino acid sequence which varies from that of SEQ ID NO: 4 or 6 and has a reduced/decreased the ability to form a functional Nogo-66 receptor or activate signal transduction pathways. The function may be reduced/decreased by any amount as discussed above. A non-functional variant is a protein that has an amino acid sequence which varies from that of SEQ ID NO: 4 or 6 and does not have the ability to form a functional Nogo-66 receptor or activate signal transduction pathways. For instance, the non-functional variant may have one or more mutations in the site that forms the dimeric receptor or interacts with the signal transduction pathways. The non-functional variant may also be a truncated form that sequesters Nogo-66 or other Nogo-66 receptor ligands. The non-functional variant may also be a soluble form of the receptor that sequesters Nogo-66 or other Nogo-66 receptor ligands. The ability of a variant to function as Nogo-66 receptor can be assayed using any method known in the art. The comparative functional ability of reduced-function and non-functional variants is typically measured in comparison to the wild-type Nogo-66 receptor, such as SEQ ID NO: 4 or 6.

Over the entire length of the amino acid sequence of SEQ ID NO: 4 or 6, a reduced-function or non-functional variant will in one embodiment be at least 50% homologous to that sequence based on amino acid identity, i.e. have at least 50% amino acid identity over the entire sequence. In one embodiment, the reduced-function or non-functional variant may be at least 60%, at least 70%, at least 80%, at least 85%, at least 90% and in one embodiment at least 95%, 97% or 99% homologous based on amino acid identity (or identical) to the amino acid sequence of SEQ ID NO: 4 or 6 over the entire sequence. There may be at least 80%, for example at least 85%, 90% or 95%, amino acid identity over a stretch of 100 or more, for example 200 or 300 or more, contiguous amino acids ("*hard homology*").

Standard methods in the art may be used to determine homology. For example the UWGCG Package provides the BESTFIT program, which can be used to calculate homology, for example used on its default settings (Devereux et al (1984) Nucleic Acids Research 12, p387-395). The PILEUP and BLAST algorithms can be used to calculate homology or line up sequences (such as identifying equivalent residues or corresponding sequences (typically on their default settings)), for example as described in Altschul S. F. (1993) J Mol Evol 36:290-300; Altschul, S.F et al (1990) J Mol Biol 215:403-10. Software for performing BLAST analyses is publicly available through the National Center for Biotechnology Information (http://www.ncbi.nlm.nih.gov/).

Amino acid substitutions may be made to the amino acid sequence of SEQ ID NO: 4 or 6, for example up to 1, 2, 3, 4, 5, 10, 20, 30, 50, 100 or 200 substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in the Table below. Where amino acids have similar polarity, this can also be determined by reference to the hydropathy scale for amino acid side chains in the second table below.

**Table - Chemical properties of amino acids**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

**Table - Hydropathy scale**

| Side Chain | Hydropathy |
|---|---|
| Ile | 4.5 |
| Val | 4.2 |
| Leu | 3.8 |
| Phe | 2.8 |
| Cys | 2.5 |
| Met | 1.9 |
| Ala | 1.8 |
| Gly | -0.4 |
| Thr | -0.7 |
| Ser | -0.8 |
| Trp | -0.9 |
| Tyr | -1.3 |
| Pro | -1.6 |
| His | -3.2 |
| Glu | -3.5 |
| Gln | -3.5 |
| Asp | -3.5 |
| Asn | -3.5 |
| Lys | -3.9 |
| Arg | -4.5 |

One or more amino acid residues of the amino acid sequence of SEQ ID NO: 4 or 6 may additionally be deleted from the polypeptides described above. Up to 1, 2, 3, 4, 5, 10, 20, 30 or 50 residues may be deleted, or more.

Reduced-function or non-functional variants may include fragments of SEQ ID NO: 4 or 6. Such fragments typically retain the domain of SEQ ID NO: 4 or 6 which binds Nogo-66 or other ligands of the Nogo-66 receptor but are reduced-function or non-functional. Fragments may be at least 200, 300, 400 or 500 amino acids in length. One or more amino acids may be alternatively or additionally added to the polypeptides described above.

Alternatively, the inhibitor may be a polynucleotide encoding a reduced-function or non-functional variant of the Nogo-66 receptor. The reduced-function or non-functional variant may be any of those discussed above.

A polynucleotide, such as a nucleic acid, is a polymer comprising two or more nucleotides. The nucleotides can be naturally occurring or artificial. A nucleotide typically contains a nucleobase, a sugar and at least one linking group, such as a phosphate, 2'O-methyl, 2' methoxy-ethyl, phosphoramidate, methylphosphonate or phosphorothioate group. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The nucleotide is typically a ribonucleotide or deoxyribonucleotide. The nucleotide typically contains a monophosphate, diphosphate or triphosphate. Phosphates may be attached on the 5' or 3' side of a nucleotide.

Nucleotides include, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), 5-methylcytidine monophosphate, 5-methylcytidine diphosphate, 5-methylcytidine triphosphate, 5-hydroxymethylcytidine monophosphate, 5-hydroxymethylcytidine diphosphate, 5-hydroxymethylcytidine triphosphate, cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP), 5-methyl-2'-deoxycytidine monophosphate, 5-methyl-2'-deoxycytidine diphosphate, 5-methyl-2'-deoxycytidine triphosphate, 5-hydroxymethyl-2'-deoxycytidine monophosphate, 5-hydroxymethyl-2'-deoxycytidine diphosphate and 5-hydroxymethyl-2'-deoxycytidine triphosphate. The nucleotides are in one embodiment selected from AMP, TMP, GMP, UMP, dAMP, dTMP, dGMP or dCMP.

The nucleotides may contain additional modifications. In particular, suitable modified nucleotides include, but are not limited to, 2'amino pyrimidines (such as 2'-amino cytidine and 2'-amino uridine), 2'-hyrdroxyl purines (such as , 2'-fluoro pyrimidines (such as 2'-fluorocytidine and 2'fluoro uridine), hydroxyl pyrimidines (such as 5'-α-P-borano uridine), 2'-O-methyl nucleotides (such as 2'-O-methyl adenosine, 2'-O-methyl guanosine, 2'-O-methyl cytidine and 2'-O-methyl uridine), 4'-thio pyrimidines (such as 4'-thio uridine and 4'-thio cytidine) and nucleotides have modifications of the nucleobase (such as 5-pentynyl-2'-deoxy uridine, 5-(3-aminopropyl)-uridine and 1,6-diaminohexyl-N-5-carbamoylmethyl uridine).

One or more nucleotides in the polynucleotide can be oxidized or methylated. One or more nucleotides in the polynucleotide may be damaged. For instance, the polynucleotide may comprise a pyrimidine dimer. Such dimers are typically associated with damage by ultraviolet light.

The nucleotides in the polynucleotide may be attached to each other in any manner. The nucleotides may be linked by phosphate, 2'O-methyl, 2' methoxy-ethyl, phosphoramidate, methylphosphonate or phosphorothioate linkages. The nucleotides are typically attached by their sugar and phosphate groups as in nucleic acids. The nucleotides may be connected via their nucleobases as in pyrimidine dimers.

The polynucleotide can be a nucleic acid, such as deoxyribonucleic acid (DNA) or a ribonucleic acid (RNA). The polynucleotide may be any synthetic nucleic acid known in the art, such as peptide nucleic acid (PNA), glycerol nucleic acid (GNA), threose nucleic acid (TNA), locked nucleic acid (LNA), morpholino nucleic acid or other synthetic polymers with nucleotide side chains. The polynucleotide may be single stranded or double stranded.

The polynucleotide sequence in one embodiment encodes a reduced-function or non-functional variant of SEQ ID NO: 4 or 6 as discussed above. The polynucleotide sequence in one embodiment comprises a variant of SEQ ID NO: 3 or 5 with at least 50%, at least 60%, at least 70%, at least 80%, at least 90%, at least 95%, at least 97%, at least 98% or at least 99% homology based on nucleotide identity over the entire sequence, i.e. nucleotide identity over the entire sequences. There may be at least 80%, for example at least 85%, 90% or 95% nucleotide identity over a stretch of 300 or more, for example 400, 500, 600, 700, 800 or 900 or more, contiguous nucleotides ("*hard homology*"). Homology may be calculated as described above.

Polynucleotide sequences may be derived and replicated using standard methods in the art, for example using PCR involving specific primers. It is straightforward to generate polynucleotide sequences using such standard techniques.

The amplified sequences may be incorporated into a recombinant replicable vector such as a cloning vector. The vector may be used to replicate the polynucleotide in a compatible host cell. Thus polynucleotide sequences may be made by introducing the polynucleotide into a replicable vector, introducing the vector into a compatible host cell, and growing the host cell under conditions which bring about replication of the vector. The vector may be recovered from the host cell. Suitable host cells for cloning of polynucleotides are known in the art and described in more detail below.

The polynucleotide sequence may be cloned into any suitable expression vector. In an expression vector, the polynucleotide sequence encoding a construct is typically operably linked to a control sequence which is capable of providing for the expression of the coding sequence by the host cell. Such expression vectors can be used to express a construct.

The term "*operably linked*" refers to a juxtaposition wherein the components described are in a relationship permitting them to function in their intended manner. A control sequence "*operably linked*" to a coding sequence is ligated in such a way that expression of the coding sequence is achieved under conditions compatible with the control sequences. Multiple copies of the same or different polynucleotide may be introduced into the vector.

The expression vector may then be introduced into a suitable host cell. Thus, a construct can be produced by inserting a polynucleotide sequence encoding a construct into an expression vector, introducing the vector into a compatible bacterial host cell, and growing the host cell under conditions which bring about expression of the polynucleotide sequence. The vectors may be for example, plasmid, virus or phage vectors provided with an origin of replication, optionally a promoter for the expression of the said polynucleotide sequence and optionally a regulator of the promoter. The vectors may contain one or more selectable marker genes, for example an ampicillin resistance gene. Promoters and other expression regulation signals may be selected to be compatible with the host cell for which the expression vector is designed. A T7, *trc, lac, ara* or λ_{L} promoter is typically used.

The host cell typically expresses the construct at a high level. Host cells transformed with a polynucleotide sequence encoding a construct will be chosen to be compatible with the expression vector used to transform the cell. The host cell is typically bacterial and in one embodiment *E. coli.* Any cell with a λ DE3 lysogen, for example C41 (DE3), BL21 (DE3), JM109 (DE3), B834 (DE3), TUNER, Origami and Origami B, can express a vector comprising the T7 promoter.

Inhibitors of the Nogo-66 receptor may also reduce amounts of the Nogo-66 receptor present in the patient or the CNS, for example by knocking down expression of the Nogo-66 receptor. Antisense and RNA interference (RNAi) technology for knocking down protein expression are well known in the art and standard methods can be employed to knock down expression of the Nogo-66 receptor.

Both antisense and siRNA technology interfere with mRNA. Antisense oligonucleotides interfere with mRNA by binding to (hybridising with) a section of the mRNA. The antisense oligonucleotide is therefore designed to be complementary to the mRNA (although the oligonucleotide does not have to be 100% complementary as discussed below). In other words, the antisense oligonucleotide may be a section of the cDNA. Again, the oligonucleotide sequence may not be 100% identical to the cDNA sequence. This is also discussed below.

RNAi involves the use of double-stranded RNA, such small interfering RNA (siRNA) or small hairpin RNA (shRNA), which can bind to the mRNA and inhibit protein expression.

Accordingly, the inhibitor in one embodiment comprises an oligonucleotide which specifically hybridises to a part of the Nogo-66 receptor mRNA. The inhibitor in one embodiment comprises an oligonucleotide which specifically hybridises to a part of SEQ ID NO: 3 or 5 (human *NGRH1 or NGRH2* mRNA). Oligonucleotides are short nucleotide polymers which typically have 50 or fewer nucleotides, such 40 or fewer, 30 or fewer, 22 or fewer, 21 or fewer, 20 or fewer, 10 or fewer or 5 or fewer nucleotides. The oligonucleotide used in one example is 20 to 25 nucleotides in length, in one embodiment 21 or 22 nucleotides in length. The nucleotides can be naturally occurring or artificial. The nucleotides can be any of those described above.

An oligonucleotide in one embodiment specifically hybridises to a part of SEQ ID NO: 3 or 5, hereafter called the target sequence. The length of the target sequence typically corresponds to the length of the oligonucleotide. For instance, a 21 or 22 nucleotide oligonucleotide typically specifically hybridises to a 21 or 22 nucleotide target sequence. The target sequence may therefore be any of the lengths discussed above with reference to the length of the oligonucleotide. The target sequence is typically consecutive nucleotides within the target polynucleotide.

An oligonucleotide "*specifically hybridises*" to a target sequence when it hybridises with in one embodiment or high affinity to the target sequence but does not substantially hybridise, does not hybridise or hybridises with only low affinity to other sequences.

An oligonucleotide "*specifically hybridises*" if it hybridises to the target sequence with a melting temperature (Tₘ) that is at least 2°C, such as at least 3 °C, at least 4°C, at least 5 °C, at least 6°C, at least 7 °C, at least 8°C, at least 9 °C or at least 10 °C, greater than its Tₘ for other sequences. In one embodiment, the oligonucleotide hybridises to the target sequence with a Tₘ that is at least 2°C, such as at least 3 °C, at least 4°C, at least 5 °C, at least 6°C, at least 7 °C, at least 8°C, at least 9 °C, at least 10 °C, at least 20°C, at least 30 °C or at least 40°C, greater than its Tₘ for other nucleic acids. In one embodiment, the portion hybridises to the target sequence with a Tₘ that is at least 2°C, such as at least 3 °C, at least 4°C, at least 5 °C, at least 6°C, at least 7 °C, at least 8°C, at least 9 °C, at least 10 °C, at least 20°C, at least 30 °C or at least 40 °C, greater than its Tₘ for a sequence which differs from the target sequence by one or more nucleotides, such as by 1, 2, 3, 4 or 5 or more nucleotides. The portion typically hybridises to the target sequence with a Tₘ of at least 90°C, such as at least 92 °C or at least 95°C. Tₘ can be measured experimentally using known techniques, including the use of DNA microarrays, or can be calculated using publicly available Tₘ calculators, such as those available over the internet.

Conditions that permit the hybridisation are well-known in the art (for example, Sambrook et al., 2001, Molecular Cloning: a laboratory manual, 3rd edition, Cold Spring Harbour Laboratory Press; and Current Protocols in Molecular Biology, Chapter 2, Ausubel et al., Eds., Greene Publishing and Wiley-lnterscience, New York (1995)). Hybridisation can be carried out under low stringency conditions, for example in the presence of a buffered solution of 30 to 35% formamide, 1 M NaCl and 1 % SDS (sodium dodecyl sulfate) at 37°C followed by a 20 wash in from 1X (0.1650 M Na⁺) to 2X (0.33 M Na⁺) SSC (standard sodium citrate) at 50°C. Hybridisation can be carried out under moderate stringency conditions, for example in the presence of a buffer solution of 40 to 45% formamide, 1 M NaCl, and 1 % SDS at 37°C, followed by a wash in from 0.5X (0.0825 M Na⁺) to 1X (0.1650 M Na⁺) SSC at 55 °C. Hybridisation can be carried out under high stringency conditions, for example in the presence of a buffered solution of 50% formamide, 1 M NaCl, 1% SDS at 37 °C, followed by a wash in 0.1X (0.0165 M Na⁺) SSC at 60 °C.

The oligonucleotide may comprise a sequence which is substantially complementary to the target sequence. Typically, the oligonucleotides are 100% complementary. However, lower levels of complementarity may also be acceptable, such as 95%, 90%, 85% and even 80%. Complementarity below 100% is acceptable as long as the oligonucleotides specifically hybridise to the target sequence. An oligonucleotide may therefore have 1, 2, 3, 4, 5, 6, 7, 8, 9, 10 or more mismatches across a region of 5, 10, 15, 20, 21, 22, 30, 40 or 50 nucleotides.

Alternatively, the inhibitor in one embodiment comprises an oligonucleotide which comprises 50 or fewer consecutive nucleotides from the reverse complement of SEQ ID NO: 3 or 5. The oligonucleotide may be any of the lengths discussed above. It is in one embodiment 21 or 22 nucleotides in length. The oligonucleotide may comprise any of the nucleotides discussed above, including the modified nucleotides.

The oligonucleotide can be a nucleic acid, such as any of those discussed above. The oligonucleotide is in one embodiment RNA.

The oligonucleotide may be single stranded. The oligonucleotide may be double stranded. The oligonucleotide may comprise a hairpin.

Oligonucleotides may be synthesised using standard techniques known in the art. Alternatively, oligonucleotides may be purchased.

The inhibitor is in one embodiment an antibody which specifically binds the Nogo-66 receptor. The antibody in one embodiment binds the sequence shown in SEQ ID NO: 4 or 6.

An antibody "specifically binds" to a protein when it binds with preferential or high affinity to that protein but does not substantially bind, does not bind or binds with only low affinity to other proteins. For instance, an antibody "specifically binds" to SEQ ID NO: 31 when it binds with preferential or high affinity to SEQ ID NO: 31 but does not substantially bind, does not bind or binds with only low affinity to other human proteins.

An antibody binds with preferential or high affinity if it binds with a Kd of 1 × 10-7 M or less, in one embodiment 5 × 10-8 M or less, in one embodiment 1 × 10-8 M or less or in one embodiment 5 × 10-9 M or less. An antibody binds with low affinity if it binds with a Kd of 1 × 10-6 M or more, in one embodiment 1 × 10-5 M or more, in one embodiment 1 × 10-4 M or more, in one embodiment 1 × 10-3 M or more, in one embodiment 1 × 10-2 M or more. A variety of protocols for competitive binding or immunoradiometric assays to determine the specific binding capability of compounds, such as antibodies or antibody constructs and oligonucleotides are well known in the art (see for example Maddox et al, J. Exp. Med. 158, 1211-1226, 1993).

The antibody may be, for example, a monoclonal antibody, a polyclonal antibody, a single chain antibody, a chimeric antibody, a bispecific antibody, a CDR-grafted antibody or a humanized antibody. The antibody may be an intact immunoglobulin molecule or a fragment thereof such as a Fab, F(ab')2 or Fv fragment.

### Inhibitors of Nogo-66

The inhibitor is in one embodiment an inhibitor of Nogo-66. The inhibitor may decrease the production of or expression of Nogo-66 or Nogo-66A. The inhibitor may decrease the transcription of Nogo-66. The inhibitor may disrupt the DNA of Nogo-66, for instance by site-specific mutagenesis using methods such as Zinc-finger nucleases. The inhibitor may decrease the mRNA level of Nogo-66 or interfere with the processing of Nogo-66 mRNA, for instance by antisense RNA or RNA interference. This is discussed in more detail below.

The inhibitor may increase protein degradation of Nogo-66. The inhibitor may increase the level of natural inhibitors of Nogo-66. The inhibitor may decrease the function of Nogo-66 by inhibitory phosphorylation, ubiquitylation, sumoylation or the like.

The inhibitor of Nogo-66 is in one embodiment a small molecule inhibitor, a protein, an antibody, a polynucleotide, an oligonucleotide, an antisense RNA, small interfering RNA (siRNA) or small hairpin RNA (shRNA).

The inhibitor of Nogo-66 may be a protein. The inhibitor is in one embodiment a reduced-function form of Nogo-66. The function of the reduced-function form may be reduced/decreased by any amount. For instance, the function may be reduced/decreased by at least 10%, at least 30% at least 40%, at least 50%, at least 60%, at least 70%, at least 80%, at least 90% or at least 95% compared with wild-type Nogo-66. The inhibitor may be a non-functional form of Nogo-66. A reduced-function or non-functional form of Nogo-66 will compete with native (i.e. wild-type) Nogo-66 and reduce Nogo-66 signalling.

The amino acid sequence of human Nogo-66 is shown in residues 1055 to 1120 of SEQ ID NO: 2. Inhibitors may be based on SEQ ID NO: 2 itself or just residues 1055 to 1120 of SEQ ID NO: 2. The inhibitor is in one embodiment a reduced-function variant of, such as a non-functional variant of, SEQ ID NO: 2 or residues 1055 to 1120 of SEQ ID NO: 2. A reduced-function variant is a protein that has an amino acid sequence which varies from that of SEQ ID NO: 2 or residues 1055 to 1120 of SEQ ID NO: 2, has the ability to bind the Nogo-66 receptor, such as Nogo-66R2, and has a reduced/decreased ability to activate or agonise the Nogo-66 receptor. The function may be reduced/decreased by any amount as discussed above. A non-functional variant is a protein that has an amino acid sequence which varies from that of SEQ ID NO: 2 or residues 1055 to 1120 of SEQ ID NO: 2, has the ability to bind the Nogo-66 receptor, such as Nogo-66R2, and does not have the ability to activate or agonise the Nogo-66 receptor.

The comparative binding ability of reduced-function and non-functional variants is typically measured in comparison to wild-type Nogo-66 (such as SEQ ID NO: 2 or residues 1055 to 1120 of SEQ ID NO: 2). Binding can be measured using know techniques.

The reduced-function variant may reduce Nogo-66 signalling by competing with the natural ligands for binding to the Nogo-66 receptor, but activating the receptor to a lesser degree. The non-functional variant may reduce Nogo-66 signalling by competing with the natural ligands for binding to the Nogo-66 receptor, but not activating the receptor. The ability of a variant to bind to and activate or agonise the Nogo-66 receptor can be assayed using any method known in the art.

Over the entire length of the amino acid sequence of SEQ ID NO: 2 or residues 1055 to 1120 of SEQ ID NO: 2, a reduced-function or non-functional variant will in one embodiment be at least 50% homologous to that sequence based on amino acid identity, i.e. have at least 50% amino acid identity over the entire sequence. In one embodiment, the reduced-function or non-functional variant may be at least 60%, at least 70%, at least 80%, at least 85%, at least 90% and in one embodiment at least 95%, 97% or 99% homologous based on amino acid identity (or identical) to the amino acid sequence of SEQ ID NO: 2 or residues 1055 to 1120 of SEQ ID NO: 2 over the entire sequence. There may be at least 80%, for example at least 85%, 90% or 95%, amino acid identity over a stretch of 100 or more, for example 200 or 300 or more, contiguous amino acids ("*hard homology*").

Amino acid substitutions may be made to the amino acid sequence of SEQ ID NO: 2 or residues 1055 to 1120 of SEQ ID NO: 2, for example up to 1, 2, 3, 4, 5, 10, 20, 30, 50 or 100 substitutions. Conservative substitutions as discussed above may be made.

One or more amino acid residues of the amino acid sequence of SEQ ID NO: 2 or residues 1055 to 1120 of SEQ ID NO: 2 may additionally be deleted from the polypeptides described above. Up to 1, 2, 3, 4, 5, 10, 20, 30 or 50 residues may be deleted, or more.

Reduced-function or non-functional variants may include fragments of SEQ ID NO: 2 or residues 1055 to 1120 of SEQ ID NO: 2. Such fragments typically retain the domain which binds the Nogo-66 receptor but lack the domain that activates the receptor. Fragments may be at least 20, 30, 40, 50, 100, 200, 300, 400 or 500 amino acids in length. One or more amino acids may be alternatively or additionally added to the polypeptides described above.

An inhibitor for use in one example is Nogo-66(140) antagonist peptide (NEP1 40) which consists of residues 1055 to 1094 of SEQ ID NO: 2 (GrandPré T1, Li S, Strittmatter SM, Nature. 2002 May 30;417(6888):547-51).

Alternatively, the inhibitor may be a polynucleotide encoding a reduced-function or non-functional variant of Nogo-66. The reduced-function or non-functional variant may be any of those discussed above. Polynucleotides are defined above.

Inhibitors of Nogo-66 may also reduce amounts of Nogo-66 present in the patient or the CNS, for example by knocking down expression of Nogo-66. Antisense and RNA interference (RNAi) technology for knocking down protein expression are well known in the art and standard methods can be employed to knock down expression of Nogo-66.

Both antisense and siRNA technology interfere with mRNA. Antisense oligonucleotides interfere with mRNA by binding to (hybridising with) a section of the mRNA. The antisense oligonucleotide is therefore designed to be complementary to the mRNA (although the oligonucleotide does not have to be 100% complementary as discussed below). In other words, the antisense oligonucleotide may be a section of the cDNA. Again, the oligonucleotide sequence may not be 100% identical to the cDNA sequence. This is also discussed below.

RNAi involves the use of double-stranded RNA, such small interfering RNA (siRNA) or small hairpin RNA (shRNA), which can bind to the mRNA and inhibit protein expression.

Accordingly, the inhibitor in one embodiment comprises an oligonucleotide which specifically hybridises to a part of the *Nogo-66* mRNA. The inhibitor in one embodiment comprises an oligonucleotide which specifically hybridises to a part of SEQ ID NO: 1 (the human *reticulon-4* mRNA).

Oligonucleotides are short nucleotide polymers which typically have 50 or fewer nucleotides, such 40 or fewer, 30 or fewer, 22 or fewer, 21 or fewer, 20 or fewer, 10 or fewer or 5 or fewer nucleotides. The oligonucleotide used in one embodiment is 20 to 25 nucleotides in length, or in one embodiment 21 or 22 nucleotides in length. The nucleotides can be naturally occurring or artificial. The nucleotides can be any of those described above.

An oligonucleotide in one embodiment specifically hybridises to a part of SEQ ID NO: 1, hereafter called the target sequence. The length of the target sequence typically corresponds to the length of the oligonucleotide. For instance, a 21 or 22 nucleotide oligonucleotide typically specifically hybridises to a 21 or 22 nucleotide target sequence. The target sequence may therefore be any of the lengths discussed above with reference to the length of the oligonucleotide. The target sequence is typically consecutive nucleotides within the target polynucleotide. The target sequence is in one embodiment within nucleotides 3461 to 3658 of SEQ ID NO: 1.

An oligonucleotide "*specifically hybridises*" to a target sequence as defined above.

The oligonucleotide may comprise a sequence which is substantially complementary to the target sequence. Typically, the oligonucleotides are 100% complementary. However, lower levels of complementarity may also be acceptable, such as 95%, 90%, 85% and even 80%. Complementarity below 100% is acceptable as long as the oligonucleotides specifically hybridise to the target sequence. An oligonucleotide may therefore have 1, 2, 3, 4, 5, 6, 7, 8, 9, 10 or more mismatches across a region of 5, 10, 15, 20, 21, 22, 30, 40 or 50 nucleotides.

Alternatively, the inhibitor in one embodiment comprises an oligonucleotide which comprises 50 or fewer consecutive nucleotides from the reverse complement of SEQ ID NO: 1. The oligonucleotide may be any of the lengths discussed above. It is in one embodiment 21 or 22 nucleotides in length. The oligonucleotide may comprise any of the nucleotides discussed above, including the modified nucleotides. The olignucleoitde may be any of the types discussed above.

The inhibitor is in one embodiment an antibody which specifically binds Nogo-66. The antibody in one embodiment binds the sequence shown by residues 1055 to 1120 of SEQ ID NO: 2. Specific binding is defined above. The antibody may be any of the types discussed above.

### Other inhibitors

The inhibition of other molecules in the CNS may promote nerve reinnervation. The pharmaceutical is in one embodiment an inhibitor of NI-35, an inhibitor of chondroitin sulphate proteoglycans (CSPGs), an inhibitor of glycosaminoglycans or an inhibitor of kerata sulphate proteoglycan (KSPG), an inhibitor of myelin-associated glycoprotein (MAG, an inhibitor of oligodendrocyte myelin glycoprotein (OMgp), an inhibitor of Ephrin B3, an inhibitor of semaphoring 4D (Sema 4D) or an inhibitor of Sema 3A, in one embodiment when the damaged nerve is in the CNS. The CSPGs are in one embodiment CS-E, aggrecan, phosphocan, neurocan or NG-2. Any of the definitions of inhibitors with reference to Nogo-66 equally apply to these inhibitors.

### Administration

In the method, the substance, composition or inhibitor is administered to the patient. The substance, composition or inhibitor of may be administered to the patient in any appropriate way. The substance, composition or inhibitor may be administered in a variety of dosage forms. Thus, it can be administered orally, for example as tablets, troches, lozenges, aqueous or oily suspensions, dispersible powders or granules. It may also be administered byenteral or parenteral routes such as via buccal, anal, pulmonary, intravenous, intra-arterial, intramuscular, intraperitoneal, intraarticular, topical or other appropriate administration routes. The substance, composition or inhibitor may be administered directly into the eye to be treated. The route of administration is one embodiment intravenous. A physician will be able to determine the required route of administration for each particular patient.

The formulation of the substance, composition or inhibitor will depend upon factors such as the nature of the exact substance, composition or inhibitor, etc. The substance, composition or inhibitor may be formulated for simultaneous, separate or sequential use with other therapies or with electrical stimulation.

The substance, composition or inhibitor is typically formulated for administration with a pharmaceutically acceptable carrier or diluent. The pharmaceutical carrier or diluent may be, for example, an isotonic solution. For example, solid oral forms may contain, together with the active substance, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active substance, a pharmaceutically acceptable carrier, e.g. sterile water, olive oil, ethyl oleate, glycols, e.g. propylene glycol, and if desired, a suitable amount of lidocaine hydrochloride.

Solutions for intravenous administration or infusion may contain as carrier, for example, sterile water or one embodiment they may be in the form of sterile, aqueous, isotonic saline solutions.

For suppositories, traditional binders and carriers may include, for example, polyalkylene glycols or triglycerides; such suppositories may be formed from mixtures containing the active ingredient in the range of 0.5% to 10%, one embodiment 1% to 2%.

Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, one embodiment 25% to 70%. Where the pharmaceutical composition is lyophilised, the lyophilised material may be reconstituted prior to administration, e.g. a suspension. Reconstitution is one embodiment effected in buffer.

Capsules, tablets and pills for oral administration to an individual may be provided with an enteric coating comprising, for example, Eudragit "S", Eudragit "L", cellulose acetate, cellulose acetate phthalate or hydroxypropylmethyl cellulose.

Polynucleotides or oligonucleotides maybe naked nucleotide sequences or be in combination with cationic lipids, polymers or targeting systems. They may be delivered by any available technique. For example, the polynucleotide or oligonucleotide may be introduced by needle injection, one embodiment intradermally, subcutaneously or intramuscularly. Alternatively, the polynucleotide or oligonucleotide may be delivered directly across the skin using a delivery device such as particle-mediated gene delivery. The polynucleotide or oligonucleotide may be administered topically to the skin, or to mucosal surfaces for example by intranasal, oral, or intrarectal administration.

Uptake of polynucleotide or oligonucleotide constructs may be enhanced by several known transfection techniques, for example those including the use of transfection agents. Examples of these agents include cationic agents, for example, calcium phosphate and DEAE-Dextran and lipofectants, for example, lipofectam and transfectam. The dosage of the polynucleotide or oligonucleotide to be administered can be altered.

A therapeutically effective amount of the substance, composition or inhibitor is typically administered to the patient. A therapeutically effective amount of is an amount effective to promote nerve reinnervation or ameliorate one or more symptoms of the nerve damage. A therapeutically effective amount is one embodiment an amount effective to abolish one or more of, or one embodiment all of, the symptoms of the nerve damage.

The dose may be determined according to various parameters, especially according to the substance used; the age, weight and condition of the patient to be treated; the route of administration; and the required regimen. Again, a physician will be able to determine the required route of administration and dosage for any particular patient. A typical daily dose is from about 0.1 to 50 mg per kg of body weight, according to the activity of the specific substance, composition or inhibitor, the age, weight and conditions of the subject to be treated and the frequency and route of administration. The dose may be provided as a single dose or may be provided as multiple doses, for example taken at regular intervals, for example 2, 3 or 4 doses administered hourly. In one embodiment, dosage levels are from 5 mg to 2 g.

Typically polynucleotides or oligonucleotides are administered in the range of 1 pg to 1 mg, in one embodiment to 1 pg to 10 µg nucleic acid for particle mediated delivery and 10 µg to 1 mg for other routes.

### Kit

A kit can also be provided for promoting reinnervation of a damaged nerve in a patient. The kit comprises a device capable of electrically stimulating the skin of the patient. Suitable devices are disclosed above. The kit also comprises a scaffold which promotes reinnervation, a graft which promotes reinnervation, a population of cells which promotes reinnervation or a pharmaceutical which promotes reinnervation. The kit may comprise any of the scaffolds, grafts, cells or pharmaceuticals discussed above.

The kit may additionally comprise one or more other reagents or instruments which enable the method to be carried out. Such reagents include means for taking a sample from the patient or to administer a substance or composition to the patient, and suitable buffers. The kit may, optionally, comprise instructions to enable the kit to be used in the method or details regarding patients on which the method may be carried out.

### Example

In our recent report on the *in vivo* application of ES on human cutaneous healing²⁸ (*n* = 20), we had the limitation of only a single time point analysis post-wounding (day 14). However, in the current study (n = 40), we have included intermediate time points such as days 3, 7 and 10 to explicitly understand the neural network formation during repair. Enhanced reinnervation post-ES was further validated with 1) TUBB3 and FIG4 expression analyses, reinnervation, neuropeptide synthesis and neural-related cellular differentiation in wound granulation tissue, specifically in melanocytes and Merkel cells, 2) kinetics of melanogenesis, 3) cell proliferation and nerve growth factor analysis, and 4) the *in vitro* role of ES in neural differentiation.

### Materials & Methods

### Patient selection and recruitment

This study was conducted in accordance with the ethical principles of Good Clinical Practice and the Declaration of Helsinki. This study received ethical approval from the local research committee (Manchester, UK), and all subjects (n=40; Table below) gave full written, informed consent.

| Sample number | Sex | Ethnic Background | Age |
|---|---|---|---|
| 1 | F | British Caucasian | 28 |
| 2 | F | British Caucasian | 21 |
| 3 | M | British Caucasian | 20 |
| 4 | M | British Caucasian | 19 |
| 5 | F | British Caucasian | 24 |
| 6 | F | British Caucasian | 30 |
| 7 | M | British Caucasian | 22 |
| 8 | F | British Caucasian | 27 |
| 9 | F | British Caucasian | 24 |
| 10 | F | British Caucasian | 22 |
| 11 | F | British Caucasian | 27 |
| 12 | F | British Caucasian | 30 |
| 13 | F | British Caucasian | 22 |
| 14 | F | British Caucasian | 31 |
| 15 | F | British Caucasian | 28 |
| 16 | F | British Caucasian | 26 |
| 17 | M | British Caucasian | 30 |
| 18 | F | British Caucasian | 30 |
| 19 | M | British Caucasian | 30 |
| 20 | F | British Caucasian | 28 |
| 21 | F | British Caucasian | 26 |
| 22 | M | British Caucasian | 21 |
| 23 | M | British Caucasian | 21 |
| 24 | M | British Caucasian | 19 |
| 25 | M | British Caucasian | 28 |
| 26 | F | British Caucasian | 27 |
| 27 | F | British Caucasian | 22 |
| 28 | F | British Caucasian | 18 |
| 29 | F | British Caucasian | 26 |
| 30 | F | British Caucasian | 21 |
| 31 | F | British Caucasian | 24 |
| 32 | F | British Caucasian | 22 |
| 33 | F | British Caucasian | 20 |
| 34 | M | British Caucasian | 18 |
| 35 | M | British Caucasian | 23 |
| 36 | F | British Caucasian | 21 |
| 37 | F | British Caucasian | 20 |
| 38 | F | British Caucasian | 28 |
| 39 | F | British Caucasian | 21 |
| 40 | F | British Caucasian | 20 |

### SHSY5Y cell culture and differentiation

Human neuroblastoma SHSY5Y cells (ATCC, CRL-2266) were grown in Dulbecco's modified eagle medium with nutrient mixture F12 (DMEM-F12; cat.11320-074; Life Technologies, Paisley, UK) supplemented with 10% fetal calf serum (FCS), 4 × 10⁻³M glutamine, 10 U/ml penicillin and 10 mg/ml streptomycin. Cells were incubated at 37 °C in a 5% (v/v) CO2 humidified atmosphere. Cells were seeded on to 22 mm round cover slips (BD Biosciences, Oxford, UK) in 6-well plates (at density of 3 × 10⁴ cells/cm²). After 24 hours, neural differentiation was induced with retinoic acid (RA; 10⁻⁵ M; cat. R2625, Sigma Aldrich, Dorset, UK) in medium supplemented with 2% FCS for 6 days. Brain derived neurotrophic factor (BDNF; 50 ng/ ml; cat. B3795, Sigma Aldrich, UK) was added for further 4 days in medium supplemented with 0.5% FCS, as previously reported⁴⁰.

### In vitro electrical stimulation

SHSY5Y cells were subjected to DW (degenerating biphasic sine wave) ES waveform²¹ at 100 mV/mm (60 Hz).

### Electrical stimulation device used on human volunteers

The electrical stimulation device used for *in vivo* ES-treated wounds was the Fenzian system (Fenzian Ltd, Hungerford, UK)²⁹ which is Food and Drug Administration (FDA) 510(k) registered and Conformite Europenne (CE) approved. This is a transcutaneous low intensity device, which detects changes in skin impedance and produces an adjusting "degenerating electrical waveform" where each impulse depends on the skin's electrical properties as they alter *(i.e.* uses electrobiofeedback). This device delivers 0.004 milliamps, 20-80V, has a frequency default of 60Hz and impulses whose width are approximately 1 to 100 µs.

### Human punch biopsy collection for wound healing studies

The protocol that we adopted for *in vivo* ES studies is mentioned in our previous study²⁹. We divided volunteers into 2 cohorts (n=20 in each cohort); first cohort (Cohort A) providing punch biopsies for normal and ES-treated wound healing on days 3 and 7 and the second cohort (Cohort B) providing for normal and ES-treated wound healing on days 10 and 14. The methodology adopted for both the sets were the same except the days assigned for punch biopsy collection. Normal (control) wound healing biopsies collected on days 3, 7, 10 and 14 are denoted as C3, C7, C10 and C14 respectively. Similarly, ES-treated healing biopsies collected on days 3, 7, 10 and 14 are denoted as ES3, ES7, ES10 and ES14 respectively.

### Cohort A

On day 0, participants had two 5 mm diameter full thickness skin biopsies performed under their upper inner arm which acted as the control arm to monitor normal wound healing. On day 3, one 7 mm diameter skin biopsy was performed encompassing one of the previous wound sites (C3). On day 7, another 7 mm diameter skin biopsy was performed to excise the second previous wound site (C7). On Day 7 itself, 2 × 5 mm biopsies were created in the same anatomical location on the contralateral upper inner arm with an intervention phase of ES treatment⁵⁸. From this day onwards (day 7), the volunteer was treated with ES until day 14. Intermediate biopsies from ES-treated volunteers were taken on days 10 (ES3) and 14 (ES7), similar to that on days 3 (C3) and 7 (C7) for normal healing.

### Cohort B

In this cohort, biopsies were collected on days 10 (C10) and 14 (C14) to obtain normal healing samples on days 10 and 14. However, the other 2 biopsies on the contralateral arm were made on day 14. From day 14 onwards, the volunteer was treated with ES and wounds were rebiopsied on days 24 (ES10) and 28 (ES14).

### Tissue preparation for wax-embedding, section cutting, histological staining and semi-quantitation

Tissues for wax-embedding were fixed in 10% neutral-buffered formalin (cat. F5304; Sigma-Aldrich, Dorset, UK) at 4°C and processed as described previously²⁸.

### Microarray analysis

In microarray (n=4), normal wound healing samples were compared to ES samples on days 3, 7, 10 and 14. Agilent Whole Human Genome (SurePrint G3 Hmn GE 8x60K V2) Oligo Microarray kit (Agilent Technologies) was used in this study following the manufacturer's protocol-One-Color Microarray-Based Gene Expression Analysis (Low Input QuickAmp Labeling kit)⁵⁹. Data was analyzed with Qlucore Omics Explorer (Qlucore AB, Lund, Sweden)⁶⁰. Statistical significance (P < 0.05) was also generated by the same software.

### RNA isolation, cDNA synthesis and quantitative real time- polymerase chain reaction

For *in vivo* biopsies obtained on different days of healing, RNA isolation, cDNA synthesis, and qRT-PCR were performed as explained previously²⁸. RPL32²⁸ was used as the internal control and ΔΔC_{T} method was used to calculate fold change of gene expression. The primers used are detailed in the Table below.

| Gene | Primers (bp) FP: Forward primer RP: Reverse primer | Accession number | Amplicon product size (bp) |
|---|---|---|---|
| FIG4 | FP: gaggtcagaggccctttcat (20) RP: agatgttctccgcattcagc (20) | cat. no. 04687582001 | 61 |
| MTM1 | FP: taaatgcagtggccaacaag (20) RP: aagttcggcgttatgatatgc (21) | cat. no. 04688627001 | 71 |
| NGF | FP: tccggacccaataacagttt (20) RP: ggacattacgctatgcacctc (21) | cat. no. 04687655001 | 75 |
| PI3KII | FP: gcaacgagaaagagatgcaa (20) RP: ctcatggatctcggcaatg (19) | cat. no. 04686918001 | 74 |
| Pikfyve | FP: tccttctggcttcctctgtc (20) RP: ggtagaacaaaacccacacca (21) | cat. no. 04687639001 | 60 |
| PGP9.5 | FP: cctgaagacagagcaaaatgc (21) RP: aaatggaaattcaccttgtcatct (24) | NM_004181.3 | 110 |

### Immunohistochemistry and immunocytochemistry

Immunohistochemical staining was performed as detailed in our previous reports²⁸. Positive controls for protein detection were all laboratory-normalized tissues, and sections without primary antibody served as negative controls. All histological slides were analysed with a phase contrast microscope (Olympus IX51 microscope; Olympus, UK) by two independent observers.

For tissue immunofluorescence experiments, dewaxing and antigen retrieval were performed as explained earlier. Further, tissues were permeabilised with 0.2% Triton X-100 in PBS for 30 min and later blocked with 5% bovine serum albumin in PBS. They were later stained with appropriate primary and secondary antibodies, and co-stained with 4',6-diamidino-2-phenylindole (DAPI; cat. H-1200, Vector Laboratories, Burlingame, CA, USA).

For immunocytochemistry experiments, cells were fixed with 4% paraformaldehyde for 20 min, permeabilised with 0.2% Triton X-100 in PBS for 30 min. They were blocked with 5% bovine serum albumin in PBS for 1 hour. Cells were then stained with appropriate primary and secondary antibodies, and co-stained with DAPI.

### Immunohistochemistry image analysis by Definiens software

Definiens tissue studio version 3.51 (Definiens AK, Munchen, Germany) was used to quantitate the IHC results²⁹ The slides were scanned and images were uploaded on Definiens

IHC software to further quantitate the results. Here, initially, in the cellular analysis module, 12 subsets were selected in the tissue specimen for site recognition. Nuclear detection, nuclear area detection and nucleus classification were also done on the subsets. For IHC-peroxidase staining, hematoxylin threshold was adjusted for cell detection. Analysis was done in 20X magnification.

### Western blotting

**For cells:** Western blotting was performed according to our previous protocol²⁹. 1-step nitro blue tetrazolium / S-bromo-4-chloroindoxyl phosphate (NBT/BCIP, cat. 34042; Thermo Scientific) was used to develop the blot.

**For tissues:** Tissues were lysed in RIPA buffer and homogenised in tissue lyser (Qiagen). They were centrifuged at 10,000 g for 20 min and the supernatant collected. Further protein separation and blotting were performed as explained for cell studies.

### Western blot band analysis by Image J software

Percentage of band intensity was calculated by Image J (Fiji, 1.47t, NIH, USA) analysis²⁹. Band intensity was calculated in the software by assessing profile plot of bands, followed by analysing and labelling the generated peaks. This band intensity was then normalised to the respective β-actin band. Then the percentage was calculated based on the intensity of the normalised individual bands compared to the total intensity of the rest of the bands in the particular row (for the particular protein), which were calculated accordingly.

### Masson-Fontana histochemistry

Briefly, 4-µm thick sections were cut from paraffinized tissue blocks. They were dewaxed in a series of xylene and ethanol. Antigen retrieval was done with citrate buffer (pH 6.0) at 60°C for 1 h. Tissues were treated with ammoniacal silver nitrate solution (10% silver nitrate (cat. 209139; Sigma-Aldrich) + conc. ammonium hydroxide (cat. 320145; Sigma-Aldrich) added drop-wise until it turns clear, leave 24 hours in dark at room temperature) for 40 min at 56°C in the dark. After washing in distilled water, the sections were treated with 5% aqueous sodium thiosulphate (cat. 72049; Sigma-Aldrich) for 1 min. Then the sections were washed in running tap water for 3 min and counterstained with haematoxylin for 3 min. After washing in distilled water, sections were dehydrated and coverslipped.

### FIG4 siRNA transfection

SHSY5Y cells (4 × 10⁵ cells/well) were cultured in 22 mm collagen coated cover slips in 6-well plates in triplicates and transfection was carried out in suspension using siRNA complexes prepared with siPORT^{™} NeoFX^{™} Transfection Reagent (Ambion) according to manufacturer's protocol. A scrambled version of siRNA (Ambion) was used as a control. The cells were later incubated for 96 hours in the transfection medium.

### Microscopy and nerve fibre thickness

Live-cell imaging was performed using an inverted Olympus IX71 microscope (Olympus, Southend-on-sea, UK) with a 20X NA objective. Fluorescence images were taken using an upright Olympus IX51 microscope (Olympus, UK) with a 100X oil immersion objective. The average fibre thickness was determined by taking 12 measurements/sample randomly from the fibre network and analysing in NIH Image J software.

### Statistical analysis

Data is presented as mean +/- standard deviation from three independent experiments performed in triplicates (n=3). Statistical analysis was calculated using one way ANOVA for comparison between three groups with Turkey post hoc test, and student's t test for comparison between two groups. Confidence intervals of 95% with corresponding P value of 0.05 was chosen throughout analysis.

### Results

### Re-innervation and neuropeptide synthesis was up-regulated in ES treated wounds

Temporal punch biopsies undergoing normal spontaneous healing and ES treatment were obtained from the upper inner arm of 40 healthy volunteers on days 3, 7, 10 and 14, following the previously published experimental design²⁹ (Materials and Methods). Initial analyses on wound healing day 14 showed that protein gene product 9.5 (PGP9.5) transcripts were significantly up-regulated in ES-treated wounds (ES14- electrically stimulated wound healing on day 14) compared to control healing wounds (C14- spontaneous wound healing on day 14; *P* < 0.05; ES14/C14 = 1.45; Fig. 1a). Also, the number of PGP9.5⁺ nerve fibres or Substance P⁺ (SP) cells (Fig. 1b-d and 1e-g respectively; Fig. 9a-b and 9a-b respectively) in wounded human skin, were significantly increased in ES-treated wounds (P < 0.05; ES14/C14 = 1.44 and 1.34 for PGP9.5⁺ and SP⁺ respectively). In normal healthy skin (day 0), thin unmyelinated nerve fibres were distributed throughout the epidermis, but sparsely dispersed above stratum granulosum. However, in healing wounds, we observed PGP9.5 expression in few cells in the dermis from day 3 onwards, which was at an earlier time point than seen previously³⁰. In the epidermis of wounds (ES-treated and untreated) on healing day 14, fibre-like nerve network were significantly higher in basal layer compared to that of healthy skin (day 0). Here, ES-treated wounds exhibited higher branching than normal healing wounds. Moreover, nerve fibre thickness in epidermis was ~ 6*%* higher in ES14 wounds compared to C14 wounds (P > 0.05; average thickness of nerve fibres were 1.02 ± 0.17 µm and 0.96 ± 0.17 µm, for ES14 and C14 respectively, normal skin was 1.08 ± 0.15 µm). Corroborative results for up-regulated PGP9.5 and SP expressions in ES-treated wounds were also observed from Western blotting (Fig. 1d and g, respectively for PGP9.5 and SP).

### TUBB3 and FIG4 were up-regulated in ES treated wounds

To identify targets of ES in re-innervation, we performed a whole genome transcriptional profiling using microarray of 1) normal healthy skin (N0), 2) spontaneous healing and 3) ES-treated healing wounds (both 2 and 3 on wound healing days 3, 7, 10 and 14). The data showed that neural differentiation as well as melanocyte-associated gene- class III β-tubulin (TUBB3)^{31,32}, were highly up-regulated in ES14 wounds (*P* = 6.19 × 10⁻⁵, ES14/N0 = 5.87; *P* = 4.5 × 10⁻³, C14/N0 = 4.4; *P* > 0.05, ES14/C14 = 1.33; Fig. 2a; Supplementary Dataset Sl). Subsequent qRT-PCR results revealed that intra-cutaneous TUBB3 transcription was indeed up-regulated (P < 0.05) in ES10 and ES14 compared to C10 and C14 respectively (Fig. 2b). This was further investigated by quantitative immunohistochemistry (IHC; utilising specific monoclonal TUJ-1 antibody³¹ against TUBB3) and Western blotting.

In normal healthy skin, TUBB3-like immunoreactivity was seen in isolated cells scattered along stratum basale, in addition to few intradermal cells (Fig. 2c). In the centre of the healing wound, TUBB3 was seen in differentiating epidermal layers, both in normal healing and ES treated wounds (Fig. 2d). However, in the wound edge and proximal to the wound centre, TUBB3 was scattered haphazardly in stratum basale cells with TUBB3⁺ dendritic-like structures branching out to the upper layers of the epidermis, reaching stratum granulosum (Fig. 2e and 2f for normal healing and ES-treated wounds respectively; Fig. 11). IHC results confirmed significant up-regulation (P < 0.05) of TUBB3 expression in ES-treated wounds compared to normal healing wounds (Fig. 2g). In wound granulation tissue, TUBB3 expression was mostly restricted to stellate and spindle-shaped cells (fibroblasts/myofibroblasts) and few cells lining neo-capillaries in the wound granulation area (Fig. 2h). Total TUBB3 protein up-regulation in ES-treated wounds compared to normal healing wounds was confirmed with Western blot results (Fig. 2i and j).

In microarray analysis, FIG4 (neural differentiation-related marker¹⁷) was significantly up-regulated in ES14 compared to C14 wounds (P < 0.05, ES14/C14 = 1.12; *P* > 0.05, C14/N0 = 0.86; P > 0.05, ES14/NO = 1.18; Supplementary Dataset S1). This was further confirmed by qRT-PCR (Fig. 3a), IHC (Fig. 3b and c) and Western blotting (Fig. 3d and e) analyses. In healthy skin, FIG4 was expressed in differentiating keratinocytes and few scattered cells in the dermis. However, in day 14 wounds, FIG4 was abundantly present in both epidermis and dermis. Throughout the repair process, FIG4⁺ cells were present in the granulation tissue and the neoepidermis (Fig. 12). Collectively, the quantitative data from both TUBB3 and FIG4 expressions suggest up-regulation of cellular differentiation by ES.

### ES expands the pool of melanocytes, up-regulates TUBB3 expression in melanocytes and promotes melanogenesis in cutaneous healing

In order to further characterise TUBB3, we examined TUBB3 expression in melanocytes (like peripheral neurons, melanocytes are derived from neural crest) since TUBB3 has been correlated to melanocyte developmental lineage³³. The effect of ES on melanocyte-associated genes and gene products have not been reported before. Here, we employed gp100 antibody (demarcates differentiated, melanogenically active melanocytes, amelanotic melanocytes, and melanoblasts³⁴) and Fontana-Masson histochemistry as melanocyte/melanogenesis read-out parameters³⁵. Gp100 was up-regulated in ES-treated wounds (*P* < 0.05, ES14/C14 = 1.26; *P* > 0.05 on all other healing days; Fig. 4a and Fig. 13a) and was present in both epidermis and dermis of healing wounds (Fig. 13a). However, a predominant population of gp100⁺ cells were localised in the epidermis of ES-treated wounds compared to a few in untreated wounds. Coimmunostaining of TUBB3 with gp100 revealed that ES up-regulated the number of intra-epidermal TUBB3/gp100 double-positive cells (*P* < 0.05; ES14/C14 = 1.32; Fig. 4b and c, Fig. 13a). ES also stimulated intra-epidermal melanogenesis (P < 0.05) as seen by quantitative Masson-Fontana histochemistry for melanin (Fig. 4d and e; Fig. 13b). These results indicate the potential of ES to promote TUBB3 expression outside the developing nervous system, recruit melanocytes and determine the extensiveness of re-pigmentation in healing human skin.

### ES up-regulates TUBB3 and PGP9.5 expression in Merkel cells in cutaneous granulation tissue

The spatial distribution of TUBB3⁺ cells during healing raised the possibility that this protein may also be expressed in Merkel cells, i.e. mechanosensory and neurosecretory epithelial cells that can be connected to a sensory nerve fibre³⁶. Moreover, supportive evidence to TUBB3 expression in Merkel cells in normal/regenerating human skin has not been cited before. Here, we employed Merkel cell-selective cytokeratin- CK20, as cell detection marker³⁷. Results showed that CK20⁺ Merkel cells increased in both the epidermal and dermal compartments with progressive healing days, especially in ES-treated wounds compared to controls (*P* > 0.05; Fig. 5a, Fig. 14a and b). From the co-expression results, few CK20⁺ cells also expressed TUBB3, and TUBB3⁺/CK20⁺ cells were observed in both epidermis and dermis (Fig. 5b and c; Fig. 14a). However, this fraction of cells increased in the epidermis with subsequent wound healing days. Interestingly, ES significantly increased TUBB3⁺/CK20⁺ cells on healing days 10 and 14 compared to respective normal healing wounds (P < 0.05, ES10/C10 and ES14/C14 = 1.44 and 1.50 respectively; P > 0.05 on all other healing days). In addition, ES significantly enhanced Merkel cell innervation, as assessed by CK20/PGP9.5 double-immunostaining (P < 0.05 for ES14/C14 = 1.48; Fig. 5d and e, Fig. 14b). Majority of CK20⁺/PGP9.5⁺ cells were localised in dermis in later wound healing days. Taken together, the data suggests enhancement of distinct sub-populations of Merkel cells (TUBB3⁺ and PGP9.5⁺), post-ES.

### Absence of proliferating melanocytes and Merkel cells in healing wounds and up-regulated nerve growth factor expression in ES-treated wounds

To better understand how melanocytes and Merkel cells were increased post-ES application (P < 0.05 and P > 0.05 respectively), Ki67/gp100 and Ki67/CK20 double-immunostaining were performed to understand the role of cell proliferation during repair. Surprisingly, the results showed absence of any proliferating (i.e. Ki67⁺) melanocytes and Merkel cells in the healing wound niche (Fig. 15a and b). This finding also raises a question on developmental biology perspective about the origin of Merkel cells, either from asymmetric divisions of basal layer cells or from the neural crest³⁸. Our results suggest that the increased number of both cell types observed post-ES could primarily be due to enhanced intra-cutaneous differentiation and migration of resident precursor cells, such as melanoblasts and Merkel cell progenitors.

Epidermal NGF (neurotrophic protein) has been partly correlated in vitro to specific differentiation processes of melanocytes (higher chemotaxis and dendricity¹⁸), as well as to higher innervation density in various tissues³⁹. Therefore we examined the expression of NGF as one plausible explanation for enhanced re-innervation, as well as Merkel cell and melanocyte cell numbers. Here, the results showed significant up-regulation of NGF mRNA (P < 0.05; ES14/C14 = 1.91; Fig. 6a) and protein expression (P < 0.05; ES14/C14 = 1.39 and 1.52, respectively, from IHC and Western blot analyses; Fig. 6b-e, Fig. 16), post-ES. However, cutaneous expression of NGF was detectable only from day 10 onwards during the repair process. Albeit, there was no significant difference in NGF expression in the dermal compartment of ES-treated and untreated wounds, NGF was significantly higher on day 14 in the epidermal compartment of ES-treated wounds. These results suggest ES-mediated differential regulation of NGF, which could also partly contribute to accelerated healing by ES (Fig. 6f).

### ES up-regulates FIG4 expression and enhances differentiation in human neuroblastoma cells

Having observed enhanced re-innervation in acute wounds, we further investigated whether ES can promote neural differentiation in *in vitro* conditions. Since FIG4 was differentially expressed in microarray analysis, we examined its role in neural differentiation. Here, we cultured SHSY5Y human neuroblastoma cells and differentiated using retinoic acid (RA) and brain-derived neurotrophic factor (BDNF)⁴⁰ (Fig. 7a; RA treatment for initial 6 days followed by BDNF treatment for 4 days). NeuN, a predominant post-mitotic neural nuclei marker, showed up-regulation in differentiated cells. Here, we observed an increase in FIG4 expression during the differentiation process (Fig. 7b). However, to determine the role of ES in neural differentiation, in addition to treating cells with RA+BDNF, they were also subjected to ES for 1 hour/day for 10 days. The electric field applied was 100 mV/mm as this value corresponded to the endogenous "current of injury" prevailing in cutaneous wound healing during initiation of granulation tissue, fibroplasia, wound contraction and neovascularization^{41,42}. qRT-PCR indicated significant increase of FIG4 on the 10^{th} day of RA+BDNF+ES treatment compared to RA+BDNF treatment (*P* < 0.05, ~ 1.5 fold increase; Fig. 7c). Moreover, Western blot analysis of neuroblastoma cells showed significant up-regulation of Class-II phosphatidylinositol-3-kinase (PI3KII) and FIG4, post-RA+BDNF+ES treatment (P < 0.05, increase of ~ 77*%* and ~ 43*%*, respectively; Fig. 7d and e).

Next, to understand the significance of FIG4 in PIP₃ pathway (Fig. 17a) during cell differentiation, FIG4 was knocked down in neuroblastoma cells by siRNA transfection (Fig. 17b and c). Here, intracellular vacuologenesis was observed after 3 days of transfection (Fig. 17d). These vacuoles were immunostained with specific lysosomal-associated membrane protein 2 (LAMP2) marker which typically represented late-stage endosomes (Fig. 8a).

Subsequently, we investigated whether siRNA transfected cells were further capable of neural differentiation and whether ES could affect the process. For this, we subjected siRNA transfected cells to differentiation by 1) RA+BDNF and 2) RA+BDNF+ES treatments. Interestingly, ES-treated cells showed better differentiation with higher nuclear NeuN, FIG4 and TUBB3 expressions, compared to non-ES-treated cells (Fig. 8b and 8c). Moreover, LAMP2 expression was also decreased post ES (Fig. 8d). qRT-PCR analysis revealed 2 fold increase in FIG4 mRNA expression post ES (Fig. 8e), which corroborated with Western blot data (Fig. 8f and 8g). Notably, FIG4 was the only protein that was up-regulated by ES in both mRNA and protein analyses. Additionally, to assess cell integrity post-cellular differentiation, ES was applied for 1 hour on the final day (10^{th} day) to RA+BDNF treated cells. Surprisingly, all cells were lysed within 35 minutes of ES (Supplementary Video S1) while the routinely exposed ES treated cells (RA+BDNF+ES) survived (Supplementary Video S2). This indicated the potential of ES to restore the integrity of neural cells post-vacuologenesis and during further differentiation process. Therefore, collectively, our studies suggest that ES enhances re-innervation and neural differentiation by targeting specific proteins such as TUBB3 and FIG4, during human cutaneous repair.

### Discussion

To our knowledge, this is the first report quantitatively investigating the expression of neural differentiation biomarkers during re-innervation in human cutaneous healing. Notably, ES robustly up-regulated 1) TUBB3 and FIG4 expression in skin wounds, 2) TUBB3 expression in wound melanocytes and Merkel cells, 2) melanocyte count and melanogenesis, 3) re-innervation in Merkel cells, 4) NGF expression in the wound granulation tissue and 5) neural differentiation as well as cell integrity post-vacuologenesis in neuroblastoma cells. Taken together, the current study provides evidence that ES can modulate pigment cell biology in melanocytes and neuroanatomy of normal human skin, stimulates the differentiation of resident melanoblasts and Merkel cell progenitors *in loco*, and contributes to the recognised enhancement of cutaneous wound healing ^{28,43}.

Up-regulation of TUBB3 in ES-treated wounds was characterised with an increase in dendritic processes of peripheral neurons (the cell bodies are located elsewhere) in dermis and epidermis. Interestingly, Shibazaki *et al.*, had noted that TUBB3 expression was cell-cycle dependent and is mediated by the binding of RE-1-silencing transcription factor- REST to RE-1 element, which is present in the first intron of TUBB3 gene³². In this context, it is possible that dysregulation of REST-TUBB3 axis may cause differential expression of TUBB3. Aside to TUBB3 being present in the microtubules of keratinocytes¹² and fibroblasts⁴⁴, no specific cellular functions have yet been attributed. However, further investigation of TUBB3 in mature melanocytes show that it is an active component of the microtubule tracks of kinesin and dynein proteins, which transport melanosomes³³. The physiological significance of TUBB3 expression in the epidermal nerve fibre network during repair has yet to be elucidated⁴⁵.

Like peripheral neurons, melanocytes are also derived from the neural crest, possessing multiple dendritic processes, similar receptors (e.g. p75^{NTR} and c-Kit) and responding to identical neurotrophins⁴⁶. NGF binds to neural/neural-cell related surface receptor- Tropomyosin receptor kinase A and initiate intracellular signalling pathways such as mitogen-activated protein kinase (MAPK) cascades, phosphatidyl inositol-3-kinase stimulation of AKT and phospholipase C γ-dependent generation of inositol triphosphate and diacylglycerol leading to mobilization and activation of Ca²⁺ stores¹¹. Also, MAPK kinase phosphorylates extracellular signal-regulated kinase 1/2 (member of MAPK family) leading to gene activation for cell differentiation. ES enhanced NGF expression in wounds, which may explain higher chemotaxis of melanocytes form neighbouring healthy interfollicular epidermis⁴⁷. Subsequent enhancement in melanogenesis is a secondary effect of either increase in melanocytes or acceleration of melanogenesis in individual cells, supplying the regenerating epidermis with sufficient melanin⁴⁸. In addition, up-regulated TUBB3 expression in melanocytes is also an indication of higher differentiation, characterised with increased dendrite formation¹⁸.

Interestingly, various pharmacological treatments have been shown to induce neurite outgrowth by over-expressing neurotrophins^{49,50}. NGF, in association with ES has been reported to induce neurite outgrowth *in vitro*¹¹, which in part is correlated to enhanced cellular differentiation process. In this context, we observed a similar effect with subsequent TUBB3 expression in association with nerve terminals with Merkel cells post-ES, which could lead to a differentiated lineage⁵¹. This data provides further insight into possible application of ES to augment the connection between innervation and undifferentiated/immature Merkel cells in the bulge area of hair follicles. Interestingly, majority of CK20⁺ cells in contact with PGP9.5⁺ cells were found only in the dermis. However, TUBB3⁺/CK20⁺ cells were observed in both epidermis and dermis with an increased numbers in the epidermis during final healing stages. This raises the question whether Merkel cells migrate to the epidermis post initiation of differentiation during wound healing or paradoxically, is it necessary for Merkel cells to have contact guidance with nerve elements to undergo differentiation?

During wound healing, the sequential migration of melanocyte progenitors from the epidermis to defined interfollicular bulge areas is reversed⁴⁸. In addition, progenitor cells exited their niche and migrated to the wound site without proliferation, e.g. melanocyte stem cells⁴⁷. Correlating to our quantitative IHC results on gp100⁺/CK20⁺ cells and proliferation with Ki67 antibody, it is concluded that progenitor cell migration and further fate of the cells could be determined by ES.

A dysregulation in the synthesis and turnover of phosphorylated phosphoinositol lipids, which function as signalling molecules⁵² during cell differentiation could lead to phenotypic alterations. Similarly, mutation in *FIG4* gene results in neurodegenerative disorders including Charcot-Marie-Tooth (CMT) disease and mucolipidosis⁵³. Our results on FIG4 knock down are in agreement with Chow *et al.*, which implicates the inactivation of FIG4 and PI(5) kinase (Pikfyve), leading to an abrogation of phosphatidylinositol 3,5-bisphosphate (PI(3,5)P₂) synthesis resulting in late endosome accumulation⁵³. Further, lack of PI(3,5)P2 leads to physiological intracellular vacuologenesis due to the defects in membrane signalling arising from late endosomes⁵⁴. Since analysis of intermediate phosphoinositides in PIP₃ pathway has not been performed, it is highly likely that a depletion in PI(3,5)P2 synthesis occurred in neuroblastoma cells as evident from vacuologenesis in the absence of FIG4. In our results, the vacuoles had late endosome biomarkers which was consistent with findings of Zhang *et al.*⁵⁴. Since up-regulation of PI3KII was not observed during RA+BDNF+ES treatment post-FIG4 siRNA (as observed without FIG4 siRNA treatment), the role of PI3KII in ES and neural differentiation is questionable.

During lipid turnover in plasma membrane during cell differentiation (Fig. 17a), the synthesis of PI(3,5)P2 from phosphatidylinositol 3-phosphate (PI(3)P) requires Pikfyve⁵⁵. The reverse reaction requires PI(3,5)P2 5-phosphatase FIG4⁵⁶ and interestingly, both the proteins for the forward and backward reactions coexist in the same protein complex⁵⁷. However, the specificity of ES towards only FIG4 in this multi-complex needs further investigation.

Finally, the apparent up-regulation of neuropeptides in the granulation tissue and the temporal and spatial advancement of nerve fibres in the epidermis of ES-treated wounds suggest the effect of ES to be more proximal to sensory nerves, albeit studies continue to examine the primary target of ES. In this context, the non-invasive methodology of ES to promote neural/neural-related cellular differentiation, in addition to our data showing increased cell survival post-ES, suggests new perspectives and molecular targets for ES in tissue repair. In conclusion, our findings provide evidence that ES application profoundly impacts on melanocytes, Merkel cells, and re-innervation of human skin wounds *in vivo.*

### Sequence listing

SEQ ID NO: 1 
1 agtccctgcc ctcccctggg gagggtgagt cacgccaaac tgggcggaga gtccgctggc
61 ctcactccta gctcatctgg gcggcggcgg caagtgggga cagggcgggt ggcgcatcac
121 cggcgcggag gcaggaggag cagtctcatt gttccgggag ccgtcaccac agtaggtccc
181 tcggctcagt cggcccagcc cctctcagtc ctccccaacc cccacaaccg cccgcggctc
241 tgagacgcgg ccccggcggc ggcggcagca gctgcagcat catctccacc ctccagccat
301 ggaagacctg gaccagtctc ctctggtctc gtcctcggac agcccacccc ggccgcagcc
361 cgcgttcaag taccagttcg tgagggagcc cgaggacgag gaggaagaag aggaggagga
421 agaggaggac gaggacgaag acctggagga gctggaggtg ctggagagga agcccgccgc
481 cgggctgtcc gcggccccag tgcccaccgc ccctgccgcc ggcgcgcccc tgatggactt
541 cggaaatgac ttcgtgccgc cggcgccccg gggacccctg ccggccgctc cccccgtcgc
601 cccggagcgg cagccgtctt gggacccgag cccggtgtcg tcgaccgtgc ccgcgccatc
661 cccgctgtct gctgccgcag tctcgccctc caagctccct gaggacgacg agcctccggc
721 ccggcctccc cctcctcccc cggccagcgt gagcccccag gcagagcccg tgtggacccc
781 gccagccccg gctcccgccg cgcccccctc caccccggcc gcgcccaagc gcaggggctc
841 ctcgggctca gtggatgaga ccctttttgc tcttcctgct gcatctgagc ctgtgatacg
901 ctcctctgca gaaaatatgg acttgaagga gcagccaggt aacactattt cggctggtca
961 agaggatttc ccatctgtcc tgcttgaaac tgctgcttct cttccttctc tgtctcctct
1021 ctcagccgct tctttcaaag aacatgaata ccttggtaat ttgtcaacag tattacccac
1081 tgaaggaaca cttcaagaaa atgtcagtga agcttctaaa gaggtctcag agaaggcaaa
1141 aactctactc atagatagag atttaacaga gttttcagaa ttagaatact cagaaatggg
1201 atcatcgttc agtgtctctc caaaagcaga atctgccgta atagtagcaa atcctaggga
1261 agaaataatc gtgaaaaata aagatgaaga agagaagtta gttagtaata acatccttca
1321 taatcaacaa gagttaccta cagctcttac taaattggtt aaagaggatg aagttgtgtc
1381 ttcagaaaaa gcaaaagaca gttttaatga aaagagagtt gcagtggaag ctcctatgag
1441 ggaggaatat gcagacttca aaccatttga gcgagtatgg gaagtgaaag atagtaagga
1501 agatagtgat atgttggctg ctggaggtaa aatcgagagc aacttggaaa gtaaagtgga
1561 taaaaaatgt tttgcagata gccttgagca aactaatcac gaaaaagata gtgagagtag
1621 taatgatgat acttctttcc ccagtacgcc agaaggtata aaggatcgtt caggagcata
1681 tatcacatgt gctcccttta acccagcagc aactgagagc attgcaacaa acatttttcc
1741 tttgttagga gatcctactt cagaaaataa gaccgatgaa aaaaaaatag aagaaaagaa
1801 ggcccaaata gtaacagaga agaatactag caccaaaaca tcaaaccctt ttcttgtagc
1861 agcacaggat tctgagacag attatgtcac aacagataat ttaacaaagg tgactgagga
1921 agtcgtggca aacatgcctg aaggcctgac tccagattta gtacaggaag catgtgaaag
1981 tgaattgaat gaagttactg gtacaaagat tgcttatgaa acaaaaatgg acttggttca
2041 aacatcagaa gttatgcaag agtcactcta tcctgcagca cagctttgcc catcatttga
2101 agagtcagaa gctactcctt caccagtttt gcctgacatt gttatggaag caccattgaa
2161 ttctgcagtt cctagtgctg gtgcttccgt gatacagccc agctcatcac cattagaagc
2221 ttcttcagtt aattatgaaa gcataaaaca tgagcctgaa aaccccccac catatgaaga
2281 ggccatgagt gtatcactaa aaaaagtatc aggaataaag gaagaaatta aagagcctga
2341 aaatattaat gcagctcttc aagaaacaga agctccttat atatctattg catgtgattt
2401 aattaaagaa acaaagcttt ctgctgaacc agctccggat ttctctgatt attcagaaat
2461 ggcaaaagtt gaacagccag tgcctgatca ttctgagcta gttgaagatt cctcacctga
2521 ttctgaacca gttgacttat ttagtgatga ttcaatacct gacgttccac aaaaacaaga
2581 tgaaactgtg atgcttgtga aagaaagtct cactgagact tcatttgagt caatgataga
2641 atatgaaaat aaggaaaaac tcagtgcttt gccacctgag ggaggaaagc catatttgga
2701 atcttttaag ctcagtttag ataacacaaa agataccctg ttacctgatg aagtttcaac
2761 attgagcaaa aaggagaaaa ttcctttgca gatggaggag ctcagtactg cagtttattc
2821 aaatgatgac ttatttattt ctaaggaagc acagataaga gaaactgaaa cgttttcaga
2881 ttcatctcca attgaaatta tagatgagtt ccctacattg atcagttcta aaactgattc
2941 attttctaaa ttagccaggg aatatactga cctagaagta tcccacaaaa gtgaaattgc
3001 taatgccccg gatggagctg ggtcattgcc ttgcacagaa ttgccccatg acctttcttt
3061 gaagaacata caacccaaag ttgaagagaa aatcagtttc tcagatgact tttctaaaaa
3121 tgggtctgct acatcaaagg tgctcttatt gcctccagat gtttctgctt tggccactca
3181 agcagagata gagagcatag ttaaacccaa agttcttgtg aaagaagctg agaaaaaact
3241 tccttccgat acagaaaaag aggacagatc accatctgct atattttcag cagagctgag
 
3301 taaaacttca gttgttgacc tcctgtactg gagagacatt aagaagactg gagtggtgtt
3361 tggtgccagc ctattcctgc tgctttcatt gacagtattc agcattgtga gcgtaacagc
3421 ctacattgcc ttggccctgc tctctgtgac catcagcttt aggatataca agggtgtgat
3481 ccaagctatc cagaaatcag atgaaggcca cccattcagg gcatatctgg aatctgaagt
3541 tgctatatct gaggagttgg ttcagaagta cagtaattct gctcttggtc atgtgaactg
3601 cacgataaag gaactcaggc gcctcttctt agttgatgat ttagttgatt ctctgaagtt
3661 tgcagtgttg atgtgggtat ttacctatgt tggtgccttg tttaatggtc tgacactact
3721 gattttggct ctcatttcac tcttcagtgt tcctgttatt tatgaacggc atcaggcaca
3781 gatagatcat tatctaggac ttgcaaataa gaatgttaaa gatgctatgg ctaaaatcca
3841 agcaaaaatc cctggattga agcgcaaagc tgaatgaaaa cgcccaaaat aattagtagg
3901 agttcatctt taaaggggat attcatttga ttatacgggg gagggtcagg gaagaacgaa
3961 ccttgacgtt gcagtgcagt ttcacagatc gttgttagat ctttattttt agccatgcac
4021 tgttgtgagg aaaaattacc tgtcttgact gccatgtgtt catcatctta agtattgtaa
4081 gctgctatgt atggatttaa accgtaatca tatctttttc ctatctatct gaggcactgg
4141 tggaataaaa aacctgtata ttttactttg ttgcagatag tcttgccgca tcttggcaag
4201 ttgcagagat ggtggagcta gaaaaaaaaa aaaaaaagcc cttttcagtt tgtgcactgt
4261 gtatggtccg tgtagattga tgcagatttt ctgaaatgaa atgtttgttt agacgagatc
4321 ataccggtaa agcaggaatg acaaagcttg cttttctggt atgttctagg tgtattgtga
4381 cttttactgt tatattaatt gccaatataa gtaaatatag attatatatg tatagtgttt
4441 cacaaagctt agacctttac cttccagcca ccccacagtg cttgatattt cagagtcagt
4501 cattggttat acatgtgtag ttccaaagca cataagctag aagaagaaat atttctagga
4561 gcactaccat ctgttttcaa catgaaatgc cacacacata gaactccaac atcaatttca
4621 ttgcacagac tgactgtagt taattttgtc acagaatcta tggactgaat ctaatgcttc
4681 caaaaatgtt gtttgtttgc aaatatcaaa cattgttatg caagaaatta ttaattacaa
4741 aatgaagatt tataccattg tggtttaagc tgtactgaac taaatctgtg gaatgcattg
4801 tgaactgtaa aagcaaagta tcaataaagc ttatagactt aaaaaaaaaa aaaaaaaaaa
4861 aaaaaaaaaa a
SEQ ID NO: 2 
MEDLDQSPLVSSSDSPPRPQPAFKYQFVREPEDEEEEEEEEEDEDEDLEELEVLERKPAAGLSAAPVPTAPAAGAP
LMDFGNDFVPPAPRGPLPAAPPVAPERQPSWDPSPVSSTVPAPSPLSAAAVSPSKLPEDDEPPARPPPPPPASVSPQ
AEPVWTPPAPAPAAPPSTPAAPKRRGSSGSVDETLFALPAASEPVIRSSAENMDLKEQPGNTISAGQEDFPSVLLET
AASLPSLSPLSAASFKEHEYLGNLSTVLPTEGTLQENVSEASKEVSEKAKTLLIDRDLTEFSELEYSEMGSSFSVSP
KAESAVIVANPREEIIVKNKDEEEKLVSNNILHNQQELPTALTKLVKEDEVVSSEKAKDSFNEKRVAVEAPMREEYA
DFKPFERVWEVKDSKEDSDMLAAGGKIESNLESKVDKKCFADSLEQTNHEKDSESSNDDTSFPSTPEGIKDRSGAYI
TCAPFNPAATESIATNIFPLLGDPTSENKTDEKKIEEKKAQIVTEKNTSTKTSNPFLVAAQDSETDYVTTDNLTKVT
EEVVANMPEGLTPDLVQEACESELNEVTGTKIAYETKMDLVQTSEVMQESLYPAAQLCPSFEESEATPSPVLPDIVM
EAPLNSAVPSAGASVIQPSSSPLEASSVNYESIKHEPENPPPYEEAMSVSLKKVSGIKEEIKEPENINAALQETEAP
YISIACDLIKETKLSAEPAPDFSDYSEMAKVEQPVPDHSELVEDSSPDSEPVDLFSDDSIPDVPQKQDETVMLVKES
LTETSFESMIEYENKEKLSALPPEGGKPYLESFKLSLDNTKDTLLPDEVSTLSKKEKIPLQMEELSTAVYSNDDLFI
SKEAQIRETETFSDSSPIEIIDEFPTLISSKTDSFSKLAREYTDLEVSHKSEIANAPDGAGSLPCTELPHDLSLKNI
QPKVEEKISFSDDFSKNGSATSKVLLLPPDVSALATQAEIESIVKPKVLVKEAEKKLPSDTEKEDRSPSAIFSAELS
KTSVVDLLYWRDIKKTGVVFGASLFLLLSLTVFSIVSVTAYIALALLSVTISFRIYKGVIQAIQKSDEGHPFRAYLE
SEVAISEELVQKYSNSALGHVNCTIKELRRLFLVDDLVDSLKFAVLMWVFTYVGALFNGLTLLILALISLFSVPVIY
ERHQAQIDHYLGLANKNVKDAMAKIQAKIPGLKRKAE
SEQ ID NO: 3 
1 atgctgcccg ggctcaggcg cctgctgcaa gctcccgcct cggcctgcct cctgctgatg
61 ctcctggccc tgcccctggc ggcccccagc tgccccatgc tctgcacctg ctactcatcc
121 ccgcccaccg tgagctgcca ggccaacaac ttctcctctg tgccgctgtc cctgccaccc
181 agcactcagc gactcttcct gcagaacaac ctcatccgca cgctgcggcc aggcaccttt
241 gggtccaacc tgctcaccct gtggctcttc tccaacaacc tctccaccat ctacccgggc
301 actttccgcc acttgcaagc cctggaggag ctggacctcg gtgacaaccg gcacctgcgc
361 tcgctggagc ccgacacctt ccagggcctg gagcggctgc agtcgctgca tttgtaccgc
421 tgccagctca gcagcctgcc cggcaacatc ttccgaggcc tggtcagcct gcagtacctc
481 tacctccagg agaacagcct gctccaccta caggatgact tgttcgcgga cctggccaac
541 ctgagccacc tcttcctcca cgggaaccgc ctgcggctgc tcacagagca cgtgtttcgc
 
 601 ggcctgggca gcctggaccg gctgctgctg cacgggaacc ggctgcaggg cgtgcaccgc
 661 gcggccttcc gcggcctcag ccgcctcacc atcctctacc tgttcaacaa cagcctggcc
 721 tcgctgcccg gcgaggcgct cgccgacctg ccctcgctcg agttcctgcg gctcaacgct
 781 aacccctggg cgtgcgactg ccgcgcgcgg ccgctctggg cctggttcca gcgcgcgcgc
 841 gtgtccagct ccgacgtgac ctgcgccacc cccccggagc gccagggccg agacctgcgc
 901 gcgctccgcg aggccgactt ccaggcgtgt ccgcccgcgg cacccacgcg gccgggcagc
 961 cgcgcccgcg gcaacagctc ctccaaccac ctgtacgggg tggccgaggc cggggcgccc
1021 ccagccgatc cctccaccct ctaccgagat ctgcctgccg aagactcgcg ggggcgccag
1081 ggcggggacg cgcctactga ggacgactac tgggggggct acgggggtga ggaccagcga
1141 ggggagcaga tgtgccccgg cgctgcctgc caggcgcccc cggactcccg aggccctgcg
1201 ctctcggccg ggctccccag ccctctgctt tgcctcctgc tcctggtgcc ccaccacctc
1261 tga
SEQ ID NO: 4 
MLPGLRRLLQAPASACLLLMLLALPLAAPSCPMLCTCYSSPPTVSCQANNFSSVPLSLPPSTQRLFLQNNLIRTLRP
GTFGSNLLTLWLFSNNLSTIYPGTFRHLQALEELDLGDNRHLRSLEPDTFQGLERLQSLHLYRCQLSSLPGNIFRGL
VSLQYLYLQENSLLHLQDDLFADLANLSHLFLHGNRLRLLTEHVFRGLGSLDRLLLHGNRLQGVHRAAFRGLSRLTI
LYLFNNSLASLPGEALADLPSLEFLRLNANPWACDCRARPLWAWFQRARVSSSDVTCATPPERQGRDLRALREADFQ
ACPPAAPTRPGSRARGNSSSNHLYGVAEAGAPPADPSTLYRDLPAEDSRGRQGGDAPTEDDYWGGYGGEDQRGEQMC
PGAACQAPPDSRGPALSAGLPSPLLCLLLLVPHHL
SEQ ID NO: 5 
1 atgcttcgca aagggtgctg tgtggaattg ctgctgctgt tggtagctgc ggagctgccc
61 ctgggtggtg gctgcccacg ggactgtgtg tgctacccgg cgcccatgac ggtcagctgc
121 caggcgcaca actttgcagc catcccggag ggcatccccg tggacagcga gcgcgtcttc
181 ctgcagaaca accgcatcgg cctcctccag cccggccact tcagccccgc catggtcacc
241 ctgtggatct actcgaacaa catcacctac atccacccca gcaccttcga gggcttcgtg
301 cacctggagg agctggacct cggcgacaac cggcagctgc ggacgctggc acccgagacc
361 ttccagggcc tggtgaagct tcacgccctc tacctctaca agtgtgggct cagcgccttg
421 ccggccggcg tctttggcgg cctgcacagc ctgcagtacc tctacctgca ggacaaccac
481 atcgagtacc tccaggacga catcttcgtg gacctggtca acctcagcca cctgtttctc
541 cacggcaaca agctgtggag tctgggcccg ggcaccttcc ggggcctggt gaacctggac
601 cgtcttttgc tgcacgagaa ccagctgcag tgggtccacc acaaggcatt ccacgacctc
661 cgcaggctga ccaccctctt cctcttcaac aacagcctct cggagctgca gggtgagtgc
721 ctggccccgc tgggggccct ggagttcctc cgcctcaacg gcaacccctg ggactgtggt
781 tgtcgcgcgc gctccctgtg ggaatggctg cagaggttcc ggggctccag ctccgctgtc
841 ccctgtgtgt cccctgggct gcggcacggc caggacctga agctgctgag ggccgaggac
901 ttccggaact gcacgggacc agcgtccccg caccagatca agtcacacac gctcaccacc
961 accgacaggg ccgcccgcaa ggaacaccac tcaccccacg gccccaccag gagcaagggc
1021 cacccgcacg gcccccggcc cggccacagg aagccgggga agaactgcac caaccccagg
1081 aaccgcaatc agatctctaa ggcgggcgcc gggaaacagg cccccgagct gccagactat
1141 gccccagact accagcacaa gttcagtttt gacatcatgc ctacggcccg gcccaagagg
1201 aagggcaagt gtgcccgcag gacccccatc cgtgccccca gcggggtgca gcaggcctcc
1261 tcggccagtt ccctgggggc ctccctcctg gcctggacac tggggctggc ggtcactctc
1321 cgctga
SEQ ID NO: 6

MLRKGCCVELLLLLVAAELPLGGGCPRDCVCYPAPMTVSCQAHNFAAIPEGIPVDSERVFLQNNRIGLLQPGHFSPA
MVTLWIYSNNITYIHPSTFEGFVHLEELDLGDNRQLRTLAPETFQGLVKLHALYLYKCGLSALPAGVFGGLHSLQYL
YLQDNHIEYLQDDIFVDLVNLSHLFLHGNKLWSLGPGTFRGLVNLDRLLLHENQLQWVHHKAFHDLRRLTTLFLFNN
SLSELQGECLAPLGALEFLRLNGNPWDCGCRARSLWEWLQRFRGSSSAVPCVSPGLRHGQDLKLLRAEDFRNCTGPA
SPHQIKSHTLTTTDRAARKEHHSPHGPTRSKGHPHGPRPGHRKPGKNCTNPRNRNQISKAGAGKQAPELPDYAPDYQ
HKFSFDIMPTARPKRKGKCARRTPIRAPSGVQQASSASSLGASLLAWTLGLAVTLR

### REFERENCES

1 Singer, H. S. et al. Advances in understanding the development of the nervous system. Current opinion in neurology 7, 153-159 (1994).
2 Pomeroy, S. L. & Kim, J. Y. Biology and pathobiology of neuronal development. Mental retardation and developmental disabilities research reviews 6, 41-46, doi:10.1002/(SICI)1098-2779(2000)6:1<41::AID-MRDD6>3.0.CO;2-V (2000).
3 Slominski, A. T. et al. Key role of CRF in the skin stress response system. Endocrine reviews 34, 827-884, doi:10.1210/er.2012-1092 (2013).
4 Hsu, Y. C., Li, L. & Fuchs, E. Emerging interactions between skin stem cells and their niches. Nature medicine 20, 847-856, doi:10.1038/nm.3643 (2014).
5 Scholzen, T. et al. Neuropeptides in the skin: interactions between the neuroendocrine and the skin immune systems. Experimental dermatology 7, 81-96 (1998).
6 Tanaka, T., Danno, K., Ikai, K. & Imamura, S. Effects of substance P and substance K on the growth of cultured keratinocytes. The Journal of investigative dermatology 90, 399-401 (1988).
7 Carmeliet, P. Angiogenesis in health and disease. Nature medicine 9, 653-660, doi:10.1038/nm0603-653 (2003).
8 Hendrix, S. & Peters, E. M. Neuronal plasticity and neuroregeneration in the skin -- the role of inflammation. Journal of neuroimmunology 184, 113-126, doi:10.1016/j.jneuroim.2006.11.020 (2007).
9 Karanth, S. S., Springall, D. R., Kuhn, D. M., Levene, M. M. & Polak, J. M. An immunocytochemical study of cutaneous innervation and the distribution of neuropeptides and protein gene product 9.5 in man and commonly employed laboratory animals. The American journal of anatomy 191, 369-383, doi:10.1002/aja.1001910404 (1991).
10 Reilly, D. M. et al. The epidermal nerve fibre network: characterization of nerve fibres in human skin by confocal microscopy and assessment of racial variations. The British journal of dermatology 137, 163-170 (1997).
11 Chang, Y. J., Hsu, C. M., Lin, C. H., Lu, M. S. & Chen, L. Electrical stimulation promotes nerve growth factor-induced neurite outgrowth and signaling. Biochimica et biophysica acta 1830, 4130-4136, doi:10.1016/j.bbagen.2013.04.007 (2013).
12 Jouhilahti, E. M., Peltonen, S. & Peltonen, J. Class III beta-tubulin is a component of the mitotic spindle in multiple cell types. The journal of histochemistry and cytochemistry : official journal of the Histochemistry Society 56, 1113-1119, doi:10.1369/jhc.2008.952002 (2008).
13 Prinetti, A. et al. Changes in the lipid turnover, composition, and organization, as sphingolipid-enriched membrane domains, in rat cerebellar granule cells developing in vitro. The Journal of biological chemistry 276, 21136-21145, doi:10.1074/jbc.M010666200 (2001).
14 Geisert, E. E., Jr. & Frankfurter, A. The neuronal response to injury as visualized by immunostaining of class III beta-tubulin in the rat. Neuroscience letters 102, 137-141 (1989).
15 Knox, S. M. et al. Parasympathetic stimulation improves epithelial organ regeneration. Nature communications 4, 1494, doi:10.1038/ncomms2493 (2013).
16 Luduena, R. F. Multiple forms of tubulin: different gene products and covalent modifications. International review of cytology 178, 207-275 (1998).
17 Winters, J. J. et al. Congenital CNS hypomyelination in the Fig4 null mouse is rescued by neuronal expression of the PI(3,5)P(2) phosphatase Fig4. The Journal of neuroscience : the official journal of the Society for Neuroscience 31, 17736-17751, doi:10.1523/JNEUROSCI.1482-11.2011 (2011).
18 Yaar, M., Grossman, K., Eller, M. & Gilchrest, B. A. Evidence for nerve growth factor-mediated paracrine effects in human epidermis. J Cell Biol 115, 821-828 (1991).
19 Vos, P., Stark, F. & Pittman, R. N. Merkel cells in vitro: production of nerve growth factor and selective interactions with sensory neurons. Developmental biology 144, 281-300 (1991).
20 Matos, M. A. & Cicerone, M. T. Alternating current electric field effects on neural stem cell viability and differentiation. Biotechnology progress 26, 664-670, doi:10.1002/btpr.389 (2010).
21 Sebastian, A. et al. A novel in vitro assay for electrophysiological research on human skin fibroblasts: degenerate electrical waves downregulate collagen I expression in keloid fibroblasts. Experimental dermatology 20, 64-68, doi:10.1111/j.1600-0625.2010.01150.x EXD1150 [pii] (2011).
22 Chang, K. A. et al. Biphasic electrical currents stimulation promotes both proliferation and differentiation of fetal neural stem cells. PloS one 6, e18738, doi:10.1371/journal.pone.0018738 (2011).
23 Zhu, B. et al. Large enhancement in neurite outgrowth on a cell membrane-mimicking conducting polymer. Nature communications 5, 4523, doi:10.1038/ncomms5523 (2014).
24 Wood, M. & Willits, R. K. Short-duration, DC electrical stimulation increases chick embryo DRG neurite outgrowth. Bioelectromagnetics 27, 328-331, doi:10.1002/bem.20214 (2006).
25 Udina, E. et al. Electrical stimulation of intact peripheral sensory axons in rats promotes outgrowth of their central projections. Experimental neurology 210, 238-247, doi:10.1016/j.expneurol.2007.11.007 (2008).
26 Ojingwa, J. C. & Isseroff, R. R. Electrical stimulation of wound healing. The Journal of investigative dermatology 121, 1-12, doi:12454 [pii] 10.1046/j.1523-1747.2003.12454.x (2003).
27 Kloth, L. C. Electrical stimulation for wound healing: a review of evidence from in vitro studies, animal experiments, and clinical trials. Int J Low Extrem Wounds 4, 23-44, doi:4/1/23 [pii] 10.1177/1534734605275733 (2005).
28 Sebastian, A. et al. Acceleration of cutaneous healing by electrical stimulation: degenerate electrical waveform down-regulates inflammation, up-regulates angiogenesis and advances remodeling in temporal punch biopsies in a human volunteer study. Wound repair and regeneration : official publication of the Wound Healing Society [and] the European Tissue Repair Society 19, 693-708, doi:10.1111/j.1524-475X.2011.00736.x (2011).
29 Sebastian, A., Iqbal, S. A., Colthurst, J., Volk, S. W. & Bayat, A. Electrical Stimulation Enhances Epidermal Proliferation in Human Cutaneous Wounds by Modulating p53-SIVA1 Interaction. The Journal of investigative dermatology, doi:10.1038/jid.2014.502 (2015).
30 Olerud, J. E., Chiu, D. S., Usui, M. L., Gibran, N. S. & Ansel, J. C. Protein gene product 9.5 is expressed by fibroblasts in human cutaneous wounds. The Journal of investigative dermatology 111, 565-572, doi:10.1046/j.1523-1747.1998.00330.x (1998).
31 Akasaka, K. et al. Loss of class III beta-tubulin induced by histone deacetylation is associated with chemosensitivity to paclitaxel in malignant melanoma cells. The Journal of investigative dermatology 129, 1516-1526, doi:10.1038/jid.2008.406 (2009).
32 Shibazaki, M. et al. Transcriptional and post-transcriptional regulation of betaIII-tubulin protein expression in relation with cell cycle-dependent regulation of tumor cells. International journal of oncology 40, 695-702, doi:10.3892/ijo.2011.1291 (2012).
33 Locher, H. et al. Class III beta-tubulin, a novel biomarker in the human melanocyte lineage. Differentiation; research in biological diversity 85, 173-181, doi:10.1016/j.diff.2013.05.003 (2013).
34 Singh, S. K. et al. The silver locus product (Silv/gp100/Pmel17) as a new tool for the analysis of melanosome transfer in human melanocyte-keratinocyte co-culture. Experimental dermatology 17, 418-426 (2008).
35 Hardman, J. A. et al. The Peripheral Clock Regulates Human Pigmentation. The Journal of investigative dermatology, doi:10.1038/jid.2014.442 (2014).
36 Lumpkin, E. A., Marshall, K. L. & Nelson, A. M. The cell biology of touch. J Cell Biol 191, 237-248, doi:10.1083/jcb.201006074 (2010).
37 Moll, I., Kuhn, C. & Moll, R. Cytokeratin-20 Is a General Marker of Cutaneous Merkel Cells While Certain Neuronal Proteins Are Absent. Journal of Investigative Dermatology 104, 910-915, doi:Doi 10.1111/1523-1747.Ep12606183 (1995).
38 Moll, I., Kuhn, C. & Moll, R. Cytokeratin 20 is a general marker of cutaneous Merkel cells while certain neuronal proteins are absent. The Journal of investigative dermatology 104, 910-915 (1995).
39 Korsching, S. & Thoenen, H. Nerve growth factor in sympathetic ganglia and corresponding target organs of the rat: correlation with density of sympathetic innervation. Proceedings of the National Academy of Sciences of the United States of America 80, 3513-3516 (1983).
40 Mastroeni, D. et al. Microglial responses to dopamine in a cell culture model of Parkinson's disease. Neurobiology of aging 30, 1805-1817, doi:10.1016/j.neurobiolaging.2008.01.001 (2009).
41 Nuccitelli, R., Nuccitelli, P., Ramlatchan, S., Sanger, R. & Smith, P. J. Imaging the electric field associated with mouse and human skin wounds. Wound repair and regeneration : official publication of the Wound Healing Society [and] the European Tissue Repair Society 16, 432-441, doi:10.1111/j.1524-475X.2008.00389.x WRR389 [pii] (2008).
42 Nuccitelli, R. et al. The electric field near human skin wounds declines with age and provides a noninvasive indicator of wound healing. Wound repair and regeneration : official publication of the Wound Healing Society [and] the European Tissue Repair Society 19, 645-655, doi:10.1111/j.1524-475X.2011.00723.x (2011).
43 Thakral, G. et al. Electrical stimulation to accelerate wound healing. Diabetic foot & ankle 4, doi:10.3402/dfa.v4i0.22081 (2013).
44 Draberova, E., Lukas, Z., Ivanyi, D., Viklicky, V. & Draber, P. Expression of class III beta-tubulin in normal and neoplastic human tissues. Histochemistry and cell biology 109, 231-239 (1998).
45 Lauria, G. et al. Tubule and neurofilament immunoreactivity in human hairy skin: markers for intraepidermal nerve fibers. Muscle & nerve 30, 310-316, doi:10.1002/mus.20098 (2004).
46 Yaar, M. & Park, H. Y. Melanocytes: a window into the nervous system. The Journal of investigative dermatology 132, 835-845, doi:10.1038/jid.2011.386 (2012).
47 Chou, W. C. et al. Direct migration of follicular melanocyte stem cells to the epidermis after wounding or UVB irradiation is dependent on Mclr signaling. Nature medicine 19, 924-929, doi:10.1038/nm.3194 (2013).
48 Paus, R. Migrating melanocyte stem cells: masters of disaster? Nature medicine 19, 818-819, doi:10.1038/nm.3264 (2013).
49 Blesch, A., Uy, H. S., Diergardt, N. & Tuszynski, M. H. Neurite outgrowth can be modulated in vitro using a tetracycline-repressible gene therapy vector expressing human nerve growth factor. Journal of neuroscience research 59, 402-409 (2000).
50 Hefti, F. Neurotrophic Factor Therapy for Nervous-System Degenerative Diseases. Journal of neurobiology 25, 1418-1435 (1994).
51 Uchigasaki, S., Suzuki, H. & Inoue, K. Merkel cells in the vellus hair follicles of human facial skin: A study using confocal laser microscopy. J Dermatol 31, 218-222 (2004).
52 Zolov, S. N. et al. In vivo, Pikfyve generates PI(3,5)P2, which serves as both a signaling lipid and the major precursor for PI5P. Proceedings of the National Academy of Sciences of the United States of America 109, 17472-17477, doi:10.1073/pnas.1203106109 (2012).
53 Chow, C. Y. et al. Mutation of FIG4 causes neurodegeneration in the pale tremor mouse and patients with CMT4J. Nature 448, 68-72, doi:10.1038/nature05876 (2007).
54 Zhang, Y. et al. Loss of Vac14, a regulator of the signaling lipid phosphatidylinositol 3,5-bisphosphate, results in neurodegeneration in mice. Proceedings of the National Academy of Sciences of the United States of America 104, 17518-17523, doi:10.1073/pnas.0702275104 (2007).
55 Gary, J. D., Wurmser, A. E., Bonangelino, C. J., Weisman, L. S. & Emr, S. D. Fablp is essential for PtdIns(3)P 5-kinase activity and the maintenance of vacuolar size and membrane homeostasis. J Cell Biol 143, 65-79, doi:Doi 10.1083/Jcb.143.1.65 (1998).
56 Duex, J. E., Nau, J. J., Kauffman, E. J. & Weisman, L. S. Phosphoinositide 5-phosphatase Fig4p is required for both acute rise and subsequent fall in stress-induced phosphatidylinositol 3,5-bisphosphate levels. Eukaryot Cell 5, 723-731, doi:Doi 10.1128/Ec.5.4.723-731.2006 (2006).
57 Dove, S. K., Dong, K., Kobayashi, T., Williams, F. K. & Michell, R. H. Phosphatidylinositol 3,5-bisphosphate and Fablp/PlKfyve underPPIn endo-lysosome function. Biochem J 419, 1-13, doi:Doi 10.1042/Bj20081950 (2009).
58 Ud-Din, S. et al. Electrical stimulation increases blood flow and haemoglobin levels in acute cutaneous wounds without affecting wound closure time: evidenced by non-invasive assessment of temporal biopsy wounds in human volunteers. Experimental dermatology 21, 758-764, doi:10.1111/exd.12005 (2012).
59 Shih, B. et al. Identification of biomarkers in sequential biopsies of patients with chronic wounds receiving simultaneous acute wounds: a genetic, histological, and noninvasive imaging study. Wound repair and regeneration : official publication of the Wound Healing Society [and] the European Tissue Repair Society 20, 757-769, doi:10.1111/j.1524-475X.2012.00832.x (2012).
60 Larsen., M. J. et al. Classifications within Molecular Subtypes Enables Identification of BRCA1/BRCA2 Mutation Carriers by RNA Tumor Profiling. PloS one 8, doi:10.1371/journal.pone.0064268 (2013).

## Claims

1. . A kit for promoting reinnervation of a damaged nerve in a patient, wherein the kit comprises:
a device for promoting reinnervation of a damaged nerve in a patient, the device comprising means to electrically stimulating the skin of the patient at a different location from the site of damage to the nerve; and
a scaffold which promotes reinnervation, a graft which promotes reinnervation, a population of cells which promotes reinnervation, or a pharmaceutical which promotes reinnervation;
wherein the means to electrically stimulate the skin of the patient is configured to electrically stimulate the skin of the patient with a fluctuating frequency cycle of from about 15 Hz to about 351 Hz and wherein the frequency changes between the upper and lower limits of the frequency range within a cycle of from about 1 second to about 20 seconds.

2. . A kit according to claim 1, wherein the means to electrically stimulate the skin of the patient is configured to electrically stimulate the skin of the patient with a current from about 0.001 mA to about 0.006 mA.

3. . A kit according to claim 1 or claim 2, wherein the means to electrically stimulate the skin of the patient is configured to electrically stimulate the skin of the patient with an amplitude of from about 10 V to about 100 V.

4. . A kit according to any one of claims 1 to 3, wherein the means to electrically stimulate the skin of the patient is configured to electrically stimuate the skin of the patient with interrupted cycles.

5. . A kit according to any one of claims 1 to 4, wherein the means to electrically stimulate the skin of the patient is configured to electrically stimulate the skin of the patient during one or more fluctuating frequency cycles at the end of a round of treatment.

## Patentansprüche

1. Kit zum Fördern von Reinnervation eines beschädigten Nervs in einem Patienten, wobei das Kit umfasst:
eine Vorrichtung zum Fördern von Reinnervation eines beschädigten Nervs in einem Patienten, wobei die Vorrichtung Mittel zum elektrischen Stimulieren der Haut des Patienten an einer unterschiedlichen Stelle von der Beschädigungsstelle des Nervs umfasst; und
ein Gerüst, das Reinnervation fördert, ein Transplantat, das Reinnervation fördert, eine Population von Zellen, die Reinnervation fördert, oder ein Arzneimittel, das Reinnervation fördert;
wobei das Mittel zum elektrischen Stimulieren der Haut des Patienten dazu konfiguriert ist, die Haut des Patienten mit einem fluktuierenden Frequenzzyklus von etwa 15 Hz bis etwa 351 Hz elektrisch zu stimulieren, und wobei sich die Frequenz zwischen dem oberen und dem unteren Limit des Frequenzbereichs innerhalb eines Zyklus von etwa 1 Sekunde bis etwa 20 Sekunden ändert.

2. Kit nach Anspruch 1, wobei das Mittel zum elektrischen Stimulieren der Haut des Patienten dazu konfiguriert ist, die Haut des Patienten mit einem Strom von etwa 0,001 mA bis etwa 0,006 mA elektrisch zu stimulieren.

3. Kit nach Anspruch 1 oder Anspruch 2, wobei das Mittel zum elektrischen Stimulieren der Haut des Patienten dazu konfiguriert ist, die Haut des Patienten mit einer Amplitude von etwa 10 V bis etwa 100 V elektrisch zu stimulieren.

4. Kit nach einem der Ansprüche 1 bis 3, wobei das Mittel zum elektrischen Stimulieren der Haut des Patienten dazu konfiguriert ist, die Haut des Patienten mit unterbrochenen Zyklen elektrisch zu stimulieren.

5. Kit nach einem der Ansprüche 1 bis 4, wobei das Mittel zum elektrischen Stimulieren der Haut des Patienten dazu konfiguriert ist, die Haut des Patienten während eines oder mehrerer Zyklen mit fluktuierender Frequenz an dem Ende eines Behandlungsdurchgangs elektrisch zu stimulieren.

## Revendications

1. Kit destiné à favoriser la réinnervation d'un nerf endommagé chez un patient, dans lequel le kit comprend :
un dispositif destiné à favoriser la réinnervation d'un nerf endommagé chez un patient, le dispositif comprenant un moyen pour stimuler électriquement la peau du patient en un emplacement différent du site d'endommagement du nerf ; et
un échafaudage qui favorise la réinnervation, une greffe qui favorise la réinnervation, une population de cellules qui favorisent la réinnervation, ou un produit pharmaceutique qui favorise la réinnervation ;
dans lequel le moyen pour stimuler électriquement la peau du patient est configuré pour stimuler électriquement la peau du patient avec un cycle de fréquences fluctuantes d'environ 15 Hz à environ 351 Hz et dans lequel la fréquence varie entre les limites supérieure et inférieure de la plage de fréquences au sein d'un cycle d'environ 1 seconde à environ 20 secondes.

2. Kit selon la revendication 1, dans lequel le moyen pour stimuler électriquement la peau du patient est configuré pour stimuler électriquement la peau du patient avec un courant d'environ 0,001 mA à environ 0,006 mA.

3. Kit selon la revendication 1 ou la revendication 2, dans lequel le moyen pour stimuler électriquement la peau du patient est configuré pour stimuler électriquement la peau du patient avec une amplitude d'environ 10 V à environ 100 V.

4. Kit selon l'une quelconque des revendications 1 à 3, dans lequel le moyen pour stimuler électriquement la peau du patient est configuré pour stimuler électriquement la peau du patient avec des cycles interrompus.

5. Kit selon l'une quelconque des revendications 1 à 4, dans lequel le moyen pour stimuler électriquement la peau du patient est configuré pour stimuler électriquement la peau du patient pendant un ou plusieurs cycles de fréquences fluctuantes à la fin d'une série de traitement.
